# EUROPEAN PATENT SPECIFICATION

(11) **EP 2 771 490 B1**
(45) Date of publication and mention of the grant of the patent: **17.01.2018**
(21) Application number: 12844262.1
(22) Date of filing: 25.10.2012
(51) Int. Cl.: C12Q 1/68

(54) **METHODS AND COMPOSITIONS FOR ASSESSING PATIENTS WITH REPRODUCTIVE FAILURE USING IMMUNE CELL-DERIVED MICRORNA**
VERFAHREN UND ZUSAMMENSETZUNGEN ZUR BEURTEILUNG VON PATIENTEN MIT REPRODUKTIVER STÖRUNG ANHAND VON MIRNA AUS IMMUNZELLEN
PROCÉDÉS ET COMPOSITIONS POUR L'ESTIMATION DE PATIENTS ATTEINTS D'INFERTILITÉ À L'AIDE D'UN MICRO-ARN ISSU D'UNE CELLULE IMMUNITAIRE

(30) Priority: 28.10.2011 US 201113284739
(43) Date of publication of application: 03.09.2014
(73) Proprietor: Winger, Edward E., San Francisco, California 94111 (US); Reed, Jane L., Klamath Falls, Oregon 97601 (US)
(72) Inventor: Winger, Edward E., San Francisco, California 94111 (US); Reed, Jane L., Klamath Falls, Oregon 97601 (US)
(74) Representative: Messina, Pietro
(86) International application number: PCT/US2012/061994
(87) International publication number: WO 2013/063322

(56) References cited:
- EP-A1- 2 336 353
- EP-A1- 2 354 246
- WO-A1-2009/015357
- WO-A2-2009/093254
- US-A1- 2009 176 237
- GLEN REID ET AL: "Circulating microRNAs: Association with disease and potential use as biomarkers", CRITICAL REVIEWS IN ONCOLOGY / HEMATOLOGY, ELSEVIER SCIENCE IRELAND LTD., LIMERICK, IE, vol. 80, no. 2, 9 November 2010 (2010-11-09), pages 193-208, XP028314259, ISSN: 1040-8428, DOI: 10.1016/J.CRITREVONC.2010.11.004 [retrieved on 2010-11-15]
- HOLMSTROM K ET AL: "Identification of a microRNA signature in dendritic cell vaccines for cancer immunotherapy", HUMAN IMMUNOLOGY, NEW YORK, NY, US, vol. 71, no. 1, 1 January 2010 (2010-01-01), pages 67-73, XP026794737, ISSN: 0198-8859 [retrieved on 2009-10-09]
- YI HU ET AL: "Two common SNPs in pri-miR-125a alter the mature miRNA expression and associate with recurrent pregnancy loss in a Han-Chinese population", RNA BIOLOGY, vol. 8, no. 5, 1 September 2011 (2011-09-01), pages 861-872, XP055200188, ISSN: 1547-6286, DOI: 10.4161/rna.8.5.16034
- A. REVEL ET AL: "MicroRNAs are associated with human embryo implantation defects", HUMAN REPRODUCTION, vol. 26, no. 10, 16 August 2011 (2011-08-16), pages 2830-2840, XP055021218, ISSN: 0268-1161, DOI: 10.1093/humrep/der255
- MONTENEGRO ET AL: "Differential expression of microRNAs with progression of gestation and inflammation in the human chorioamniotic membranes", AMERICAN JOURNAL OF OBSTETRICS & GYNECOLOGY, MOSBY, ST LOUIS, MO, US, vol. 197, no. 3, 1 September 2007 (2007-09-01), pages 289.e1-289.e6, XP022254132, ISSN: 0002-9378, DOI: 10.1016/J.AJOG.2007.06.027
- PINELES ET AL: "Distinct subsets of microRNAs are expressed differentially in the human placentas of patients with preeclampsia", AMERICAN JOURNAL OF OBSTETRICS & GYNECOLOGY, MOSBY, ST LOUIS, MO, US, vol. 196, no. 3, 6 March 2007 (2007-03-06) , pages 261.e1-261.e6, XP005913406, ISSN: 0002-9378, DOI: 10.1016/J.AJOG.2007.01.008
- CHRISTIANSEN ET AL.: 'A randomized, double-blind, placebo-controlled trial of intravenous immunoglobulin in the prevention of recurrent miscarriage: evidence for a therapeutic effect in women with secondary recurrent miscarriage.' HUMAN REPRODUCTION. vol. 17, no. 3, March 2002, pages 809 - 816, XP055200182
- OTSUKA ET AL.: 'Impaired microRNA processing causes corpus luteum insufficiency and infertility in mice.' JOURNAL OF CLINICAL INVESTIGATION. vol. 118, no. 6, 02 June 2008, pages 1944 - 1954, XP055150207
- DIANA M MORALES PRIETO ET AL: "MicroRNAs in pregnancy", JOURNAL OF REPRODUCTIVE IMMUNOLOGY, ELSEVIER SCIENCE IRELAND LTD, IE, vol. 88, no. 2, 12 January 2011 (2011-01-12), pages 106-111, XP028193739, ISSN: 0165-0378, DOI: 10.1016/J.JRI.2011.01.004 [retrieved on 2011-01-25]

### SEQUENCE LISTING

<110> Winger, Ed
<120> test
<130> 186.03
<160> 191
<210> 1
   <211> 22
   <212> RNA
   <213> homo sapiens
   <223> hsa-miR-582-3p MIMAT0004797
<400> 1
   uaacugguug aacaacugaa cc 22
<210> 2
   <211> 22
   <212> RNA
   <213> homo sapiens
   <223> hsa-miR-7-1-3p MIMAT0004553
<400> 2
   caacaaauca cagucugcca ua 22
<210> 3
   <211> 22
   <212> RNA
   <213> homo sapiens
   <223> hsa-miR-340-5p MIMAT0004692
<400> 3
   uuauaaagca augagacuga uu 22
<210> 4
   <211> 22
   <212> RNA
   <213> homo sapiens
   <223> hsa-miR-199b-3p MIMAT0004563
<400> 4
   acaguagucu gcacauuggu ua 22
<210> 5
   <211> 22
   <212> RNA
   <213> homo sapiens
   <223> hsa-miR-199a-3p MIMAT0000232
<400> 5
   acaguagucu gcacauuggu ua 22
<210> 6
   <211> 22
   <212> RNA
   <213> homo sapiens
   <223> hsa-miR-30e-5p MIMAT0000692
<400> 6
   uguaaacauc cuugacugga ag 22
<210> 7
   <211> 19
   <212> RNA
   <213> homo sapiens
   <223> hsa-miR-575 MIMAT0003240
<400> 7
   gagccaguug gacaggagc 19
<210> 8
   <211> 23
   <212> RNA
   <213> homo sapiens
   <223> hsa-miR-7-5p MIMAT0000252
<400> 8
   uggaagacua gugauuuugu ugu 23
<210> 9
   <211> 22
   <212> RNA
   <213> homo sapiens
   <223> hsa-miR-33a-3p MIMAT0004506
<400> 9
   caauguuucc acagugcauc ac 22
<210> 10
   <211> 22
   <212> RNA
   <213> homo sapiens
   <223> hsa-miR-7-2-3p MIMAT0004554
<400> 10
   caacaaaucc cagucuaccu aa 22
<210> 11
   <211> 23
   <212> RNA
   <213> homo sapiens
   <223> hsa-miR-199b-5p MIMAT0000263
<400> 11
   cccaguguuu agacuaucug uuc 23
<210> 12
   <211> 22
   <212> RNA
   <213> homo sapiens
   <223> hsa-miR-144-5p MIMAT0004600
<400> 12
   ggauaucauc auauacugua ag 22
<210> 13
   <211> 22
   <212> RNA
   <213> homo sapiens
   <223> hsa-miR-30e-3p MIMAT0000693
<400> 13
   cuuucagucg gauguuuaca gc 22
<210> 14
   <211> 21
   <212> RNA
   <213> homo sapiens
   <223> hsa-miR-424-3p MIMAT0004749
<400> 14
   caaaacguga ggcgcugcua u 21
<210> 15
   <211> 21
   <212> RNA
   <213> homo sapiens
   <223> hsa-miR-33a-5p MIMAT0000091
<400> 15
   gugcauugua guugcauugc a 21
<210> 16
   <211> 21
   <212> RNA
   <213> homo sapiens
   <223> hsa-miR-671-3p MIMAT0004819
<400> 16
   uccgguucuc agggcuccac c 21
<210> 17
   <211> 22
   <212> RNA
   <213> homo sapiens
   <223> hsa-miR-340-3p MIMAT0000750
<400> 17
   uccgucucag uuacuuuaua gc 22
<210> 18
   <211> 21
   <212> RNA
   <213> homo sapiens
   <223> hsa-miR-1267 MIMAT0005921
<400> 18
   ccuguugaag uguaaucccc a 21
<210> 19
   <211> 23
   <212> RNA
   <213> homo sapiens
   <223> hsa-miR-1229-3p MIMAT0005584
<400> 19
   cucucaccac ugcccuccca cag 23
<210> 20
   <211> 22
   <212> RNA
   <213> homo sapiens
   <223> hsa-miR-424-5p MIMAT0001341
<400> 20
   cagcagcaau ucauguuuug aa 22
<210> 21
   <211> 23
   <212> RNA
   <213> homo sapiens
   <223> hsa-miR-221-3p MIMAT0000278
<400> 21
   agcuacauug ucugcugggu uuc 23
<210> 22
   <211> 22
   <212> RNA
   <213> homo sapiens
   <223> hsa-miR-1 MIMAT0000416
<400> 22
   uggaauguaa agaaguaugu au 22
<210> 23
   <211> 22
   <212> RNA
   <213> homo sapiens
   <223> hsa-miR-133b MIMAT0000770
<400> 23
   uuuggucccc uucaaccagc ua 22
<210> 24
   <211> 22
   <212> RNA
   <213> homo sapiens
   <223> hsa-miR-221-5p MIMAT0004568
<400> 24
   accuggcaua caauguagau uu 22
<210> 25
   <211> 22
   <212> RNA
   <213> homo sapiens
   <223> hsa-miR-210 MIMAT0000267
<400> 25
   cugugcgugu gacagcggcu ga 22
<210> 26
   <211> 24
   <212> RNA
   <213> homo sapiens
   <223> hsa-miR-1229-5p MIMAT0022942
<400> 26
   guggguaggg uuugggggag agcg 24
<210> 27
   <211> 23
   <212> RNA
   <213> homo sapiens
   <223> hsa-miR-671-5p MIMAT0003880
<400> 27
   aggaagcccu ggaggggcug gag 23
<210> 28
   <211> 23
   <212> RNA
   <213> homo sapiens
   <223> hsa-miR-582-5p MIMAT0003247
<400> 28
   uuacaguugu ucaaccaguu acu 23
<210> 29
   <211> 23
   <212> RNA
   <213> homo sapiens
   <223> hsa-miR-199a-5p MIMAT0000231
<400> 29
   cccaguguuc agacuaccug uuc 23
<210> 30
   <211> 20
   <212> RNA
   <213> homo sapiens
   <223> hsa-miR-144-3p MIMAT0000436
<400> 30
   uacaguauag augauguacu 20
<210> 31
   <211> 22
   <212> RNA
   <213> homo sapiens
   <223> hsa-miR-376a-5p MIMAT0003386
<400> 31
   guagauucuc cuucuaugag ua 22
<210> 32
   <211> 22
   <212> RNA
   <213> homo sapiens
   <223> hsa-miR-193a-3p MIMAT0000459
<400> 32
   aacuggccua caaaguccca gu 22
<210> 33
   <211> 23
   <212> RNA
   <213> homo sapiens
   <223> hsa-miR-557 MIMAT0003221
<400> 33
   guuugcacgg gugggccuug ucu 23
<210> 34
   <211> 22
   <212> RNA
   <213> homo sapiens
   <223> hsa-miR-34a-3p MIMAT0004557
<400> 34
   caaucagcaa guauacugcc cu 22
<210> 35
   <211> 22
   <212> RNA
   <213> homo sapiens
   <223> hsa-miR-584-5p MIMAT0003249
<400> 35
   uuaugguuug ccugggacug ag 22
<210> 36
   <211> 26
   <212> RNA
   <213> homo sapiens
   <223> hsa-miR-1244 MIMAT0005896
<400> 36
   aaguaguugg uuuguaugag augguu 26
<210> 37
   <211> 22
   <212> RNA
   <213> homo sapiens
   <223> hsa-miR-125b-1-3p MIMAT0004592
<400> 37
   acggguuagg cucuugggag cu 22
<210> 38
   <211> 22
   <212> RNA
   <213> homo sapiens
   <223> hsa-miR-32-3p MIMAT0004505
<400> 38
   caauuuagug ugugugauau uu 22
<210> 39
   <211> 22
   <212> RNA
   <213> homo sapiens
   <223> hsa-miR-933 MIMAT0004976
<400> 39
   ugugcgcagg gagaccucuc cc 22
<210> 40
   <211> 22
   <212> RNA
   <213> homo sapiens
   <223> hsa-miR-373-5p MIMAT0000725
<400> 40
   acucaaaaug ggggcgcuuu cc 22
<210> 41
   <211> 22
   <212> RNA
   <213> homo sapiens
   <223> hsa-let-7b-5p MIMAT0000063
<400> 41
   ugagguagua gguugugugg uu 22
<210> 42
   <211> 21
   <212> RNA
   <213> homo sapiens
   <223> hsa-miR-376a-3p MIMAT0000729
<400> 42
   aucauagagg aaaauccacg u 21
<210> 43
   <211> 22
   <212> RNA
   <213> homo sapiens
   <223> hsa-miR-129-2-3p MIMAT0004605
<400> 43
   aagcccuuac cccaaaaagc au 22
<210> 44
   <211> 22
   <212> RNA
   <213> homo sapiens
   <223> hsa-miR-548am-3p MIMAT0019076
<400> 44
   caaaaacugc aguuacuuuu gu 22
<210> 45
   <211> 22
   <212> RNA
   <213> homo sapiens
   <223> hsa-let-7f-5p MIMAT0000067
<400> 45
   ugagguagua gauuguauag uu 22
<210> 46
   <211> 22
   <212> RNA
   <213> homo sapiens
   <223> hsa-miR-876-3p MIMAT0004925
<400> 46
   uggugguuua caaaguaauu ca 22
<210> 47
   <211> 20
   <212> RNA
   <213> homo sapiens
   <223> hsa-miR-371a-5p MIMAT0004687
<400> 47
   acucaaacug ugggggcacu 20
<210> 48
   <211> 23
   <212> RNA
   <213> homo sapiens
   <223> hsa-miR-423-5p MIMAT0004748
<400> 48
   ugaggggcag agagcgagac uuu 23
<210> 49
   <211> 23
   <212> RNA
   <213> homo sapiens
   <223> hsa-miR-373-3p MIMAT0000726
<400> 49
   gaagugcuuc gauuuugggg ugu 23
<210> 50
   <211> 21
   <212> RNA
   <213> homo sapiens
   <223> hsa-miR-152 MIMAT0000438
<400> 50
   ucagugcaug acagaacuug g 21
<210> 51
   <211> 22
   <212> RNA
   <213> homo sapiens
   <223> hsa-miR-34a-5p MIMAT0000255
<400> 51
   uggcaguguc uuagcugguu gu 22
<210> 52
   <211> 23
   <212> RNA
   <213> homo sapiens
   <223> hsa-miR-335-5p MIMAT0000765
<400> 52
   ucaagagcaa uaacgaaaaa ugu 23
<210> 53
   <211> 22
   <212> RNA
   <213> homo sapiens
   <223> hsa-miR-181c-5p MIMAT0000258
<400> 53
   aacauucaac cugucgguga gu 22
<210> 54
   <211> 22
   <212> RNA
   <213> homo sapiens
   <223> hsa-miR-125b-2-3p MIMAT0004603
<400> 54
   ucacaaguca ggcucuuggg ac 22
<210> 55
   <211> 22
   <212> RNA
   <213> homo sapiens
   <223> hsa-miR-548am-5p MIMAT0022740
<400> 55
   aaaaguaauu gcgguuuuug cc 22
<210> 56
   <211> 22
   <212> RNA
   <213> homo sapiens
   <223> hsa-miR-338-3p MIMAT0000763
<400> 56
   uccagcauca gugauuuugu ug 22
<210> 57
   <211> 22
   <212> RNA
   <213> homo sapiens
   <223> hsa-miR-1225-5p MIMAT0005572
<400> 57
   guggguacgg cccagugggg gg 22
<210> 58
   <211> 22
   <212> RNA
   <213> homo sapiens
   <223> hsa-miR-362-3p MIMAT0004683
<400> 58
   aacacaccua uucaaggauu ca 22
<210> 59
   <211> 23
   <212> RNA
   <213> homo sapiens
   <223> hsa-miR-767-5p MIMAT0003882
<400> 59
   ugcaccaugg uugucugagc aug 23
<210> 60
   <211> 22
   <212> RNA
   <213> homo sapiens
   <223> hsa-miR-136-3p MIMAT0004606
<400> 60
   caucaucguc ucaaaugagu cu 22
<210> 61
   <211> 24
   <212> RNA
   <213> homo sapiens
   <223> hsa-miR-29b-1-5p MIMAT0004514
<400> 61
   gcugguuuca uauggugguu uaga 24
<210> 62
   <211> 22
   <212> RNA
   <213> homo sapiens
   <223> hsa-miR-29a-3p MIMAT0000086
<400> 62
   uagcaccauc ugaaaucggu ua 22
<210> 63
   <211> 22
   <212> RNA
   <213> homo sapiens
   <223> hsa-miR-92b-3p MIMAT0003218
<400> 63
   uauugcacuc gucccggccu cc 22
<210> 64
   <211> 24
   <212> RNA
   <213> homo sapiens
   <223> hsa-miR-362-5p MIMAT0000705
<400> 64
   aauccuugga accuaggugu gagu 24
<210> 65
   <211> 22
   <212> RNA
   <213> homo sapiens
   <223> hsa-miR-223-5p MIMAT0004570
<400> 65
   cguguauuug acaagcugag uu 22
<210> 66
   <211> 22
   <212> RNA
   <213> homo sapiens
   <223> hsa-miR-505-3p MIMAT0002876
<400> 66
   cgucaacacu ugcugguuuc cu 22
<210> 67
   <211> 22
   <212> RNA
   <213> homo sapiens
   <223> hsa-miR-634 MIMAT0003304
<400> 67
   aaccagcacc ccaacuuugg ac 22
<210> 68
   <211> 23
   <212> RNA
   <213> homo sapiens
   <223> hsa-miR-371a-3p MIMAT0000723
<400> 68
   aagugccgcc aucuuuugag ugu 23
<210> 69
   <211> 22
   <212> RNA
   <213> homo sapiens
   <223> hsa-miR-129-1-3p MIMAT0004548
<400> 69
   aagcccuuac cccaaaaagu au 22
<210> 70
   <211> 23
   <212> RNA
   <213> homo sapiens
   <223> hsa-miR-1238-5p MIMAT0022947
<400> 70
   gugaguggga gccccagugu gug 23
<210> 71
   <211> 22
   <212> RNA
   <213> homo sapiens
   <223> hsa-miR-876-5p MIMAT0004924
<400> 71
   uggauuucuu ugugaaucac ca 22
<210> 72
   <211> 22
   <212> RNA
   <213> homo sapiens
   <223> hsa-miR-181c-3p MIMAT0004559
<400> 72
   aaccaucgac cguugagugg ac 22
<210> 73
   <211> 22
   <212> RNA
   <213> homo sapiens
   <223> hsa-miR-338-5p MIMAT0004701
<400> 73
   aacaauaucc uggugcugag ug 22
<210> 74
   <211> 22
   <212> RNA
   <213> homo sapiens
   <223> hsa-miR-505-5p MIMAT0004776
<400> 74
   gggagccagg aaguauugau gu 22
<210> 75
   <211> 22
   <212> RNA
   <213> homo sapiens
   <223> hsa-miR-335-3p MIMAT0004703
<400> 75
   uuuuucauua uugcuccuga cc 22
<210> 76
   <211> 22
   <212> RNA
   <213> homo sapiens
   <223> hsa-miR-543 MIMAT0004954
<400> 76
   aaacauucgc ggugcacuuc uu 22
<210> 77
   <211> 22
   <212> RNA
   <213> homo sapiens
   <223> hsa-miR-223-3p MIMAT0000280
<400> 77
   ugucaguuug ucaaauaccc ca 22
<210> 78
   <211> 22
   <212> RNA
   <213> homo sapiens
   <223> hsa-miR-125b-5p MIMAT0000423
<400> 78
   ucccugagac ccuaacuugu ga 22
<210> 79
   <211> 20
   <212> RNA
   <213> homo sapiens
   <223> hsa-miR-1238-3p MIMAT0005593
<400> 79
   cuuccucguc ugucugcccc 20
<210> 80
   <211> 22
   <212> RNA
   <213> homo sapiens
   <223> hsa-miR-377-5p MIMAT0004689
<400> 80
   agagguugcc cuuggugaau uc 22
<210> 81
   <211> 22
   <212> RNA
   <213> homo sapiens
   <223> hsa-miR-584-3p MIMAT0022708
<400> 81
   ucaguuccag gccaaccagg cu 22
<210> 82
   <211> 22
   <212> RNA
   <213> homo sapiens
   <223> hsa-miR-22-5p MIMAT0004495
<400> 82
   aguucuucag uggcaagcuu ua 22
<210> 83
   <211> 21
   <212> RNA
   <213> homo sapiens
   <223> hsa-miR-376a-2-5p MIMAT0022928
<400> 83
   gguagauuuu ccuucuaugg u 21
<210> 84
   <211> 22
   <212> RNA
   <213> homo sapiens
   <223> hsa-miR-301a-5p MIMAT0022696
<400> 84
   gcucugacuu uauugcacua cu 22
<210> 85
   <211> 21
   <212> RNA
   <213> homo sapiens
   <223> hsa-miR-548m MIMAT0005917
<400> 85
   caaagguauu ugugguuuuu g 21
<210> 86
   <211> 23
   <212> RNA
   <213> homo sapiens
   <223> hsa-miR-29b-3p MIMAT0000100
<400> 86
   uagcaccauu ugaaaucagu guu 23
<210> 87
   <211> 22
   <212> RNA
   <213> homo sapiens
   <223> hsa-miR-99a-3p MIMAT0004511
<400> 87
   caagcucgcu ucuauggguc ug 22
<210> 88
   <211> 22
   <212> RNA
   <213> homo sapiens
   <223> hsa-miR-33b-3p MIMAT0004811
<400> 88
   cagugccucg gcagugcagc cc 22
<210> 89
   <211> 22
   <212> RNA
   <213> homo sapiens
   <223> hsa-miR-92b-5p MIMAT0004792
<400> 89
   agggacggga cgcggugcag ug 22
<210> 90
   <211> 23
   <212> RNA
   <213> homo sapiens
   <223> hsa-miR-602 MIMAT0003270
<400> 90
   gacacgggcg acagcugcgg ccc 23
<210> 91
   <211> 21
   <212> RNA
   <213> homo sapiens
   <223> hsa-miR-1237-3p MIMAT0005592
<400> 91
   uccuucugcu ccguccccca g 21
<210> 92
   <211> 21
   <212> RNA
   <213> homo sapiens
   <223> hsa-miR-129-5p MIMAT0000242
<400> 92
   cuuuuugcgg ucugggcuug c 21
<210> 93
   <211> 22
   <212> RNA
   <213> homo sapiens
   <223> hsa-miR-148b-3p MIMAT0000759
<400> 93
   ucagugcauc acagaacuuu gu 22
<210> 94
   <211> 22
   <212> RNA
   <213> homo sapiens
   <223> hsa-miR-377-3p MIMAT0000730
<400> 94
   aucacacaaa ggcaacuuuu gu 22
<210> 95
   <211> 22
   <212> RNA
   <213> homo sapiens
   <223> hsa-let-7b-3p MIMAT0004482
<400> 95
   cuauacaacc uacugccuuc cc 22
<210> 96
   <211> 24
   <212> RNA
   <213> homo sapiens
   <223> hsa-miR-125a-5p MIMAT0000443
<400> 96
   ucccugagac ccuuuaaccu guga 24
<210> 97
   <211> 22
   <212> RNA
   <213> homo sapiens
   <223> hsa-miR-125a-3p MIMAT0004602
<400> 97
   acaggugagg uucuugggag cc 22
<210> 98
   <211> 22
   <212> RNA
   <213> homo sapiens
   <223> hsa-miR-148b-5p MIMAT0004699
<400> 98
   aaguucuguu auacacucag gc 22
<210> 99
   <211> 22
   <212> RNA
   <213> homo sapiens
   <223> hsa-miR-22-3p MIMAT0000077
<400> 99
   aagcugccag uugaagaacu gu 22
<210> 100
   <211> 21
   <212> RNA
   <213> homo sapiens
   <223> hsa-miR-1237-5p MIMAT0022946
<400> 100
   cgggggcggg gccgaagcgc g 21
<210> 101
   <211> 22
   <212> RNA
   <213> homo sapiens
   <223> hsa-let-7f-1-3p MIMAT0004486
<400> 101
   cuauacaauc uauugccuuc cc 22
<210> 102
   <211> 22
   <212> RNA
   <213> homo sapiens
   <223> hsa-miR-29a-5p MIMAT0004503
<400> 102
   acugauuucu uuugguguuc ag 22
<210> 103
   <211> 22
   <212> RNA
   <213> homo sapiens
   <223> hsa-miR-193a-5p MIMAT0004614
<400> 103
   ugggucuuug cgggcgagau ga 22
<210> 104
   <211> 23
   <212> RNA
   <213> homo sapiens
   <223> hsa-miR-423-3p MIMAT0001340
<400> 104
   agcucggucu gaggccccuc agu 23
<210> 105
   <211> 22
   <212> RNA
   <213> homo sapiens
   <223> hsa-miR-191-3p MIMAT0001618
<400> 105
   gcugcgcuug gauuucgucc cc 22
<210> 106
   <211> 23
   <212> RNA
   <213> homo sapiens
   <223> hsa-miR-301a-3p MIMAT0000688
<400> 106
   cagugcaaua guauugucaa agc 23
<210> 107
   <211> 23
   <212> RNA
   <213> homo sapiens
   <223> hsa-miR-767-3p MIMAT0003883
<400> 107
   ucugcucaua ccccaugguu ucu 23
<210> 108
   <211> 19
   <212> RNA
   <213> homo sapiens
   <223> hsa-miR-563 MIMAT0003227
<400> 108
   agguugacau acguuuccc 19
<210> 109
   <211> 22
   <212> RNA
   <213> homo sapiens
   <223> hsa-miR-95 MIMAT0000094
<400> 109
   uucaacgggu auuuauugag ca 22
<210> 110
   <211> 22
   <212> RNA
   <213> homo sapiens
   <223> hsa-miR-1234-3p MIMAT0005589
<400> 110
   ucggccugac cacccacccc ac 22
<210> 111
   <211> 22
   <212> RNA
   <213> homo sapiens
   <223> hsa-miR-1225-3p MIMAT0005573
<400> 111
   ugagccccug ugccgccccc ag 22
<210> 112
   <211> 23
   <212> RNA
   <213> homo sapiens
   <223> hsa-miR-136-5p MIMAT0000448
<400> 112
   acuccauuug uuuugaugau gga 23
<210> 113
   <211> 21
   <212> RNA
   <213> homo sapiens
   <223> hsa-miR-1234-5p MIMAT0022944
<400> 113
   gggggggggg ggggggggcc g 21
<210> 114
   <211> 22
   <212> RNA
   <213> homo sapiens
   <223> hsa-miR-99a-5p MIMAT0000097
<400> 114
   aacccguaga uccgaucuug ug 22
<210> 115
   <211> 22
   <212> RNA
   <213> homo sapiens
   <223> hsa-miR-32-5p MIMAT0000090
<400> 115
   uauugcacau uacuaaguug ca 22
<210> 116
   <211> 23
   <212> RNA
   <213> homo sapiens
   <223> hsa-miR-191-5p MIMAT0000440
<400> 116
   caacggaauc ccaaaagcag cug 23
<210> 117
   <211> 20
   <212> RNA
   <213> homo sapiens
   <223> hsa-miR-33b-5p MIMAT0003301
<400> 117
   gugcauugcu guugcauugc 20
<210> 118
   <211> 71
   <212> RNA
   <213> homo sapiens
   <223> hsa-mir-1-1 MI0000651
<400> 118
   ugggaaacau acuucuuuau augcccauau ggaccugcua agcuauggaa uguaaagaag 60
   uauguaucuc a 71
<210> 119
   <211> 85
   <212> RNA
   <213> homo sapiens
   <223> hsa-mir-1-2 MI0000437
<400> 119
   accuacucag aguacauacu ucuuuaugua cccauaugaa cauacaaugc uauggaaugu 60
   aaagaaguau guauuuuugg uaggc 85
<210> 120
   <211> 110
   <212> RNA
   <213> homo sapiens
   <223> hsa-mir-7-1 MI0000263
<400> 120
   uuggauguug gccuaguucu guguggaaga cuagugauuu uguuguuuuu agauaacuaa 60
   aucgacaaca aaucacaguc ugccauaugg cacaggccau gccucuacag 110
<210> 121
   <211> 110
   <212> RNA
   <213> homo sapiens
   <223> hsa-mir-7-2 MI0000264
<400> 121
   cuggauacag aguggaccgg cuggccccau cuggaagacu agugauuuug uuguugucuu 60
   acugcgcuca acaacaaauc ccagucuacc uaauggugcc agccaucgca 110
<210> 122
   <211> 110
   <212> RNA
   <213> homo sapiens
   <223> hsa-mir-7-3 MI0000265
<400> 122
   agauuagagu ggcugugguc uagugcugug uggaagacua gugauuuugu uguucugaug 60
   uacuacgaca acaagucaca gccggccuca uagcgcagac ucccuucgac 110
<210> 123
   <211> 92
   <212> RNA
   <213> homo sapiens
   <223> hsa-mir-30e MI0000749
<400> 123
   gggcagucuu ugcuacugua aacauccuug acuggaagcu guaagguguu cagaggagcu 60
   uucagucgga uguuuacagc ggcaggcugc ca 92
<210> 124
   <211> 69
   <212> RNA
   <213> homo sapiens
   <223> hsa-mir-33a MI0000091
<400> 124
   cuguggugca uuguaguugc auugcauguu cuggugguac ccaugcaaug uuuccacagu 60
   gcaucacag 69
<210> 125
   <211> 119
   <212> RNA
   <213> homo sapiens
   <223> hsa-mir-133b MI0000822
<400> 125
   ccucagaaga aagaugcccc cugcucuggc uggucaaacg gaaccaaguc cgucuuccug 60
   agagguuugg uccccuucaa ccagcuacag cagggcuggc aaugcccagu ccuuggaga 119
<210> 126
   <211> 86
   <212> RNA
   <213> homo sapiens
   <223> hsa-mir-144 MI0000460
<400> 126
   uggggcccug gcugggauau caucauauac uguaaguuug cgaugagaca cuacaguaua 60
   gaugauguac uaguccgggc accccc 86
<210> 127
   <211> 71
   <212> RNA
   <213> homo sapiens
   <223> hsa-mir-199a-1 MI0000242
<400> 127
   gccaacccag uguucagacu accuguucag gaggcucuca auguguacag uagucugcac 60
   auugguuagg c 71
<210> 128
   <211> 110
   <212> RNA
   <213> homo sapiens
   <223> hsa-mir-199a-2 MI0000281
<400> 128
   aggaagcuuc uggagauccu gcuccgucgc cccaguguuc agacuaccug uucaggacaa 60
   ugccguugua caguagucug cacauugguu agacugggca agggagagca 110
<210> 129
   <211> 110
   <212> RNA
   <213> homo sapiens
   <223> hsa-mir-199b MI0000282
<400> 129
   ccagaggaca ccuccacucc gucuacccag uguuuagacu aucuguucag gacucccaaa 60
   uuguacagua gucugcacau ugguuaggcu gggcuggguu agacccucgg 110
<210> 130
   <211> 110
   <212> RNA
   <213> homo sapiens
   <223> hsa-mir-210 MI0000286
<400> 130
   acccggcagu gccuccaggc gcagggcagc cccugcccac cgcacacugc gcugccccag 60
   acccacugug cgugugacag cggcugaucu gugccugggc agcgcgaccc 110
<210> 131
   <211> 110
   <212> RNA
   <213> homo sapiens
   <223> hsa-mir-221 MI0000298
<400> 131
   ugaacaucca ggucuggggc augaaccugg cauacaaugu agauuucugu guucguuagg 60
   caacagcuac auugucugcu ggguuucagg cuaccuggaa acauguucuc 110
<210> 132
   <211> 95
   <212> RNA
   <213> homo sapiens
   <223> hsa-mir-340 MI0000802
<400> 132
   uuguaccugg ugugauuaua aagcaaugag acugauuguc auaugucguu ugugggaucc 60
   gucucaguua cuuuauagcc auaccuggua ucuua 95
<210> 133
   <211> 98
   <212> RNA
   <213> homo sapiens
   <223> hsa-mir-424 MI0001446
<400> 133
   cgaggggaua cagcagcaau ucauguuuug aaguguucua aaugguucaa aacgugaggc 60
   gcugcuauac ccccucgugg ggaagguaga aggugggg 98
<210> 134
   <211> 94
   <212> RNA
   <213> homo sapiens
   <223> hsa-mir-575 MI0003582
<400> 134
   aauucagccc ugccacuggc uuaugucaug accuugggcu acucaggcug ucugcacaau 60
   gagccaguug gacaggagca gugccacuca acuc 94
<210> 135
   <211> 98
   <212> RNA
   <213> homo sapiens
   <223> hsa-mir-582 MI0003589
<400> 135
   aucugugcuc uuugauuaca guuguucaac caguuacuaa ucuaacuaau uguaacuggu 60
   ugaacaacug aacccaaagg gugcaaagua gaaacauu 98
<210> 136
   <211> 118
   <212> RNA
   <213> homo sapiens
   <223> hsa-mir-671 MI0003760
<400> 136
   gcaggugaac uggcaggcca ggaagaggag gaagcccugg aggggcugga ggugauggau 60
   guuuuccucc gguucucagg gcuccaccuc uuucgggccg uagagccagg gcuggugc 118
<210> 137
   <211> 69
   <212> RNA
   <213> homo sapiens
   <223> hsa-mir-1229 MI0006319
<400> 137
   guggguaggg uuugggggag agcgugggcu gggguucagg gacacccucu caccacugcc 60
   cucccacag 69
<210> 138
   <211> 78
   <212> RNA
   <213> homo sapiens
   <223> hsa-mir-1267 MI0006404
<400> 138
   cucccaaauc uccuguugaa guguaauccc caccuccagc auuggggauu acauuucaac 60
   augagauuug gaugagga 78
<210> 139
   <211> 74
   <212> RNA
   <213> homo sapiens
   <223> hsa-let-7a-3 MI0000062
<400> 139
   gggugaggua guagguugua uaguuugggg cucugcccug cuaugggaua acuauacaau 60
   cuacugucuu uccu 74
<210> 140
   <211> 79
   <212> RNA
   <213> homo sapiens
   <223> hsa-let-7e MI0000066
<400> 140
   cccgggcuga gguaggaggu uguauaguug aggaggacac ccaaggagau cacuauacgg 60
   ccuccuagcu uuccccagg 79
<210> 141
   <211> 85
   <212> RNA
   <213> homo sapiens
   <223> hsa-mir-22 MI0000078
<400> 141
   ggcugagccg caguaguucu ucaguggcaa gcuuuauguc cugacccagc uaaagcugcc 60
   aguugaagaa cuguugcccu cugcc 85
<210> 142
   <211> 64
   <212> RNA
   <213> homo sapiens
   <223> hsa-mir-29a MI0000087
<400> 142
   augacugauu ucuuuuggug uucagaguca auauaauuuu cuagcaccau cugaaaucgg 60
   uuau 64
<210> 143
   <211> 81
   <212> RNA
   <213> homo sapiens
   <223> hsa-mir-29b-1 MI0000105
<400> 143
   cuucaggaag cugguuucau auggugguuu agauuuaaau agugauuguc uagcaccauu 60
   ugaaaucagu guucuugggg g 81
<210> 144
   <211> 70
   <212> RNA
   <213> homo sapiens
   <223> hsa-mir-32 MI0000090
<400> 144
   ggagauauug cacauuacua aguugcaugu ugucacggcc ucaaugcaau uuagugugug 60
   ugauauuuuc 70
<210> 145
   <211> 96
   <212> RNA
   <213> homo sapiens
   <223> hsa-mir-33b MI0003646
<400> 145
   gcgggcggcc ccgcggugca uugcuguugc auugcacgug ugugaggcgg gugcagugcc 60
   ucggcagugc agcccggagc cggccccugg caccac 96
<210> 146
   <211> 110
   <212> RNA
   <213> homo sapiens
   <223> hsa-mir-34a MI0000268
<400> 146
   ggccagcugu gaguguuucu uuggcagugu cuuagcuggu uguugugagc aauaguaagg 60
   aagcaaucag caaguauacu gcccuagaag ugcugcacgu uguggggccc 110
<210> 147
   <211> 96
   <212> RNA
   <213> homo sapiens
   <223> hsa-mir-92b MI0003560
<400> 147
   cgggccccgg gcgggcggga gggacgggac gcggugcagu guuguuuuuu cccccgccaa 60
   uauugcacuc gucccggccu ccggcccccc cggccc 96
<210> 148
   <211> 81
   <212> RNA
   <213> homo sapiens
   <223> hsa-mir-95 MI0000097
<400> 148
   aacacagugg gcacucaaua aaugucuguu gaauugaaau gcguuacauu caacggguau 60
   uuauugagca cccacucugu g 81
<210> 149
   <211> 81
   <212> RNA
   <213> homo sapiens
   <223> hsa-mir-99a MI0000101
<400> 149
   cccauuggca uaaacccgua gauccgaucu uguggugaag uggaccgcac aagcucgcuu 60
   cuaugggucu gugucagugu g 81
<210> 150
   <211> 86
   <212> RNA
   <213> homo sapiens
   <223> hsa-mir-125a MI0000469
<400> 150
   ugccagucuc uaggucccug agacccuuua accugugagg acauccaggg ucacagguga 60
   gguucuuggg agccuggcgu cuggcc 86
<210> 151
   <211> 88
   <212> RNA
   <213> homo sapiens
   <223> hsa-mir-125b-1 MI0000446
<400> 151
   ugcgcuccuc ucagucccug agacccuaac uugugauguu uaccguuuaa auccacgggu 60
   uaggcucuug ggagcugcga gucgugcu 88
<210> 152
   <211> 89
   <212> RNA
   <213> homo sapiens
   <223> hsa-mir-125b-2 MI0000470
<400> 152
   accagacuuu uccuaguccc ugagacccua acuugugagg uauuuuagua acaucacaag 60
   ucaggcucuu gggaccuagg cggagggga 89
<210> 153
   <211> 72
   <212> RNA
   <213> homo sapiens
   <223> hsa-mir-129-1 MI0000252
<400> 153
   ggaucuuuuu gcggucuggg cuugcuguuc cucucaacag uagucaggaa gcccuuaccc 60
   caaaaaguau cu 72
<210> 154
   <211> 90
   <212> RNA
   <213> homo sapiens
   <223> hsa-mir-129-2 MI0000473
<400> 154
   ugcccuucgc gaaucuuuuu gcggucuggg cuugcuguac auaacucaau agccggaagc 60
   ccuuacccca aaaagcauuu gcggagggcg 90
<210> 155
   <211> 82
   <212> RNA
   <213> homo sapiens
   <223> hsa-mir-136 MI0000475
<400> 155
   ugagcccucg gaggacucca uuuguuuuga ugauggauuc uuaugcucca ucaucgucuc 60
   aaaugagucu ucagaggguu cu 82
<210> 156
   <211> 99
   <212> RNA
   <213> homo sapiens
   <223> hsa-mir-148b MI0000811
<400> 156
   caagcacgau uagcauuuga ggugaaguuc uguuauacac ucaggcugug gcucucugaa 60
   agucagugca ucacagaacu uugucucgaa agcuuucua 99
<210> 157
   <211> 87
   <212> RNA
   <213> homo sapiens
   <223> hsa-mir-152 MI0000462
<400> 157
   uguccccccc ggcccagguu cugugauaca cuccgacucg ggcucuggag cagucagugc 60
   augacagaac uugggcccgg aaggacc 87
<210> 158
   <211> 110
   <212> RNA
   <213> homo sapiens
   <223> hsa-mir-181c MI0000271
<400> 158
   cggaaaauuu gccaaggguu ugggggaaca uucaaccugu cggugaguuu gggcagcuca 60
   ggcaaaccau cgaccguuga guggacccug aggccuggaa uugccauccu 110
<210> 159
   <211> 92
   <212> RNA
   <213> homo sapiens
   <223> hsa-mir-191 MI0000465
<400> 159
   cggcuggaca gcgggcaacg gaaucccaaa agcagcuguu gucuccagag cauuccagcu 60
   gcgcuuggau uucguccccu gcucuccugc cu 92
<210> 160
   <211> 88
   <212> RNA
   <213> homo sapiens
   <223> hsa-mir-193a MI0000487
<400> 160
   cgaggauggg agcugagggc ugggucuuug cgggcgagau gagggugucg gaucaacugg 60
   ccuacaaagu cccaguucuc ggcccccg 88
<210> 161
   <211> 110
   <212> RNA
   <213> homo sapiens
   <223> hsa-mir-223 MI0000300
<400> 161
   ccuggccucc ugcagugcca cgcuccgugu auuugacaag cugaguugga cacuccaugu        60
   gguagagugu caguuuguca aauaccccaa gugcggcaca ugcuuaccag                  110
<210> 162
   <211> 86
   <212> RNA
   <213> homo sapiens
   <223> hsa-mir-301a MI0000745
<400> 162
   acugcuaacg aaugcucuga cuuuauugca cuacuguacu uuacagcuag cagugcaaua        60
   guauugucaa agcaucugaa agcagg                                              86
<210> 163
   <211> 94
   <212> RNA
   <213> homo sapiens
   <223> hsa-mir-335 MI0000816
<400> 163
   uguuuugagc gggggucaag agcaauaacg aaaaauguuu gucauaaacc guuuuucauu        60
   auugcuccug accuccucuc auuugcuaua uuca                                     94
<210> 164
   <211> 67
   <212> RNA
   <213> homo sapiens
   <223> hsa-mir-338 MI0000814
<400> 164
   ucuccaacaa uauccuggug cugagugaug acucaggcga cuccagcauc agugauuuug        60
   uugaaga                                                                   67
<210> 165
   <211> 65
   <212> RNA
   <213> homo sapiens
   <223> hsa-mir-362 MI0000762
<400> 165
   cuugaauccu uggaaccuag gugugagugc uauuucagug caacacaccu auucaaggau 60
   ucaaa 65
<210> 166
   <211> 67
   <212> RNA
   <213> homo sapiens
   <223> hsa-mir-371a MI0000779
<400> 166
   guggcacuca aacugugggg gcacuuucug cucucuggug aaagugccgc caucuuuuga 60
   guguuac 67
<210> 167
   <211> 69
   <212> RNA
   <213> homo sapiens
   <223> hsa-mir-373 MI0000781
<400> 167
   gggauacuca aaaugggggc gcuuuccuuu uugucuguac ugggaagugc uucgauuuug 60
   ggguguccc 69
<210> 168
   <211> 68
   <212> RNA
   <213> homo sapiens
   <223> hsa-mir-376a-1 MI0000784
<400> 168
   uaaaagguag auucuccuuc uaugaguaca uuauuuauga uuaaucauag aggaaaaucc 60
   acguuuuc 68
<210> 169
   <211> 80
   <212> RNA
   <213> homo sapiens
   <223> hsa-mir-376a-2 MI0003529
<400> 169
   gguauuuaaa agguagauuu uccuucuaug guuacguguu ugaugguuaa ucauagagga 60
   aaauccacgu uuucaguauc 80
<210> 170
   <211> 69
   <212> RNA
   <213> homo sapiens
   <223> hsa-mir-377 MI0000785
<400> 170
   uugagcagag guugcccuug gugaauucgc uuuauuuaug uugaaucaca caaaggcaac 60
   uuuuguuug 69
<210> 171
   <211> 94
   <212> RNA
   <213> homo sapiens
   <223> hsa-mir-423 MI0001445
<400> 171
   auaaaggaag uuaggcugag gggcagagag cgagacuuuu cuauuuucca aaagcucggu 60
   cugaggcccc ucagucuugc uuccuaaccc gcgc 94
<210> 172
   <211> 87
   <212> RNA
   <213> homo sapiens
   <223> hsa-mir-425 MI0001448
<400> 172
   gaaagcgcuu uggaaugaca cgaucacucc cguugagugg gcacccgaga agccaucggg 60
   aaugucgugu ccgcccagug cucuuuc 87
<210> 173
   <211> 84
   <212> RNA
   <213> homo sapiens
   <223> hsa-mir-505 MI0003190
<400> 173
   gaugcaccca gugggggagc caggaaguau ugauguuucu gccaguuuag cgucaacacu 60
   ugcugguuuc cucucuggag cauc 84
<210> 174
   <211> 78
   <212> RNA
   <213> homo sapiens
   <223> hsa-mir-543 MI0005565
<400> 174
   uacuuaauga gaaguugccc guguuuuuuu cgcuuuauuu gugacgaaac auucgcggug 60
   cacuucuuuu ucaguauc 78
<210> 175
   <211> 86
   <212> RNA
   <213> homo sapiens
   <223> hsa-mir-548m MI0006400
<400> 175
   auauuagguu ggugcaaagg uauuuguggu uuuugucauu aaaguaaugc aaaagccaca 60
   aauaccuuug caccaaccua auauua 86
<210> 176
   <211> 98
   <212> RNA
   <213> homo sapiens
   <223> hsa-mir-557 MI0003563
<400> 176
   agaaugggca aaugaacagu aaauuuggag gccuggggcc cucccugcug cuggagaagu 60
   guuugcacgg gugggccuug ucuuugaaag gaggugga 98
<210> 177
   <211> 79
   <212> RNA
   <213> homo sapiens
   <223> hsa-mir-563 MI0003569
<400> 177
   agcaaagaag uguguugccc ucuaggaaau guguguugcu cugauguaau uagguugaca 60
   uacguuuccc ugguagcca 79
<210> 178
   <211> 97
   <212> RNA
   <213> homo sapiens
   <223> hsa-mir-584 MI0003591
<400> 178
   uagggugacc agccauuaug guuugccugg gacugaggaa uuugcuggga uaugucaguu 60
   ccaggccaac caggcugguu ggucucccug aagcaac 97
<210> 179
   <211> 98
   <212> RNA
   <213> homo sapiens
   <223> hsa-mir-602 MI0003615
<400> 179
   uucucacccc cgccugacac gggcgacagc ugcggcccgc uguguucacu cgggccgagu 60
   gcgucuccug ucaggcaagg gagagcagag ccccccug 98
<210> 180
   <211> 97
   <212> RNA
   <213> homo sapiens
   <223> hsa-mir-634 MI0003649
<400> 180
   aaacccacac cacugcauuu uggccaucga ggguuggggc uuggugucau gccccaagau 60
   aaccagcacc ccaacuuugg acagcaugga uuagucu 97
<210> 181
   <211> 109
   <212> RNA
   <213> homo sapiens
   <223> hsa-mir-767 MI0003763
<400> 181
   gcuuuuauau uguagguuuu ugcucaugca ccaugguugu cugagcaugc agcaugcuug 60
   ucugcucaua ccccaugguu ucugagcagg aaccuucauu gucuacugc 109
<210> 182
   <211> 81
   <212> RNA
   <213> homo sapiens
   <223> hsa-mir-876 MI0005542
<400> 182
   ugaagugcug uggauuucuu ugugaaucac cauaucuaag cuaauguggu ggugguuuac 60
   aaaguaauuc auagugcuuc a 81
<210> 183
   <211> 77
   <212> RNA
   <213> homo sapiens
   <223> hsa-mir-933 MI0005755
<400> 183
   acuuggguca guucagaggu ccucggggcg cgcgucgagu cagccgugug cgcagggaga 60
   ccucucccac ccacagu 77
<210> 184
   <211> 90
   <212> RNA
   <213> homo sapiens
   <223> hsa-mir-1225 MI0006311
<400> 184
   guggguacgg cccagugggg gggagaggga cacgcccugg gcucugccca gggugcagcc 60
   ggacugacug agccccugug ccgcccccag 90
<210> 185
   <211> 84
   <212> RNA
   <213> homo sapiens
   <223> hsa-mir-1234 MI0006324
<400> 185
   gugagugugg gguggcuggg gggggggggg gggggccggg gacggcuugg gccugccuag 60
   ucggccugac cacccacccc acag 84
<210> 186
   <211> 102
   <212> RNA
   <213> homo sapiens
   <223> hsa-mir-1237 MI0006327
<400> 186
   gugggagggc ccaggcgcgg gcaggggugg ggguggcaga gcgcuguccc gggggcgggg 60
   ccgaagcgcg gcgaccguaa cuccuucugc uccguccccc ag 102
<210> 187
   <211> 83
   <212> RNA
   <213> homo sapiens
   <223> hsa-mir-1238 MI0006328
<400> 187
   gugaguggga gccccagugu gugguugggg ccauggcggg ugggcagccc agccucugag 60
   ccuuccucgu cugucugccc cag 83
<210> 188
   <211> 85
   <212> RNA
   <213> homo sapiens
   <223> hsa-mir-1244-1 MI0006379
<400> 188
   aucuuauucc gagcauucca guaacuuuuu uguguaugua cuuagcugua cuauaaguag 60
   uugguuugua ugagaugguu aaaaa 85
<210> 189
   <211> 85
   <212> RNA
   <213> homo sapiens
   <223> hsa-mir-1244-2 MI0015974
<400> 189
   aucuuauucc gagcauucca guaacuuuuu uguguaugua cuuagcugua cuauaaguag 60
   uugguuugua ugagaugguu aaaaa 85
<210> 190
   <211> 85
   <212> RNA
   <213> homo sapiens
   <223> hsa-mir-1244-3 MI0015975
<400> 190
   aucuuauucc gagcauucca guaacuuuuu uguguaugua cuuagcugua cuauaaguag 60
   uugguuugua ugagaugguu aaaaa 85
<210> 191
   <211> 53
   <212> RNA
   <213> homo sapiens
   <223> hsa-mir-1825 MI0008193
<400> 191
   agagacuggg gugcugggcu ccccuagacu aggacuccag ugcccuccuc ucc 53

## Description

### FIELD OF THE PRESENT INVENTION

This disclosure generally relates to immunology and more specifically to methods and compositions for characterizing individuals or groups of individuals using patterns of expression of one or more microRNA sequences.

### BACKGROUND OF THE INVENTION

Physicians are faced with diagnostic as well as therapeutic decisions. Execution of these decisions requires categorizing patients into diagnostic and therapeutic groups. Many vehicles for such decisions are available to the physician. However, no single grouping of methods are universally successful. There is continued need for novel methods for segregation of patients into diagnostic and therapeutic categories.

A class of short regulatory RNA molecules known as microRNA has been found to have profound effects on subsequent translation of mRNA into gene products. Short single-stranded polymers of ribonucleic acids found in eukaryotic cells act post-transcriptionally, binding to substantially complementary sequences on messenger RNA transcripts. Regulatory effects usually through translational repression, mRNA degradation and gene silencing. Morozova N. , Zinovyev A. , Nonne N., Pritchard L.-L., Gorban A.N., and Harel-Bellan A. (September 2012). "Kinetic signatures of microRNA modes of action". RNA 18 (9): 1635-1655) Complementarity to the target mRNA in metazoans is incomplete but typically encompasses a sequence comprising bases 2-7, the "seed sequence". The resulting low stringency requirement permits miRNAs to target more than a single mRNA. Pri-miRNA sequences may be found in a variety of preferred sites throughout the genome. Their location, in part, guides their selective transcription. Many are found in intergenic regions or anti-sense to a neighboring-regulated gene. ("Kinetic signatures of microRNA modes of action". RNA 18 (9): 1635-1655. Lee Y, Kim M, Han J, Yeom KH, Lee S, Baek SH, Kim VN (October 2004). "MicroRNA genes are transcribed by RNA polymerase II". EMBO J. 23 (20): 4051-60.)

MicroRNAs may also be transcribed together with the target mRNA thereby permitting coupling regulation of both the microRNA and the target protein-encoding gene. Mraz, M.; Dolezalova, D.; Plevova, K.; Stano Kozubik, K.; Mayerova, V.; Cerna, K.; Musilova, K.; Tichy, B. et al. (2012). "MicroRNA-650 expression is influenced by immunoglobulin gene rearrangement and affects the biology of chronic lymphocytic leukemia". Blood 119 (9): 2110-2113.) A large fraction of them lie within intronic sequences, protein-encoding and non-protein encoding transcripts as well as within exons of non-protein encoding transcripts. (Rodriguez A, Griffiths-Jones S, Ashurst JL, Bradley A (October 2004). "Identification of mammalian microRNA host genes and transcription units". Genome Res. 14 (10A): 1902-10.) Coordinate regulation may also be exerted through the use of common promoter sequences. (Lee Y, Kim M, Han J, Yeom KH, Lee S, Baek SH, Kim VN (October 2004). "MicroRNA genes are transcribed by RNA polymerase II". EMBO J. 23 (20): 4051-60.) (Altuvia Y, Landgraf P, Lithwick G, Elefant N, Pfeffer S, Aravin A, Brownstein MJ, Tuschl T, Margalit H (2005). "Clustering and conservation patterns of human microRNAs". Nucleic Acids Res. 33 (8): 2697-706.)

MicroRNA expression can represent organism-wide gene regulation or represent organ-limited gene regulation. Plasma or serum based assays have been an appealing area of investigation. An example is the release of microRNA into plasma in a variety of different RNase-resistant forms that permits their interrogation through peripheral blood collection. (Mitchell et al., Circulating microRNAs as stable blood-based markers for cancer detection, PNAS (2008)105 (30): 10513-10518. Vishnu Swarup, M.R. Rajeswari, Circulating (cell-free) nucleic acids - A promising, non-invasive tool for early detection of several human diseases, FEBS Letters 581 (2007) 795-799.) Placental microRNA expression can be used as an example to illustrate an area of active study in organ-limited gene regulation. (Chim SSC, Detection and Characterization of Placental MicroRNAs in Maternal Plasma, Clinical Chemistry, (2008) 54(3): 482-490.) It is the hope of investigators to characterize and quantify "placenta-associated" miRNAs with pregnancy-associated diseases. (Gilad S, Meiri E, Yogev Y, Benjamin S, Lebanony D, et al. (2008) Serum MicroRNAs Are Promising Novel Biomarkers. PLoS ONE 3(9): e3148. doi:10.1371/journal.pone.0003148. Miura K, Identification of Pregnancy-Associated MicroRNAs in Maternal Plasma, Clinical Chemistry (2010) 56(11): 1767-1771.)

While serum and plasma comprise organ and tissue-specific miRNAs as a result of their release, miRNAs present in cells circulating in blood constitute and entirely separate and measurable pool. Such cellular material constitutes a compartment of the immune system. Interrogation of blood-borne cells for their miRNA constituents provides insights regarding immune function. Separation of blood-borne cellular constituents such as lymphocytes, monocytes, neutrophils or other distinct populations permits further insight into behavior of these separate constituents and related biology. Further segregation of blood-borne cellular constituents, in particular but not limited to lymphocytes, by surface and other distinguishing markers permits further characterization.

The utility of interrogation of blood-borne cells for their expression of microRNAs is clearly demonstrated in identification of patterned alterations in the quantities of microRNA associated with various types of inflammation and either degrees response or lack of response to treatment. Tang et al. interrogated peripheral blood mononuclear cells (PBMCs) for miRNA-146a. They found underexpression of this microRNA in PBMCs of patients with lupus erythematosus (SLE). (Tang Y et al., MicroRNA-146a Contributes to Abnormal Activation of the Type I Interferon Pathway in Human Lupus by Targeting the Key Signaling Proteins, Arthritis and Rheumatism, (2009) 60(4): 1065-1075.) Mir-146a has been shown to be an important regulator of innate immune function, a negative regulator of type I interferon (IFN I). (Taganov KD, Boldin MP, Chang KJ, Baltimore D. NF-'B- dependent induction of microRNA miR-146, an inhibitor targeted to signaling proteins of innate immune responses. Proc Natl Acad Sci U S A 2006;103:12481-6.) Thus, interrogation of blood cells, in particular, PBMCs provides information separable from corresponding interrogation of serum or plasma.

The use of blood markers, either within plasma/serum or cellular constituents, in clinical practice is well-established. Clinicians must respond to patient care issues whether they are preventive or pathologically based with an appropriate and graded response. The clinician must arrive at a clinical question and determine the most appropriate means of diagnosis and treatment. If a particular treatment is thought necessary, it is necessary to predict the efficacy of the selected therapy and make appropriate judgments as to further assessment and finally to prognostication. It is recognized that treatments may be costly, involve inconvenience, discomfort and risk. In addition, lack of treatment may involve cost, discomfort and risk. Therefore, an appropriate level of assessment must precede a therapeutic intervention. Interventions, themselves assessed as a component of response, may further permit diagnostic assessment and prognostication.

Quantification of selected microRNAs in the cells of the immune system, preferably in peripheral blood, has certain advantages over conventional diagnostic modalities. Through selection of characterized microRNA markers, it may permit assessment of mechanisms of pathology not easily or practically determined by other clinically available means. Such techniques are presently under investigation.

It is now appreciated that MicroRNAs substantially regulate translation of mRNA into its final protein product. Increased expression of a microRNA targeting a specific mRNA diminishes its translation into the polynucleotide encoded by the mRNA. In metazoan species, the approximately 22 base sequence of a microRNA is usually incompletely complementary to the target region within an mRNA. Moreover, full complementarity is confined to a short sequence located at bases 2 through 7 of the microRNA strand. This low fidelity permits targeting of a plurality of mRNAs. Thus the expression of a single microRNA may have broad regulatory effect on a cluster of mRNAs. Advantages accrue to the evolutionary accumulation of mRNA sequences comprising such target sequences. An example of such a strategy involves miR-144. NRF2 is a central regulator of the anti-oxidant response. NRF2 is a transcription factor that binds to an anti-oxidant response element (ARE) expressed in the mRNAs of superoxide dismutase (SOD), catalase (CAT), phase II detoxification enzymes NAD(P)H:quinone oxidoreductase (NQO1) and glutathione (GSH) synthese enzymes for example. NRF2-regulates expression of these ARE-comprising genes. miR-144 regulates NRF2 where increased miR-144 expression results in diminished expression of NRF2 and diminished expression of anti-oxidant genes. (Carolyn Sangokoya, Marilyn J. Telen, and Jen-Tsan Chi, microRNA miR-144 modulates oxidative stress tolerance and associates with anemia severity in sickle cell disease, Blood November 18, 2010 vol. 116 no. 20 4338-4348.)

Identification of patients in whom an immunotherapy is contemplated is important. In addition to disorders of reproduction, a wide variety of other conditions have been shown or are under study or may come under study in the future that may benefit from immunotherapy. In a similar manner, this invention can be applied to assessing selection of therapy, dosing, and efficacy of immunotherapy in these conditions. Exemplary of these conditions are diseases in which an inflammatory component is thought to play a role in pathogenesis. For diverse condtions immunotherapy is commonly provided, preferably IVIg. For example, these include Allogeneic bone marrow transplant, Chronic lymphocytic leukemia, Common variable immunodeficiency (CVID) a group of approximately 150 primary immunodeficiencies (PIDs), which have a common set of features (including hypogammaglobulinemia) but which have different underlying causes, Idiopathic thrombocytopenic purpura, Pediatric HIV, Primary immunodeficiencies, Kawasaki disease, Chronic inflammatory demyelinating polyneuropathy (CIDP) Kidney transplant with a high antibody recipient or with an ABO incompatible donor, Alzheimer's disease, Autism, Capillary leak syndrome, Chronic fatigue syndrome, Clostridium difficile colitis, Dermatomyositis and polymyositis, Graves' ophthalmopathy, Guillain-Barré syndrome, Muscular Dystrophy, Inclusion body myositis, Infertility, Lambert-Eaton syndrome, Lupus erythematosus, Multifocal motor neuropathy, Multiple sclerosis, Myasthenia gravis, Neonatal alloimmune thrombocytopenia, Parvovirus B19, Pemphigus, Post-transfusion purpura, Renal transplant rejection, Spontaneous Abortion/Miscarriage, Sjogren's Syndrome, Stiff person syndrome, Opsoclonus Myoclonus, Severe sepsis and septic shock in critically ill adults[14], Toxic epidermal necrolysis, In chronic lymphocytic leukemia and multiple myelomaas well as various rare deficiencies of immunoglobulin synthesis (e.g. X-linked agammaglobulinemiahypogammaglobulinemia)IVIG is administered to maintain adequate immunoglobulin levels to prevent infections. The present invention is contemplated to be useful in the management of such conditions in a manner similar to that described for reproductive disorders.

The therapeutic trial of an intervention may reveal features of the complex interaction of disease, host and intervention not discernible through conventional diagnostic testing. Intravenously administered pooled-donor gamma globulin (IVIg) empirically has been found effective in treatment of a broad spectrum of immunologic conditions. A comparatively broad range of mechanisms for its action has also been proposed. These include anti-idiotypic antibody mediated autoantibody neutralization, mononuclear phagocytic system blockade, sialylated-Fc receptor mediated interaction with dendritic cells are amongst a few mechanisms advanced to explain the many therapeutic effects of IVIg administration. It is probable that no single mechanism accounts for its efficacy in the many conditions empirically found responsive. Response may not be predictable from the clinical presentation or laboratory characterization of the disease process. Separate and distinguishable patient characteristics may be important in shaping a patient-specific response to an intervention. Identification of such therapy-specific responses may improve patient selection and tailoring of therapy.

Infertility and pregnancy failure is a vexing problem faced by couples who want to raise families. Spontaneous abortion occurs in 15 percent of pregnancies. Recurrent spontaneous abortion, defined as the loss of at least three consecutive pregnancies under 24 weeks gestation, occurs in 3-4 percent of women. In addition, very early losses may go unrecognized in couples thought to be infertile where no diagnostic test has confirmed the existence of a transient pregnant state. Still other women suffer from pregnancy complications such as pre-eclampsia, intrauterine growth retardation (IUGR), preterm labor, premature rupture of membranes (PROM) and stillbirth. Childhood conditions like asthma, autism, attention deficit hyperactivity disorder (ADHD), diabetes, schizophrenia and Tourette's syndrome have been associated with pregnancy related disorders and the late complications of pregnancy-related disorders according to recent literature.

A significant body of literature now exists associating a number of these conditions with immunologic dysfunction. Immunologic intervention has been shown to improve reproductive success in women who have immunological conditions such as low levels of T regulatory cells, elevated natural killer cells and high TNF-alpha/Il-10 cell T-helper ratios as determined by in vitro assays. Further it has been shown that immunotherapeutic intervention is helpful in specific subgroups of those afflicted. For example, intravenous immunoglobulin (IVIg) has been useful to reduce natural killer cell numbers in a subset of women in whom in vitro measurements of immune function are elevated. This immunotherapeutic intervention has been shown to improve reproductive success in these women.JAMA (1995) 273:1933-36 Hum Reprod (1998) 13(9):2620-3, Am J Reprod Immunol (2002) 38(5):312-18, Ann NY Acad Sci (2005) 1051:743-78. In addition, heparin has been shown to reduce miscarriage rates in those with elevated antiphospholipid antibodies (Salmon JE, Girardi G. Antiphospholipid antibodies and pregnancy loss: an inflammatory disorder. Reprod Immunol. 2008 Jay;77(1):51-6. Epub 2007 Apr 5.Review.) Many reproductive conditions that have previously been considered to be "unexplained" may be immunologically caused.

Amongst immunologic cells including lymphocytes, monocytes (including their derivatives macrophages and dendritic cells) various lymphoid subpopulations, alternatively known as subsets, mediate and regulate immunologic cytotoxicity. These cells may include NK cells, NKT cells, CD8 T cells, CD4 T cells, gamma delta T cells T regulatory cells and Th17 cells. It has been suggested that the activity of such subsets may have particular significance in defining populations of women suffering from or at risk of suffering from reproductive failure who might benefit from immunotherapy. In addition, assays quantifying the numbers and activity of such cells have been helpful in monitoring such immunotherapy. In the clinical setting, the immunologic cells cited above are collected from the peripheral blood compartment and assessed.

For example, one area of clinical interest has been the T helper lymphocytes (CD3+/CD4+). These cells can be classified into subpopulations according to cytokine profiles revealed following in vitro stimulation as either T helper 1 (Th1) or T helper 2 (Th2) cells. T helper cells selectively secrete specific clusters of cytokines. For example, Th2 cells produce interleukins, IL-4, IL-5, IL-6, IL-9, IL-10 and IL-13, that, in turn, are involved in the development of humoral immunity against extracellular pathogens but inhibit several functions of phagocytic cells. Th1 cells, alternatively, produce interferon-gamma (IFN-gamma.), IL-2 and tumor necrosis factor-alpha (TNF-alpha). These cytokines are involved cell-mediated immunity and phagocyte dependent inflammation (Mosmann & Coffman, 1989; Romagnani, 2000).

Timely intervention with anticoagulants prevent many of these losses. Another area of clinical interest has been complement-mediated pregnancy loss. (Cohen D, Buurma A, Goemaere NN, Girardi G, le Cessie S, Scherjon S, Bloemenkamp KW, de Heer E, Bruijn JA, Bajema IM. Classical complement activation as a footprint for murine and human antiphospholipid antibody-induced fetal loss. (J Pathol. (2011) : 502-511. Yu G, Sun Y, Foerster K, Manuel J, Molina H, Levy GA, Gorczynski RM, Clark DA.LPS-induced murine abortions require C5 but not C3, and are prevented by upregulating expression of the CD200 tolerance signaling molecule. Am J Reprod Immunol. 2008 Aug;60(2):135-40.)

Raghupathy observed significantly higher serum levels of Th2 cytokines, IL-6 and IL-10, in normal pregnancy compared to unexplained recurrent pregnancy losses. Further, significantly higher serum levels of the Th1 cytokine, IFN-gamma, were present in women with recurrent pregnancy losses compared to normal pregnancy (Raghupathy et al., 1999). Together, these observations suggest a Th2 bias in women with normal pregnancies while a Th1 bias exists in many women with a history of recurrent pregnancy loss, unexplained infertility and pregnancy complications as, for example, delineated above.

Alan Beer reasoned that rebalancing the Th1/Th2 ratio of women with a Th1 bias toward a Th2 bias might help them achieve greater reproductive success. He proposed that treatment of such women with anti-TNF alpha agents would result in shifting the Th1/Th2 balance toward a Th2 bias.

To identify candidates for anti-TNF alpha therapy, Beer proposed that in vitro assessment of the proportions of CD4 expressing T cells differentiated toward producing cytokines of either the Th1 or Th2 groupings could be conducted. If a patient with a reproductive disorder, in particular the issues of unexplained infertility and recurrent unexplained abortion, demonstrated an elevation in the Th1/Th2 ratio compared with normal control patients, then the patient is considered a candidate for anti-TNF alpha therapy. Beer proposed that patients under treatment undergo repeated assessments of the Th1/Th2 ratio to assess efficacy of the therapy. Effective therapy, he reasoned, should result in a shift in the Th1/Th2 ratio toward a Th2 bias. Winger, Reed et al. have shown that women with preconception Th1/Th2 ratio elevation do indeed demonstrate improved reproductive success when treated with anti-TNF alpha therapy over similar women who do not undergo such therapy. Moreover, the treatment period was found to be effective even when the treatment period concluded prior to implantation. (Reference 1: Edward E. Winger, Jane L. Reed, Treatment with Tumor Necrosis Factor Inhibitors and Intravenous Immunoglobulin Improves Live Birth Rates in Women with Recurrent Spontaneous Abortion, 60(1), 8 - 16, Published Online: 28 Jun 2008. Reference 2: Edward E. Winger, Jane L. Reed, Sherif Ashoush, Sapna Ahuja, Tarek El-Toukhy, Mohamed Taranissi, Treatment with Adalimumab (Humira and Intravenous Immunoglobulin Improves Pregnancy Rates in Women Undergoing IVF, American Journal of Reproductive Immunology 61 (2009) 113-120)).

The method of Beer has received significant criticism. One such criticism is articulated by Chaouat who notes that implantation of the embryo into the uterine lining is an inflammatory event. (Gérard Chaouat, Natalie Ledée-Bataille, Sylvie Dubanchet, Sandrine Zourbas, Olivier Sandra, Jacques Marta, Th1/Th2 Paradigm in Pregnancy: Paradigm Lost?, Cytokines in Pregnancy/Early Abortion: Reexamining the Th1/Th2 Paradigm, Int Arch Allergy Immunol 2004;134:93-119) Predominance of Th1 cytokines at the time and place of implantation is-essential to the process thereby identifying a deficiency in the Th1/Th2 hypothesis. Moreover, anti-TNF alpha therapy, as suggested in the patent application of Kwak Kim et al. (USPTO # 20040105858) might result in diminished reproductive efficiency. Moreover, determination of the Th1/Th2 ratio at the site of implantation may not be effectively determined from the analysis of lymphocytes isolated from the peripheral blood. Another criticism that has been leveled at attempts to assay peripheral blood cells has been made by Moffett et al. They challenge the examination of peripheral blood white cells as non-representative of cellular events within the placenta. (Ashley Moffett, Lesley Regan, Peter Braude, Natural killer cells, miscarriage, and infertility, BMJ 2004;329:1283-5.) They conclude 1) "Uterine NK cells are different from those circulating in peripheral blood", 2) "Tests to measure NK cells in peripheral blood give no useful information on uterine NK cells" and 3) "Enthusiasm for new treatments aimed at natural killer cells in women with reproductive failure is unfortunately not backed up by the science".)

Anticoagulant therapy for antiphospholipid antibody related recurrent miscarriage has also received criticism. Antiphospholipid antibody tests have been criticized as unreliable and poorly standardized. (Lakos G, Favaloro EJ, Harris EN, Meroni PL, Tincani A, Wong RC, Pierangeli SS.International consensus guidelines on anticardiolipin and anti-â(2)glycoproteinI testing: A report from the APL task force at the 13(th) international congress on antiphospholipid antibodies. Arthritis Rheum. 2011 Sep 27. doi:10.1002/art.33349. Review.) In addition, it has been suggested that miscarriages associated with Antiphospholipid Antibody Syndrome are actually caused by increased complement activity rather than increased thrombotic activity (Salmon JE, Girardi G. Theodore E. Woodward Award: antiphospholipid syndrome revisited: a disorder initiated by inflammation. Trans Am Clin Climatol Assoc.2007;118:99-114.) (Lynch AM, Salmon JE. Dysregulated complement activation as a common pathway of injury in preeclampsia and other pregnancy complications. Placenta. 2010Jul;31(7):561-7. Epub 2010 Apr 27. Review.) MicroRNA markers may better identify the underlying inflammatory markers that respond well to anticoagulant treatment.

In addition, mechanisms of maternal tolerance of the fetal hemiallograft have invoked the interaction of another group of CD4 T cells known as T regulatory cells. Jasper et al. (Molecular Human Reproduction Vol.12, No.5 pp. 301-308, 2006) quantified FoxP3 mRNA, a master regulator of T regulatory cell differentiation, in mid-secretory endometrial tissues from women with unexplained infertility and levels from unaffected women. They found reduced levels of FoxP3 mRNA in affected women when compared with controls. Winger and Reed have found diminished levels of CD4+CD25+ Foxp3+ T regulatory cells in the peripheral blood lymphocytes of women experiencing recurrent abortion (Edward E. Winger, Jane L. Reed, Low Circulating CD4+ CD25+ Foxp3+ T Regulatory Cell Levels Predict Miscarriage Risk in Newly Pregnant Women with a History of Failure, Am J Reprod Immunol. 2011 Oct;66(4):320-8).

Th17 cells are now thought to play a role in the immunology of pregnancy (Shigeru Saito, Akitoshi Nakashima, Tomoko Shima, Mika Ito, Th1/Th2/Th17 and Regulatory T-Cell Paradigm in Pregnancy, American Journal of Reproductive Immunology 63 (2010) 601-610). Together with T regulatory cells, with which they appear to act in concert, they affect the balance in much the same manner as Th1 and Th2 cells have been proposed to affect pregnancy success.

Recently, a variety of conditions affecting infants born wherein the mother demonstrated immunologic abnormalities in or about the time of pregnancy have been described. For example, immunologic abnormalities during the course of pregnancy have been implicated a cause of autism. Autism is thought to be a spectrum of disorders the etiology of which remains unknown. However, immunological factors during early pregnancy have been invoked. Skewed Th1/Th2 cytokine profiles, altered lymphocyte numbers and decreased T cell mitogen responses have been identified in affected children. The authors suggest that immune abnormalities during early pregnancy may be involved (Paul Ashwood, Sharifia Wills, and Judy Van de Water, The immune response in autism: a new frontier for autism research, Journal of Leukocyte Biology. 2006;80:1-15)

Controversy regarding the Th1/Th2 hypothesis is also related to timing of testing and therapy. Chaouat, as noted above, teaches that the hypothesis is flawed and Th bias may not remain constant through the implantation period. Winger, Reed et al. show that patients with elevation in their Th1/Th2 cell ratio assessed in the preconception period enjoy significantly superior pregnancy results when treated with anti-TNF alpha therapy. (Winger EE, Reed JL, Ashoush S, El-Toukhy T, Ahuja S, Taranissi M. Degree of TNF-α/IL-10 cytokine elevation correlates with IVF success rates in women undergoing treatment with Adalimumab (Humira) and IVIG. Am J Reprod Immunol. 2011Jun;65(6):610-8) Winger and Reed have also demonstrated that T regulatory cells assessed during the period of implantation predict pregnancy outcome as well. (Winger EE, Reed JL. Low Circulating CD4(+) CD25(+) Foxp3(+) T Regulatory Cell Levels Predict Miscarriage Risk in Newly Pregnant Women with a History of Failure. Am J Reprod Immunol. 2011 Oct;66(4):320-8.) Timing and the nature of the laboratory parameters used appears quite significant.

In addition, Winger and Reed have identified a subset of patients in whom the behavior of the ratio was not as expected. Occasionally Th1/Th2 ratios rise following therapy while efficacy of therapy appears intact. (Winger EE, Reed JL, Ashoush S, El-Toukhy T, Ahuja S, Taranissi M. Degree of TNF-α/IL-10 cytokine elevation correlates with IVF success rates in women undergoing treatment with Adalimumab (Humira) and IVIG. Am J Reprod Immunol. 2011 Jun;65(6):610-8.)

Moreover, the technique for determining the Th1/Th2 ratio requires isolation of viable mononuclear cells from peripheral blood (PBMCs), stimulating them in vitro after having blocked cytokine secretion, a step that is somewhat toxic to the cells. Successful induction of intracellular cytokine expression may thus be compromised and test results theoretically less accurate. Further, while Th1 cell quantification appears relatively robust, Th2 quantification is hampered by its low prevalence amongst CD4 positive T cells. Gating of the cells as currently practiced can be somewhat subjective leading to imprecise results. Assay results, therefore may vary because of minor gating variations. For these and other reasons, an improved technique for assessing Th1 and Th2 numbers is needed.

In addition, the predictive power of the different assays currently being employed to assess pregnancy risk factors is not sufficiently sensitive to detect all affected individuals. Additional immune cell testing parameters are sorely needed. For example, currently two tests of natural killer cells are currently performed. The first is a phenotypic assay quantifying NK cells. Lymphocytes expressing CD56 but not expressing CD3 are defined as NK cells and can be enumerated by flow cytometry. A second test assesses NK cell function whereby mononuclear cells are incubated with labeled cells known to be damaged by NK cells or "target cells" are coincubated and subsequently detected and quantified by flow cytometry. Both of these tests can be used to assess a patient's risk of reproductive failure with some degree of success. However, some patients with normal natural killer cell results still continue suffer from immunological -based pregnancy failure.

As noted, T regulatory cells (Treg) are a new and important cell type that may help in diagnosis and assessment in many of these cases. Diminished numbers of these cells in peripheral blood have been associated with pregnancy loss particularly in the immediate post-conceptual period. Jasper et al quantified FoxP3 mRNA, a master regulator of T regulatory cell differentiation, in mid-secretory endometrial tissues from women with unexplained infertility and levels from unaffected women (Molecular Human Reproduction Vol.12, No.5 pp. 301-308, 2006). They found reduced levels of FoxP3 mRNA in affected women when compared with controls. Winger and Reed have found diminished levels of T regulatory cells in the peripheral blood lymphocytes defined by their concurrent expression of CD4, CD25 and FoxP3 in pregnant women who go on to early pregnancy loss. Winger EE, Reed JL. Low Circulating CD4(+) /CD25(+)/Foxp3(+) T Regulatory Cell Levels Predict Miscarriage Risk in Newly Pregnant Women with a History of Failure. Am J Reprod Immunol. 2011 Oct;66(4):320-8.)

A variety of markers are currently being employed for T regulatory cells quantification, however, no comparable assay is available that permits facile functional assessment of T regulatory activity. Such an assay is sorely needed.

In addition to lack of sensitivity demonstrated by the current reproductive immunology assays, they can be vulnerable to specimen collection transportation conditions. The NK cytotoxicity assay is particularly vulnerable. In the assay, effector cell activity is tested. Any stress upon the effector cells can be expected to diminish the measured cytotoxic activity. Not withstanding these effects, target cells are subject to significant variation in their vulnerability to cytotoxic effect. Thus, the assay system is subject to considerable variability. An assay system that is not subject the variability of a functional assay is also sorely needed.

The current functional assays are further limited in their inability to detect functional cell intermediaries. T regulatory cells exert their regulatory activity through a number of intermediaries. The current assays that enumerate the numbers of T regulatory cells do not identify nor quantify any intermediaries. An assay system that permits recognition and quantification of known intermediaries would provide a significant improvement.

More recently, CD4 expressing T cells have been divided into additional subgroups based on their cytokine secreting profile. In addition to Th1 and Th2 cell types, T regulatory, Th3 and Th17 cells have been defined. Also, Th9 and T follicular helper cells (T_{FH}) have been described. Jasper et al. have assessed the relative amount of FoxP3 mRNA in luteal phase endometrium and have shown distinct patterns distinguishing patients with a history of recurrent loss from normal patients. Similarly quantification of mRNA for transcriptional regulators and comparing them with control patients can provide information supporting the classification of patients into candidates for immune-based therapies and subsequent monitoring of interventional therapy.

In addition to the aforementioned deficiencies of immune tests of function and phenotype, specificity and sensitivity are known to be limited. An assay system that permits assessment of multiple different parameters that together provide a profile or signature of the PBMC status of a patient might overcome deficiencies seen in single tests. The present testing methods assess individual characteristics of the patient's immune system. A combinatorial approach wherein a number of different parameters are assessed together might improve both sensitivity and specificity as well improve discrimination of different forms of immune dysfunction that individually affect a single immune parameter in the same manner. An approach broader than assessing immune status of PBMCs might provide better information as to the diagnosis and status of pregnancy disorders. Ideally, such an approach would discriminate between different mechanisms resulting in a shared abnormality in an immune or other parameter.

Personalized medicine, as understood, utilizes testing, in particular testing at the level of DNA and RNA, to determine the most appropriate therapeutic intervention for an individual rather than applying a single therapeutic intervention to all patients with a particular complaint. Ideally, a diagnostic strategy would divide patients into categories where patients are identified who would likely respond to a therapeutic intervention. Ideally, patients the testing strategy would identify patients in whom a therapeutic strategy is unlikely to be of benefit thereby saving application of a costly therapy or a therapy with potential risks to those patients who are unlikely to enjoy a positive therapeutic response. Because of aforementioned deficiencies, it would be useful to useful to identify more robust and stable surrogate markers for the immunologic tests currently being performed in clinical practice as well as identifying surrogate markers for inflammation and coagulation markers.

In addition, new tests are needed that can identify patients who will not benefit from therapy despite a positive disease diagnosis using traditional testing.

Also, new tests are needed that can predict which patients will experience negative side effects from immunotherapy.

MicroRNAs are small, endogenous, non-protein encoding RNA sequences of approximately 22 nucleotide bases that predominantly negatively regulate gene expression. Several hundred such sequences have been identified in humans (Lee et al., PLoS Comput Biol 3:e67 (2007); O'Driscoll, Anticancer Res 26:4271 (2006); Kusenda et al., Biomed Pap Med Fac Univ Palacky Olomouc Czech Repub 150:205 (2006)). The primary transcript or "pri-microRNA" comprises one or more microRNA precursors each comprised within a hairpin structure. These sequences are most commonly found within the introns of their host genes (Lee et al., PLoS Comput Biol 3:e67 (2007)). They may also be found within exons and across exon-intron boundaries (Kusenda et al., Biomed Pap Med Fac Univ Palacky Olomouc Czech Repub 150:205 (2006)). These sequences are known to target at least 30 percent of all human genes, fine-tuning their expression. The final short sequence is generated through a series of cleavages involving two enzymes, Drosha and Dicer, from relatively long RNA primary RNA sequences. The final, cleaved form is incorporated into a complex known as RISC (RNA induced silencing complex) that comprises catalytic proteins such as Argonaut, specifically Ago-2. Jeker and Bluestone (Journal of Clinical Immunology (2010), 30:347-357) hypothesize microRNAs act to stabilize cell phenotype, sharpen gene expression, aid in setting thresholds amongst other regulatory functions. microRNAs appear to be important regulators of cell growth, differentiation, and apoptosis (Lee et al., PLoS Comput Biol 3:e67 (2007)). microRNAs have been extensively studied in cancer pathogenesis because of their known impact on cell dedifferentiation, growth, and apoptosis, each of which individually are important cellular events in the development of cancer (Esau and Monia, Adv Drug Deliv. Rev. 59: 101-114 (2007); Hammond, Nat Genet 39:582 (2007)). microRNA profiles within cancer cells has been an area of intense study. Information gained provides information about the functional state of individual cells.

Investigators have found that global expression of microRNA appears to be more useful than mRNA expression in the classification of cancers (Eis et al. Proc Natl Acad Sci USA 102: 3627 (2005)). More recently, a variety of studies have demonstrated the importance of microRNA in homeostasis and function of the immune system of B lymphocytes, T lymphocytes, macrophages, dendritic cells and the heart (That et al., Science 316:604 (2007); Rodriguez et al., Science 316:608 (2007); O'Connell et al., Proc Natl Acad Sci USA 104:1604 (2007); Care et al., Nat Med 13:613 (2007); Taganov et al., Proc Natl Acad Sci USA I (2006).

MicroRNA 155 (mir-155) is exemplary of microRNA effects upon immune system function as well as immune cell differentiation. The microRNAs disclosed herein are all of human origin , mir-155 acts to stabilize the expression FoxP3 expression in T regulatory cells. (Foxp3-Dependent MicroRNA155 Confers Competitive Fitness to Regulatory T Cells by Targeting SOCS1 Protein Immunity, Volume 30, Issue 1, Pages 80-91 L. Lu, T. Thai, D. Calado, A. Chaudhry, M. Kubo, K. Tanaka, G. Loeb, H. Lee, A. Yoshimura, K. Rajewsky) T regulatory cells are profoundly important in the prevention of autoimmunity and for the establishment of foreign tissue tolerance. Further, That et al. have shown mir-155 plays a role in regulating both T helper cell differentiation and the germinal center reaction regulating the T cell-dependent antibody response (That et al., Science 316:604 (2007); Rodriguez et al., Science 316:608 (2007). Further, transcriptosome analysis of microRNA-155-deficient CD4+ T cells demonstrate a wide spectrum of mir-155 regulated genes, including cytokines, chemokines, and transcription factors (That et al., Science 316:604 (2007); Rodriguez et al., Science 316:608 (2007).

mir-155 is exemplary of the pleitropism of microRNAs. Evidence to date has shown that mir-155 is involved in numerous biologic processes. These include hematopoiesis, inflammation and immunity. It is also involved in regulation of the angiotensin II receptor. Deregulation of mir-155 has been associated with certain cancers, cardiovascular disease as well as viral infections. T regulatory cell development as well as mediation of FoxP3 effects may both involve mir-155 (Susan Kohlhaas, Oliver A. Garden, Cheryl Scudamore, Martin Turner, Klaus Okkenhaug, Elena Vigorito, Cutting Edge: The Foxp3 Target mir-155 Contributes to the Development of Regulatory T Cells, The Journal of Immunology (2009) 182:2578 -2582). Surprisingly, these investigators found that while Treg development could not dispense with bic/mir-155, the pri-microRNA, it was dispensable for Treg proliferation and survival in the periphery. Despite the lower number of Tregs, their suppressor function in vitro remained intact. Such unexpected actions are characteristic of microRNAs. microRNAs can act at different time points in the development of cells and their subsequent functional activation. While they may act individually, they may also act in concerted action of a plurality of microRNAs on a single target mRNA. To add to the complexity of microRNA action, individual microRNAs may also act on many different target mRNAs. Together, these complexities make prediction of their action under differing circumstances tenuous. To date, only empiric methods have defined the role of individual and multiple microRNAs under specific disease conditions.

Other microRNAs are significantly associated with immune responses. For example, TNF alpha and IL-1 beta are regulated by yet another microRNA, mir-146a. (K.D. Taganov, M.P. Boldin, K.J. Chang and D. Baltimore, NF-B-dependent induction of the microRNA mir-146, an inhibitor targeted to signaling proteins of innate immune responses, Proc Natl Acad Sci U S A 103 (2006), pp. 12481-12486, M.M. Perry, S.A. Moschos, A.E. Williams, N.J. Shepherd, H.M. Larner-Svensson and M.A. Lindsay, Rapid changes in microRNA-146a expression negatively regulate the IL-1beta-induced inflammatory response in human lung alveolar epithelial cells, J Immunol 180 (2008), pp. 5689-5698.) Quantification of these microRNAs provides insight into regulatory status. Further, studies in patients suffering from rheumatoid arthritis display interesting microRNA profiles in their PBMCs. (Kaleb M Pauley, Minoru Satoh, Annie L Chan, Michael R Bubb, Westley H Reeves and Edward KL Chan, Upregulated mir-146a expression in PBMCs from rheumatoid arthritis patients, Arthritis Research & Therapy 2008, 10:R101 (doi:10.1186/ar2493) This article is online at: http://arthritis-research.com/content/10/4/R101) They observed significant increases in mir-146a, mir-155, mir-132, mir-16, mir-let-7a relative expression over normal controls as well as significant differences between active and inactive clinical states.

Hunter et al. (Hunter MP, Ismail N, Zhang X, Aguda BD, Lee EJ, et al. (2008) Detection of microRNA Expression in Human Peripheral Blood Microvesicles. PLoS ONE 3(11): e3694. doi:10.1371/journal.pone.0003694) find microRNAs circulating in peripheral blood in several compartments, plasma microvesicles, platelets and PBMCs. They suggest differing roles for microRNAs residing in the different compartments. microRNAs resident within PBMCs are most closely associated with CD4 expressing T cell subclass specification and stable expression. Interrogation of microRNAs resident within the PBMC population is most likely to provide information regarding CD4 expressing T cells. Moreover, monocytes and cells of the monocyte lineage and of dendritic cells, one of the two peripheral blood mononuclear populations, regulate the functional state and activity of T cells and in particular CD4 expressing T cells. Importantly, monocytes participlate in initiation of coagulation expressing Tissue Factor on their surface. Mir-19b and mir-20a appear to modulate tissue factor expression in patients with lupus (Raúl Teruel, Carlos Pérez-Sánchez, Javier Corral, Maria Teresa Herranz, Virginia Pérez-Andreu, Encarnación Saiz, Nuria García-Barberá, Irene Martínez-Martínez, Vanessa Roldán, Vicente Vicente, López-Pedrera, Constantino Martinez, Identification of microRNAs as potential modulators of tissue factor expression in patients with systemic lupus erythematosus and antiphospholipid syndrome, Journal of Thrombosis and Haemostasis, in press). Therefore, interrogation of microRNAs within PBMCs as a whole provides significant information regarding the immune balance and stability of circulating mononuclear cells.

Selective interrogation of subsets of mononuclear cells can impart additional information. For example, removal of monocytes from isolated PBMCs permits selective microRNA interrogation of lymphocytes. Conversely, monocytes can be interrogated directly for microRNAs. Further, lymphocyte subpopulations, themselves, can be individually interrogated following their selective isolation by such techniques, for example, flow cytometric sorting following interaction with fluorescently labeled monoclonal antibody combinations that are capable of discreetly characterizing the individual subclasses. For example, T regulatory cells may be contacted under selective binding conditions with fluorescently labeled anti-CD3, CD4, CD25 and CD127 and selected by their expression of CD3, CD4, CD25 and absence or low expression of CD127.

MicroRNA provides information that is different than quantification of lymphocyte subsets, their function or markers of these subsets that might be surrogates for these subsets such as FoxP3 mRNA which might be regarded as a surrogate for quantification of T regulatory cells. microRNAs may be found in a variety of different cell types and represent different functions in different cell types or may represent activity states or other features that are not disclosed from quantification of cell types or functional states (such as cytotoxicity). Alterations in microRNA expression varies from one disease to another. The microRNA alterations noted in rheumatoid arthritis differ from those seen in lupus. (Pauley KM, Satoh M, Chan AL, Bubb MR, Reeves WH, Chan EK. Upregulated mir-146a expression in PBMCs from rheumatoid arthritis patients. Arthritis Res Ther. 2008;10(4):R101, Gastroenterol Hepatol (N Y). 2010 November; 6(11): 714-722. MicroRNA (microRNA) Expression in Ulcerative Colitis (UC) and Crohn's Disease (CD) and Dai Y, Huang YS, Tang M, Lv TY, Hu CX, Tan YH, Xu ZM, Yin YB. Microarray analysis of microRNA expression in peripheral blood cells of systemic lupus erythematosus patients. Lupus. 2007;16(12):939-46., Raúl Teruel, Carlos Pérez-Sánchez, Javier Corral, Maria Teresa Herranz, Virginia Pérez-Andreu, Encarnación Saiz, Nuria García-Barberá, Irene Martínez-Martínez, Vanessa Roldán, Vicente Vicente, §hary López-Pedrera, Constantino Martinez, Identification of microRNAs as potential modulators of tissue factor expression in patients with systemic lupus erythematosus and antiphospholipid syndrome, Journal of Thrombosis and Haemostasis, in press).

Aspects of immune disorders have been implicated in alterations in microRNA expression. MicroRNAs have significant effects in the regulation of immunological functions and the prevention of autoimmunity (Kaleb M. Pauley, Seunghee Cha, and Edward K.L. Chan, MicroRNA in autoimmunity and autoimmune diseases, J Autoimmun. (2009) 32(3-4): 189-194). Anti-phospholipid antibody syndrome is an example of an autoimmune condition associated with diminished fertility, recurrent unexplained abortion and pregnancy complications as well as increased risk of autoimmune, cardiovascular and thrombotic disease. Diminished expression of certain microRNAs (mir-19b and 20a) may identify patients at increased risk of pregnancy complications treatable by anticoagulation therapy (e.g. aspirin and/or heparins) (Raúl Teruel, Carlos Pérez-Sánchez, Javier Corral, Maria Teresa Herranz, Virginia Pérez-Andreu, Encarnación Saiz, Nuria García-Barberá, Irene Martinez-Martinez, Vanessa Roldán, Vicente Vicente, §hary López-Pedrera, Constantino Martinez, Identification of microRNAs as potential modulators of tissue factor expression in patients with systemic lupus erythematosus and antiphospholipid syndrome, Journal of Thrombosis and Haemostasis, in press)).

Identification of surrogate measures of the dysfunctional status of patients suffering reproductive disorders, it is recalled that pregnancy constitutes an immunologic paradox where typical alloimmune responses to tissues such as tissue/organ grafts are rejected, in the healthy pregnancy tolerance to alloantigen prevails preventing the rejection of the hemialloantigenic embryo. Multiple and seemingly redundant mechanisms have been described that appear to maintain allo-tolerance. The existence of these mechanisms differentiate allo-responses to the fetus and autoimmunity. It is expected, therefore, that microRNA patterns identified amongst patients suffering reproductive disorders are unlikely to be similar to those identified in autoimmunity. Moreover, there appears to be no direct surrogacy of microRNAs for currently applied immune, coagulation tests currently being applied because of their broad and varied presence and function in different cell types.

Mishra (US patent application 20100216142 A1, Mishra; Nilamadhab, microRNA Biomarkers in Lupus) identifies a variety of microRNAs that are deregulated in lupus. Comprised amongst immune abnormalities seen in lupus are antibodies directed against various phospholipids and increased levels of inflammatory markers such as tissue factor. A recent study by Ceribelli identifies antibodies directed against Ago2, a component of the RISC moiety required for the genesis of microRNAs (Angela Ceribelli. Angela Tincani , Franco Franceschini, Roberto Cattaneo, Brad A. Pauley, Jason Y. F. Chan, Edward K. L. Chan, Anti-argonaute2 (Ago2/Su) and -Ro antibodies identified by immunoprecipitation in primary anti-phospholipid syndrome (PAPS), Posted online on August 9, 2010 (doi:10.3109/08916934.2010.499886)). Intravenous immunoglobulin (IVIG) therapy is an example an immune therapy used to reduce miscarriage incidence in women with a history of immunologic recurrent spontaneous abortion. Winger and Reed have demonstrated efficacy of IVIG therapy in a group of women with recurrent spontaneous abortion when compared with a group of women not receiving IVIG therapy. (Winger EE, Reed JL, Ashoush S, El-Toukhy T, Ahuja S, Taranissi M. Elevated Preconception CD56(+) 16(+) and/or Th1:Th2 Levels Predict Benefit from IVIG Therapy in Subfertile Women Undergoing IVF. Am J Reprod Immunol. 2011 May 30.) In addition, when patients with anti-phospholipid antibodies were excluded from the patient pool in this study, significant differences could no longer be found between the treated and non-treated groups (unpublished data). Therefore the ability to identify patients with markers associated with antiphospholipid antibodies may be particularly useful when deciding who may benefit from immunologic treatment.

Detection of microRNAs in PBMCs and their constituent selected subsets, provides the clinician with an additional means of characterizing patients with or at risk of reproductive immunologically-related disorder into groups whose disorder is mediated by an imbalance, for example, in immune cell activity, inflammation or coagulation. Detection of microRNAs in women with pregnancy disorders is taught by Taylor and Gercel-Taylor (Taylor and Gercel-Taylor in US patent application 20100151480 A1). They teach methods for diagnosis of cancer and adverse pregnancy outcomes in a subject by measuring the amount of one or more RNAs present in exosomes isolated from a biologic sample from the affected individual. The methods they teach can be distinguished from the present subject matter. They teach that the microRNAs they interrogate are derived directly from placental tissues and, thereby represents the pathophysiologic state of that organ. The presently disclosed material teaches methods for defining the systemic and/or local immune status of the affected individual thereby providing an alternative and supplemental means for assessing the immune status of an individual that might adversely affect their reproductive health. The addition of such parameters should increase the sensitivity of a diagnostic panel. For example, mir-155 has been shown to be important in the stabilization of FoxP3 expression and, moreover, for the effector function of T regulatory cells, in part, through the regulation of CTLA-4 expression (Lu et al. Immunity 30, 80-91 (2009)). Numerous and redundant mechanisms are postulated to induce a tolerant state in the mother operative at various time points that include the egg development (oogenesis) period, preconceptual period, insemination, implantation through the remaining course of pregnancy and post-parturition. Egg quality may also be affected by immunologic events. A complex interplay of cytokines, hormones, growth factors are involved in oogenesis which engage immune cell interactions. microRNAs quantification in immune cells may profile both a normal course and be distinguishable from the course in patients with reproductive and other immunologically related disorders.

MicroRNA quantification provides additional benefits. Were patterns of response to immunotherapies identified, they could be used to predict response patterns amongst patient subgroups identified by their microRNA profiles. Were specific microRNAs or groups of microRNAs identified whose responses following therapies could be used to dichotomize or multiperize patient groups, then patients so grouped could be assessed according to the responses to therapies and used to more precisely predict responses of individual patients so grouped.

The ability to separate patients into groups of two or more is important. The use of Herceptin to treat breast cancer could not be shown to be effective when used in otherwise clinical similar breast cancers. Herceptin (trastuzumab) targets the HER2/neu receptor. In early breast cancer, presence or absence of the receptor is not clinically apparent. However, when expressed by a breast cancer, it denotes a class of tumors with more aggressive future behavior. Molecular techniques can be used to assess the HER2/neu status of the tumor. Clinical studies revealed that when use of the drug was restricted to those patients whose tumor was positive for HER2/neu expression, the drug was shown to be effective. The identification of microRNA profiles comprising one or more individual microRNAs could be of similar importance in separating patients into separate groups otherwise indistinguishable that could be assessed to define groups of individuals with similar drug responses.
Furthermore, methods for identifying at least two characteristic groups in a patient population quantifying a microRNA from a biological sample are known from prior art documents.
Some of these documents are:
- WO 2009/093254;
- WO 2009/015357;
- GLEN REID ET AL "Circulating micro RNAs: Association with disease and potential use as biomarkers";
- YI HU ET AL "Two common SNPs in pri-miR-125° alter the mature miRNA expression and associate with recurrent pregnancy loss in a Han-Chinese population";
- A. REVEL ET AL "MicroRNAs are associated with human embryo implantation defects";
- PINELES ET AL "Distinct subsets of microRNAs are expressed differentially in the human placentas of patients with preeclampsia";
- DIANA M MORALES PRIETO ET AL "MicroRNAs in pregnancy"
However such a biological sample, as disclosed in these prior art documents, is generally derived by milk, blood, or serum.

This invention accomplishes these and other goals.

### SUMMARY OF THE INVENTION

In accordance with the above needs and those that will be mentioned and will become apparent below, this disclosure is directed to a method for identifying at least two characteristic groups in a patient population on the basis of microRNA expression including the steps of collecting immune cells, extracting microRNA-comprising RNA from the immune cells, quantifying at least one microRNA within the extracted RNA, and segregating the patient population into the groups on the basis of expression of the at least one microRNA. Preferably, the step of collecting immune cells comprises collecting peripheral blood mononuclear cells.

In one aspect, the step of segregating the patient population includes assigning patients expressing a relatively high level of the at least one microRNA to a first group and assigning patients expressing a relatively low level of the at least one microRNA to a second group. Collecting immune cells may include collecting cells before or after an immunotherapy treatment. A further aspect is directed to collecting the cells before and after an immunotherapy treatment such that segregating the patient population includes determining the change in expression level of the at least one microRNA after the immunotherapy treatment. Preferably, segregating the patient population comprises assigning patients exhibiting a first change in the expression level to a first group and assigning patients exhibiting a second change in the expression level to a second group. The first change may be a relatively large change in expression level and the second change may be a relatively small change in expression level. Alternatively, the first change may be a positive change in expression level and the second change may be a negative change in expression level.

Preferably, the absolute value of the mean of the change in the expression level of the at least one microRNA in the first group divided by the standard deviation is greater than or equal to one. Further, one embodiment is directed to the further step of identifying a subset of microRNAs within the group of known microRNAs that exhibit a change in expression level in the first group such that the absolute value of the mean of the change in expression level divided by the standard deviation is greater than or equal to one. In addition, the method can also include identifying a microRNA within the group of known microRNAs that exhibits the greatest change in expression level in the first group.

Another embodiment of the invention is directed to the additional steps of collecting immune cells from an additional patient, extracting microRNA-comprising RNA from the immune cells of the additional patient, quantifying at least one microRNA within the extracted RNA from the additional patient, and identifying the additional patient as belonging to one of the segregated groups on the basis of expression of the at least one microRNA. Preferably, this also include administering a treatment to the additional patient based on the identification, such as IVIG.

In another aspect, the methods of the invention also include diagnosing a patient as having a condition based on membership in a segregated group. One embodiment is directed to diagnosing a patient having a reproductive disorder.

Yet another aspect of the invention includes the additional step of monitoring treatment of a patient belonging to one of the segregated groups by collecting immune cells, extracting at least one microRNA and quantifying the at least one microRNA at a subsequent time.

Examples of suitable microRNAs that may be used according to this disclosure include, without limitation, hsa-miR-582-3p MIMAT0004797 (SEQ ID NO: 1); hsa-miR-7-1-3p MIMAT0004553 (SEQ ID NO: 2); hsa-miR-340-5p MIMAT0004692 (SEQ ID NO: 3); hsa-miR-199b-3p MIMAT0004563 (SEQ ID NO: 4); hsa-miR-199a-3p MIMAT0000232 (SEQ ID NO: 5); hsa-miR-30e-5p MIMAT0000692 (SEQ ID NO: 6); hsa-miR-575 MIMAT0003240 (SEQ ID NO: 7); hsa-miR-7-5p MIMAT0000252 (SEQ ID NO: 8); hsa-miR-33a-3p MIMAT0004506 (SEQ ID NO: 9); hsa-miR-7-2-3p MIMAT0004554 (SEQ ID NO: 10); hsa-miR-199b-5p MIMAT0000263 (SEQ ID NO: 11); hsa-miR-144-5p MIMAT0004600 (SEQ ID NO: 12); hsa-miR-30e-3p MIMAT0000693 (SEQ ID NO: 13); hsa-miR-424-3p MIMAT0004749 (SEQ ID NO: 14); hsa-miR-33a-5p MIMAT0000091 (SEQ ID NO: 15); hsa-miR-671-3p MIMAT0004819 (SEQ ID NO: 16); hsa-miR-340-3p MIMAT0000750 (SEQ ID NO: 17); hsa-miR-1267 MIMAT0005921 (SEQ ID NO: 18); hsa-miR-1229-3p MIMAT0005584 (SEQ ID NO: 19); hsa-miR-424-5p MIMAT0001341 (SEQ ID NO: 20); hsa-miR-221-3p MIMAT0000278 (SEQ ID NO: 21); hsa-miR-1 MIMAT0000416 (SEQ ID NO: 22); hsa-miR-133b MIMAT0000770 (SEQ ID NO: 23); hsa-miR-221-5p MIMAT0004568 (SEQ ID NO: 24); hsa-miR-210 MIMAT0000267 (SEQ ID NO: 25); hsa-miR-1229-5p MIMAT0022942 (SEQ ID NO: 26); hsa-miR-671-5p MIMAT0003880 (SEQ ID NO: 27); hsa-miR-582-5p MIMAT0003247 (SEQ ID NO: 28); hsa-miR-199a-5p MIMAT0000231 (SEQ ID NO: 29); hsa-miR-144-3p MIMAT0000436 (SEQ ID NO: 30); hsa-miR-376a-5p MIMAT0003386 (SEQ ID NO: 31); hsa-miR-193a-3p MIMAT0000459 (SEQ ID NO: 32); hsa-miR-557 MIMAT0003221 (SEQ ID NO: 33); hsa-miR-34a-3p MIMAT0004557 (SEQ ID NO: 34); hsa-miR-584-5p MIMAT0003249 (SEQ ID NO: 35); hsa-miR-1244 MIMAT0005896 (SEQ ID NO: 36); hsa-miR-125b-1-3p MIMAT0004592 (SEQ ID NO: 37); hsa-miR-32-3p MIMAT0004505 (SEQ ID NO: 38); hsa-miR-933 MIMAT0004976 (SEQ ID NO: 39); hsa-miR-373-5p MIMAT0000725 (SEQ ID NO: 40); hsa-let-7b-5p MIMAT0000063 (SEQ ID NO: 41); hsa-miR-376a-3p MIMAT0000729 (SEQ ID NO: 42); hsa-miR-129-2-3p MIMAT0004605 (SEQ ID NO: 43); hsa-miR-548am-3p MIMAT0019076 (SEQ ID NO: 44); hsa-let-7f-5p MIMAT0000067 (SEQ ID NO: 45); hsa-miR-876-3p MIMAT0004925 (SEQ ID NO: 46); hsa-miR-371a-5p MIMAT0004687 (SEQ ID NO: 47); hsa-miR-423-5p MIMAT0004748 (SEQ ID NO: 48); hsa-miR-373-3p MIMAT0000726 (SEQ ID NO: 49); hsa-miR-152 MIMAT0000438 (SEQ ID NO: 50); hsa-miR-34a-5p MIMAT0000255 (SEQ ID NO: 51); hsa-miR-335-5p MIMAT0000765 (SEQ ID NO: 52); hsa-miR-181c-5p MIMAT0000258 (SEQ ID NO: 53); hsa-miR-125b-2-3p MIMAT0004603 (SEQ ID NO: 54); hsa-miR-548am-5p MIMAT0022740 (SEQ ID NO: 55); hsa-miR-338-3p MIMAT0000763 (SEQ ID NO: 56); hsa-miR-1225-5p MIMAT0005572 (SEQ ID NO: 57); hsa-miR-362-3p MIMAT0004683 (SEQ ID NO: 58); hsa-miR-767-5p MIMAT0003882 (SEQ ID NO: 59); hsa-miR-136-3p MIMAT0004606 (SEQ ID NO: 60); hsa-miR-29b-1-5p MIMAT0004514 (SEQ ID NO: 61); hsa-miR-29a-3p MIMAT0000086 (SEQ ID NO: 62); hsa-miR-92b-3p MIMAT0003218 (SEQ ID NO: 63); hsa-miR-362-5p MIMAT0000705 (SEQ ID NO: 64); hsa-miR-223-5p MIMAT0004570 (SEQ ID NO: 65); hsa-miR-505-3p MIMAT0002876 (SEQ ID NO: 66); hsa-miR-634 MIMAT0003304 (SEQ ID NO: 67); hsa-miR-371a-3p MIMAT0000723 (SEQ ID NO: 68); hsa-miR-129-1-3p MIMAT0004548 (SEQ ID NO: 69); hsa-miR-1238-5p MIMAT0022947 (SEQ ID NO: 70); hsa-miR-876-5p MIMAT0004924 (SEQ ID NO: 71); hsa-miR-181c-3p MIMAT0004559 (SEQ ID NO: 72); hsa-miR-338-5p MIMAT0004701 (SEQ ID NO: 73); hsa-miR-505-5p MIMAT0004776 (SEQ ID NO: 74); hsa-miR-335-3p MIMAT0004703 (SEQ ID NO: 75); hsa-miR-543 MIMAT0004954 (SEQ ID NO: 76); hsa-miR-223-3p MIMAT0000280 (SEQ ID NO: 77); hsa-miR-125b-5p MIMAT0000423 (SEQ ID NO: 78); hsa-miR-1238-3p MIMAT0005593 (SEQ ID NO: 79); hsa-miR-377-5p MIMAT0004689 (SEQ ID NO: 80); hsa-miR-584-3p MIMAT0022708 (SEQ ID NO: 81); hsa-miR-22-5p MIMAT0004495 (SEQ ID NO: 82); hsa-miR-376a-2-5p MIMAT0022928 (SEQ ID NO: 83); hsa-miR-301a-5p MIMAT0022696 (SEQ ID NO: 84); hsa-miR-548m MIMAT0005917 (SEQ ID NO: 85); hsa-miR-29b-3p MIMAT0000100 (SEQ ID NO: 86); hsa-miR-99a-3p MIMAT0004511 (SEQ ID NO: 87); hsa-miR-33b-3p MIMAT0004811 (SEQ ID NO: 88); hsa-miR-92b-5p MIMAT0004792 (SEQ ID NO: 89); hsa-miR-602 MIMAT0003270 (SEQ ID NO: 90); hsa-miR-1237-3p MIMAT0005592 (SEQ ID NO: 91); hsa-miR-129-5p MIMAT0000242 (SEQ ID NO: 92); hsa-miR-148b-3p MIMAT0000759 (SEQ ID NO: 93); hsa-miR-377-3p MIMAT0000730 (SEQ ID NO: 94); hsa-let-7b-3p MIMAT0004482 (SEQ ID NO: 95); hsa-miR-125a-5p MIMAT0000443 (SEQ ID NO: 96); hsa-miR-125a-3p MIMAT0004602 (SEQ ID NO: 97); hsa-miR-148b-5p MIMAT0004699 (SEQ ID NO: 98); hsa-miR-22-3p MIMAT0000077 (SEQ ID NO: 99); hsa-miR-1237-5p MIMAT0022946 (SEQ ID NO: 100); hsa-let-7f-1-3p MIMAT0004486 (SEQ ID NO: 101); hsa-miR-29a-5p MIMAT0004503 (SEQ ID NO: 102); hsa-miR-193a-5p MIMAT0004614 (SEQ ID NO: 103); hsa-miR-423-3p MIMAT0001340 (SEQ ID NO: 104); hsa-miR-191-3p MIMAT0001618 (SEQ ID NO: 105); hsa-miR-301a-3p MIMAT0000688 (SEQ ID NO: 106); hsa-miR-767-3p MIMAT0003883 (SEQ ID NO: 107); hsa-miR-563 MIMAT0003227 (SEQ ID NO: 108); hsa-miR-95 MIMAT0000094 (SEQ ID NO: 109); hsa-miR-1234-3p MIMAT0005589 (SEQ ID NO: 110); hsa-miR-1225-3p MIMAT0005573 (SEQ ID NO: 111); hsa-miR-136-5p MIMAT0000448 (SEQ ID NO: 112); hsa-miR-1234-5p MIMAT0022944 (SEQ ID NO: 113); hsa-miR-99a-5p MIMAT0000097 (SEQ ID NO: 114); hsa-miR-32-5p MIMAT0000090 (SEQ ID NO: 115); hsa-miR-191-5p MIMAT0000440 (SEQ ID NO: 116); hsa-miR-33b-5p MIMAT0003301 (SEQ ID NO: 117); hsa-mir-1-1 MI0000651 (SEQ ID NO: 118); hsa-mir-1-2 MI0000437 (SEQ ID NO: 119); hsa-mir-7-1 MI0000263 (SEQ ID NO: 120); hsa-mir-7-2 MI0000264 (SEQ ID NO: 121); hsa-mir-7- (SEQ ID NO:3 MI0000265 122); hsa-mir-30e MI0000749 (SEQ ID NO: 123); hsa-mir-33a MI0000091 (SEQ ID NO: 124); hsa-mir-133b MI0000822 (SEQ ID NO: 125); hsa-mir-144 MI0000460 (SEQ ID NO: 126); hsa-mir-199a-1 MI0000242 (SEQ ID NO: 127); hsa-mir-199a-2 MI0000281 (SEQ ID NO: 128); hsa-mir-199b MI0000282 (SEQ ID NO: 129); hsa-mir-210 MI0000286 (SEQ ID NO: 130); hsa-mir-221 MI0000298 (SEQ ID NO: 131); hsa-mir-340 MI0000802 (SEQ ID NO: 132); hsa-mir-424 MI0001446 (SEQ ID NO: 133); hsa-mir-575 MI0003582 (SEQ ID NO: 134); hsa-mir-582 MI0003589 (SEQ ID NO: 135); hsa-mir-671 MI0003760 (SEQ ID NO: 136); hsa-mir-1229 MI0006319 (SEQ ID NO: 137); hsa-mir-1267 M10006404 (SEQ ID NO: 138); hsa-let-7a-3 M10000062 (SEQ ID NO: 139); hsa-let-7e M10000066 (SEQ ID NO: 140); hsa-mir-22 M10000078 (SEQ ID NO: 141); hsa-mir-29a M10000087 (SEQ ID NO: 142); hsa-mir-29b-1 MI0000105 (SEQ ID NO: 143); hsa-mir-32 MI0000090 (SEQ ID NO: 144); hsa-mir-33b MI0003646 (SEQ ID NO: 145); hsa-mir-34a MI0000268 (SEQ ID NO: 146); hsa-mir-92b MI0003560 (SEQ ID NO: 147); hsa-mir-95 MI0000097 (SEQ ID NO: 148); hsa-mir-99a MI0000101 (SEQ ID NO: 149); hsa-mir-125a MI0000469 (SEQ ID NO: 150); hsa-mir-125b-1 MI0000446 (SEQ ID NO: 151); hsa-mir-125b-2 MI0000470 (SEQ ID NO: 152); hsa-mir-129-1 MI0000252 (SEQ ID NO: 153); hsa-mir-129-2 MI0000473 (SEQ ID NO: 154); hsa-mir-136 MI0000475 (SEQ ID NO: 155); hsa-mir-148b MI0000811 (SEQ ID NO: 156); hsa-mir-152 MI0000462 (SEQ ID NO: 157); hsa-mir-181c MI0000271 (SEQ ID NO: 158); hsa-mir-191 MI0000465 (SEQ ID NO: 159); hsa-mir-193a MI0000487 (SEQ ID NO: 160); hsa-mir-223 MI0000300 (SEQ ID NO: 161); hsa-mir-301a MI0000745 (SEQ ID NO: 162); hsa-mir-335 MI0000816 (SEQ ID NO: 163); hsa-mir-338 MI0000814 (SEQ ID NO: 164); hsa-mir-362 MI0000762 (SEQ ID NO: 165); hsa-mir-371a MI0000779 (SEQ ID NO: 166); hsa-mir-373 MI0000781 (SEQ ID NO: 167); hsa-mir-376a-1 MI0000784 (SEQ ID NO: 168); hsa-mir-376a-2 MI0003529 (SEQ ID NO: 169); hsa-mir-377 MI0000785 (SEQ ID NO: 170); hsa-mir-423 MI0001445 (SEQ ID NO: 171); hsa-mir-425 MI0001448 (SEQ ID NO: 172); hsa-mir-505 MI0003190 (SEQ ID NO: 173); hsa-mir-543 MI0005565 (SEQ ID NO: 174); hsa-mir-548m MI0006400 (SEQ ID NO: 175); hsa-mir-557 MI0003563 (SEQ ID NO: 176); hsa-mir-563 MI0003569 (SEQ ID NO: 177); hsa-mir-584 MI0003591 (SEQ ID NO: 178); hsa-mir-602 MI0003615 (SEQ ID NO: 179); hsa-mir-634 MI0003649 (SEQ ID NO: 180); hsa-mir-767 MI0003763 (SEQ ID NO: 181); hsa-mir-876 MI0005542 (SEQ ID NO: 182); hsa-mir-933 MI0005755 (SEQ ID NO: 183); hsa-mir-1225 MI0006311 (SEQ ID NO: 184); hsa-mir-1234 MI0006324 (SEQ ID NO: 185); hsa-mir-1237 MI0006327 (SEQ ID NO: 186); hsa-mir-1238 MI0006328 (SEQ ID NO: 187); hsa-mir-1244-1 MI0006379 (SEQ ID NO: 188); hsa-mir-1244-2 MI0015974 (SEQ ID NO: 189); hsa-mir-1244-3 MI0015975 (SEQ ID NO: 190); and hsa-mir-1825 MI0008193 (SEQ ID NO: 191)

Embodiments of the invention may include the use of at least one microRNA selected from hsa-let-7e, mir-1, hsa-mir-1181, hsa-miR-1183, hsa-miR-1224-5p, hsa-miR-127-3p, hsa-mir-1296, hsa-mir-132, hsa-mir-136, hsa-miR-139-3p, hsa-mir-141, hsa-miR-142-3p, hsa-mir-142-5p, hsa-mir-144, hsa-mir-153, hsa-mir-1537, hsa-miR-154, hsa-miR-191, hsa-mir-193a-3p, hsa-miR-19a, hsa-mir-219-5p, hsa-mir-29b, hsa-mir-301a, hsa-miR-301b, hsa-miR-30e, hsa-mir-32, hsa-mir-33a, hsa-miR-340, hsa-miR-362-3p, hsa-miR-371-5p, hsa-377, hsa-miR-423-3p, hsa-miR-432, hsa-mir-513a-5p, hsa-mir-545, hsa-miR-548a-5p, hsa-miR-574-5p, hsa-mir-582-3p, hsa-mir-590-5p, hsa-mir-15a, hsa-mir-548c-5p, hsa-mir-1225-3p, hsa-mir-29b, hsa-mir-21, hsa-mir-1237, hsa-mir-101, hsa-mir-1539, hsa-mir-557, hsa-mir-125a-3p and hsa-mir-423-5p. In another aspect, the microRNA is selected from hsa-mir-136, hsa-mir-141, hsa-mir-142-5p, hsa-mir-144, hsa-mir-153, hsa-mir-1537, hsa-mir-193a-3p, hsa-mir-219-5p, hsa-mir-29b, hsa-mir-301a, hsa-mir-32, hsa-mir-33a, hsa-mir-545, hsa-mir-582-3p, hsa-mir-590-5p, hsa-mir-1181, hsa-mir-513a-5p, hsa-mir-132 and hsa-mir-1296. In another aspect, the microRNA is selected from hsa-miR-144, hsa-miR-582-5p, hsa-miR-30e-3p, hsa-miR-340-5p, hsa-miR-424-5p, hsa-miR-199a-5p, hsa-miR-199b-5p, hsa-miR-210, hsa-miR-221-5p, hsa-miR-33a-5p, hsa-miR-575, hsa-miR-7-5p, hsa-miR-1229, hsa-miR-1267, hsa-miR-671-3p, hsa-miR-1244, hsa-miR-1 and hsa-miR-133b. In another aspect, the at least one microRNA may be mir-1229 or mir-671-3p. In yet another aspect, quantifying and segregating may include using at least four microRNAs selected from the group consisting of miR-7-5p, miR-1229, miR-1267, miR-671-3p, miR-340-5p, hsa-miR-1, hsa-miR-133b and hsa-miR-33a-5p.

### BRIEF DESCRIPTION OF THE DRAWINGS

Further features and advantages will become apparent from the following and more particular description of the preferred embodiments of the invention, as illustrated in the accompanying drawings, and in which like referenced characters generally refer to the same parts or elements throughout the views, and in which:
FIG. 1 shows the CT levels of the patients having high initial microRNA expression before and after IVIG treatment, according to an embodiment of the invention;
FIG. 2 shows the CT levels of the patients having low initial microRNA expression before and after IVIG treatment, according to an embodiment of the invention;
FIG. 3 shows a scoring system for assessing selected microRNAs, according to an embodiment of the invention;
FIGs. 4a-4g show top 100 and bottom 100 microRNAs as related to patient outcomes, according to an embodiment of the invention;and
FIGs. 5a-b show differences in expression of the top 25 and bottom 25 microRNAs of FIGs. 4a-g, according to an embodiment of the invention.

### DETAILED DESCRIPTION OF THE INVENTION

At the outset, it is to be understood that this disclosure is not limited to particularly exemplified materials, architectures, routines, methods or structures as such may, of course, vary. Thus, although a number of such option, similar or equivalent to those described herein, can be used in the practice of embodiments of this disclosure, the preferred materials and methods are described herein.

It is also to be understood that the terminology used herein is for the purpose of describing particular embodiments of this disclosure only and is not intended to be limiting.

This summary provides a listing of several embodiments of the presently disclosed subject matter. However, it should be understood that variations and permutations of these embodiments exist. This summary is intended to serve as exemplary of potential embodiments.

The term "immune cells" as used herein shall mean lymphocytes, monocytes and granulocytes, their precursors and maturational derivatives. These shall include, for example, plasma cells, dendritic cells, mast cells, granulocytes and macrophages. It is understood that immune cells participate in a broad range of activities. These include immunologic surveillance and intervention (such as elimination of malignant cells and elimination of infectious agents). Moreover, cells of the immune system, in particular monocytes and their derivatives are involved in coagulation. Further, it is understood inflammation is a manifestation of activities of cells of the immune system.

As used herein, the term "immunotherapy", "immunotherapeutic" or "immune therapy" shall include therapeutic intervention directed to modification of activities of cells of the immune system thereby contemplating actions affecting immune cells wherein said cells affect coagulation and inflammation.

Herein, specific microRNAs may be identified by their prefix mir-and their identifier, such as mir-155 in this case. Sequences within an RNA transcript targeted by miRNAs may lie anywhere within the transcript. However, sequences within the 3' untranslated region are most common. MicroRNA nomenclature comprises a three-letter prefix "mir" followed by a number assigned generally in order of the description of the microRNA. In one convention when the "R" is lower case, the sequence refers to the pre-microRNA while when upper case is employed (miR), the mature form is indicated. Variants where the sequences vary by one or two bases may be designated by the letters "a" and "b". Occasionally, pre-microRNAs located within separate regions of the genome result in an identical mature microRNA. These microRNAs are distinguished by a numeric suffix ("miR-123-1" and "miR-123-2"). When two microRNAs originate from opposite arms of the same pre-microRNA they are designated with the suffix -3p or -5p according to whether the 3' or 5' strand is used. As used herein, the numeric code, e.g. "mir-123" shall include its variants such as mir-123-1, mir123-2, and the -3p and -5p variants. As used herein the term "pri-miRNA" shall mean the RNA targeted by the Drosha-Pasha complex. As used herein the term "pre-miRNA" shall mean the product of the cleavage by the Drosha-Pasha complex. As used herein, no distinction shall be made between sequences between the parent nomenclature for example mir-123 and any more selective sequence for example mir-123-5p and other than by description within the text.

Specific microRNA abbreviations may also include an additional prefix identifying the species of origin, such as hsa for homo sapiens. Although the primary embodiments described herein are directed to humans, one of skill in the art will appreciate that the techniques of this disclosure can be applied to other species.

The term "control individual" as used herein has a special meaning. A "control individual" shall mean individuals of comparable characteristics such as age and sex who do not have a reproductive disorder and are not at known risk of developing a reproductive disorder. The term "control sample" as used herein shall mean a biologic sample from the same source, such a peripheral blood, and collected under the same or comparable conditions as a patient sample comprising immune cells collected from a control individual that is processed and analyzed in the same manner as a patient sample.

It is further understood that the term "control sample" as used herein may represent the mathematical mean of multiple samples from control individuals wherein a number of samples considered sufficient by an individual of ordinary skill in the art are collected. Additional statistical parameters may be derived from said samples such as standard deviation of the mean. Said additional statistical parameters may be used for purposes of comparison of a patient test result with control samples to estimate the probability that the patient's test result represents an abnormal finding and, thereby suggests that the patient is suffering from a reproductive disorder or risk of a reproductive disorder. For purposes of simplicity the term may also be used in another way wherein a plurality of comparable, temporally separate, samples are collected and assayed from a single individual and compared with one another such that a first sample or a particular subsequent sample are compared as though the first is a control for the second, permitting assessment of a change in condition potentially as a function of the clinical state, or stage of pregnancy or as a result of therapeutic intervention.

The term "reproductive immune dysfunction" or "reproductive disorder" as used herein shall comprise those disorders of the reproductive system suspected of having an immune component. These shall include but not limited to the following: infertility and post-conceptive failure such as implantation failure that may be unrecognized and thereby diagnosed as infertility; miscarriage; conditions that do not lead to miscarriage but compromise optimal pregnancy outcome such as intrauterine growth retardation, PROM (Premature Rupture Of Membranes), pre-eclampsia, preterm labor, placental abruption and stillbirth; those conditions known to contribute to infertility, pregnancy complications and early implantation failure such as endometriosis and autoimmune thyroiditis and anti-phospholipid antibody syndrome, those pregnancy disorders that compromise optimal fetal growth, maturation and development in pregnancy and/or compromise potential childhood development after delivery, those reproductive disorders that compromise the long term reproductive potential of the mother over the course of her reproductive lifespan.

The term "Immunologic disorder" as used herein shall comprise disorders caused by abnormal, whether humoral, cell-mediated, or both and/or related to an immune component such as an inflammatory, complement_or coagulation- mediated component.

As used herein, the term "differentially expressed" or "differential expression" shall mean a detectable difference by the selected detection means in the quantification of a specific microRNA between the biologic sample of the patient and the corresponding mean value of a control population wherein said difference has been identified between a statistically significant patient population with a reproductive disorder and a corresponding control population without the disorder or risk of the disorder. The terms may also be applied wherein quantification of a plurality of individual microRNAs form a pattern that can be distinguished from a corresponding pattern identified in controls. Differential expression of one or more microRNAs between patients with a reproductive disorder and/or risk of a reproductive disorder and control individuals is preferably determined in a screen of a panel of microRNAs such as that provided by SABiosciences (catalog MAH-104A). Differentially expressed microRNAs between patient and control values may be determined by a variety of means. Each method requires inclusion of a minimum number of samples from each group so that a significant difference in expression between the two groups can be ascertained. A preferred embodiment to define differentially expressed microRNAs between patients and controls utilizes the microRNA human immunopathology-related microRNA array (SuperArray technology, SABiosciences, Frederick, MD catalogue MAH-104A for Stratagene Mx3005p). Reactions of three or more RNA extracts from patients with a reproductive disorder or at risk of a reproductive disorder and three or more control samples are performed following the manufacturer's instructions. The quantitative PCR is run on a Stratagene 3005p real-time thermocycler following the manufacturer's instructions. For each set of triplicates or greater, the mean value for each microRNA is determined and used to calculate the differences in levels. A microRNA value is determined to be differentially expressed when there is a difference between patient value and control value with a P value of ≤0.05. Other P values may be selected as determined by someone with ordinary skill in the art. It should be understood that a reproductive immunologic disorder or risk of such a disorder can comprise different patterns of differential expression. Further, the time at which a patient is tested may result in a different pattern of microRNA expression and further, patterns may differ with respect to ongoing therapy. For example, T regulatory cells physiologically increase following conception. It would be expected that microRNAs such as mir-155 which is closely involved in regulation of FoxP3, a transcriptional regulator involved in T regulatory function, would be differentially regulated between pre and post conception.

The term "bimodal" or "bimodal distribution" will have the meaning commonly understood by those of ordinary skill in the art of statistics. Histograms of data that comprise two peaks are referred to as "bimodal" while those with a single peak are referred to as "unimodal". Erhard Reschenhofer of the University of Vienna in the Journal of Statistics Education, 9(1) (2001) http://www.amstat.org/publications/ jse/v9n1/reschenhofer.html) downloaded 10-24-2011) formally defines "bimodality" and provides statistical tests for determining bimodality. It is clear that not all distributions with two peaks, particularly overlapping peaks are bimodal. They must be clearly separable. Bimodality can be assessed less formally by inspection of a histogram of the data. Where there is significant overlapping of the ranges as determined from the means and standard deviations of the two peaks, bimodality cannot be claimed.

The embodiments discussed herein are primarily discussed in terms of bimodality such that dichotomous groups groups exist. However, it should be understood that if multiple patient groups are distinguishable using the techniques of the disclosure, the principles will still apply.

The term "heuristic" as used herein shall refer to experience-based techniques for problem solving. More specifically, it shall comprise techniques designed for solving problems based on experience such as those comprised in a database. Moreover, the techniques may involve a process of continual refinement wherein a problem-solving model is continually updated based upon accrual of additional data into the database. These techniques may be incorporated into computer algorithms.

As used herein the term "making a diagnosis" or equivalent term as used herein shall refer to the aggregate of methods used by an individual, preferably a physician skilled in the art of reproductive medicine, shall mean predicting a clinical outcome with or without treatment, selecting treatment and monitoring treatment utilizing measurement of one or more microRNA levels or profiles derived from immune cells comprised in one or more biologic samples of the patient and compared with appropriate controls. It is further understood that said diagnosis may involve concomitant assessment of other clinical findings together with said assessment of microRNA quantification.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification are approximations that can vary depending upon the desired properties sought to be obtained by the presently disclosed subject matter.

As used herein, the term "about," when referring to a value or to an amount of mass, weight, time, volume, concentration or percentage is meant to encompass variations of in some embodiments ±20%, in some embodiments ±10%, in some embodiments ±5%, in some embodiments ±1 %, in some embodiments ±0.5%, and in some embodiments ±0.1 % from the specified amount, as such variations are appropriate to perform the disclosed methods.

All patents and references whether conventionally cited in the literature or addressed through internet links herein are incorporated in entirety by reference. All technical and scientific terms used within this description shall have the same meaning as commonly understood by those of ordinary skill in the art disclosed herein except where otherwise specifically defined. Following long-standing patent law convention, the terms "a", "an", and "the" refer to "one or more" when used in this application, including the claims. Thus, for example, reference to "a peptide" includes a plurality of such peptides, and so forth.

As referenced above, an unmet need remains. Clinicians may be presented by patients in whom an immunotherapy is thought to be useful. It is well established (see Winger and Reed) that appropriate selection of patients for immunotherapy is central to effective therapeutic intervention. The authors identified appropriate patients for such intervention by the use of various PBMC in vitro markers. In the present invention, Winger and Reed have quantified various microRNAs and patterns of microRNA change in PBMCs at various time points prior to and following immunotherapeutic intervention. These microRNA "signatures" support the clinical diagnosis, through identification of candidates for particular therapeutic intervention(s), and prognosticate outcome in patients with various disorders, for example, pregnancy-related disorders. Moreover, it is contemplated that the diagnostic procedures of the present invention may be applied to different clinical conditions and different immunotherapeutic interventions. Their use simplifies complex diagnostic strategies into a single procedure and provides information heretofore unavailable.

While initial studies and examples described herein substantially relate to pregnancy and disorders of the reproductive system of women, these studies should be regarded as exemplary of the broader application of the present invention to other disease states involving other organ systems. Moreover, while some descriptions relate to changes in the expression of one or more microRNAs before and after a selected intervention, it is understood that the present invention is applicable to measurements made at a single time point whether before or after a contemplated intervention.

A novel aspect of the present invention is the separation of patients into groups distinguishable by characteristic changes in single or multiple microRNAs following the selected immunotherapeutic intervention. Identification of patients belonging to microRNA response groups is associated with improved efficacy, prognosis and utility of particular immunotherapeutic intervention(s). Moreover, quantitative levels of certain microRNAs and patterns of change within microRNAs may predict patient response group(s) and post-therapy levels may have additional predictive value. Use of microRNA patterns responsive to therapeutic intervention or predictive thereof provides useful insights into management unavailable through identification of markers directly related to the pathologic process.

The presence, absence or level of the DNA-interacting proteins is the primary regulator of the effect of such native DNA interactions. With respect to purification, it is clear that such interactions may occur only in the native, unpurified form of the DNA where additional interactions with auxiliary-interacting proteins effecting of a result of such an interaction. The "state" of a cell e.g. proliferation, stressed, differentiation, is not comprised within such sequences but rather the concerted interaction of DNA and the interacting proteins. Separation of DNA by isolation or disruption in situ by denaturing processes including heat or chemicals or by the interaction of invasive polynucleotide probes (locked-DNA, PNAs etc.) disrupts these interactions. In terms of RNA, the effects are even more clear. It is now well established that RNA molecules form non-canonic interactions resulting in unexpected activities such as found in ribozymes. Here essential interactions with specific divalent cations are required for assumption of a catalytic conformation. MicroRNAs require interaction with proteins that must be available in the appropriate format.

The process of discovery involved is a transformative step. The procedure used to discover the microRNAs of this invention involved two testing points, one preceding and following an intervention designed to perturb the system. MicroRNAs were identified that demonstrated markedly different behavior following the perturbing intervention. The intervention of this invention was transformative resulting in a distinguishable response amongst selected microRNAs between different clinical subsets. Moreover, a single testing done prior to intervention reveals multiple microRNAs, mainly the same microRNAs showing distinguishable changes between clinical subgroups may be used to predict membership amongst clinical subgroups.

Quantification of microRNAs provides insights into physiologic and pathologic processes wherein their levels are measurably distinguishable from the "steady-state". The interactions between genetic and environmental factors are understood to result in an expressed phenotype not predictable by genetic factors alone. In the last few years, interactions at the level of microRNAs and environmental factors have become apparent. MicroRNAs are known to play critical roles in biological processes that include cell growth, proliferation, differentiation, development, apoptosis, stress, inflammation and carcinogenesis. (Esquela-Kerscher A., Slack F.J. Oncomirs - microRNAs with a role in cancer. Nat. Rev. Cancer 2006;6:259-269, Catania A.S., et al Vitamins and minerals with antioxidant properties and cardiometabolic risk: controversies and perspectives. Arq. Bras. Endocrinol. Metabol. 2009;53:550-559, Chow W.H., et al Epidemiology and risk factors for kidney cancer. Nat. Rev. Urol. 2010;7:245-257, Das U.N. Obesity: genes, brain, gut, and environment. Nutrition 2010;26:459-473.) Dysfunction of various microRNAs has been associated with various disease processes (Lu M., et al An analysis of human microRNA and disease associations. PLoS One 2008;3:e3420.) Treatment of pathologic conditions can result in the restoration of an altered microRNA expression pattern similar to that seen in unaffected individuals. Alternatively, treatment can result in microRNA expression patterns consequent to specific intervention (s) or type of intervention(s). Identification of affected microRNAs in a physiologic or pathologic process may not be apparent by application of a detection and comparison strategy between affected and non-affected subjects. Differential expression may be revealed by the application of a perturbing event such as a therapeutic intervention. In an example wherein still water smoothly covers an invisible object close to the surface, the regular ripple pattern of a stone tossed into the water near the object might be distorted by the subsurface object revealing its presence. Likewise, the microRNA pattern of a therapeutic intervention might be altered revealing a different pattern(s) of their expression. In the study reported herein, we demonstrate an altered pattern of expression of mir-132 following IVIG therapeutic intervention. An important feature of mir-132 expression following IVIG therapy is its dichotomous nature. This differential microRNA expression unexpectedly reveals two responding patient groups. When microarray analyses of known microRNAs are segregated into two groups by their mir-132 response to IVIG, markedly different patterns of microRNA response are revealed. In a subsequent analyses where patients were grouped by clinical outcomes (healthy, preeclampsia and miscarriage) distinct patterns of microRNA IVIG response were also identified between the separate groups. Environmental effects on microRNA expression have been explored where environmental effects include drug interventions (Yang Q, Qiu C, Yang J, Wu Q, and Cui Q*. miREnvironment Database: providing a bridge for microRNAs, environmental factors, and phenotypes. Bioinformatics 2011 27: 3329-3330, Qiu C, Chen G, and Cui Q*. Towards the understanding of microRNA and environmental factor interactions and their relationships to human diseases. Scientific Reports 2012, 2:318.) An online, searchable database has been developed by the same authors (http://202.38.126.151/hmdd/tools/miren.html, downloaded 10/21/2012).

Multiple different microRNAs may coordinate in their regulation of individual mRNAs such that expression of any particular mRNA is the product of the regulatory effects of the individual microRNAs. MicroRNA expression itself may be regulated in a similar manner by other, upstream microRNAs. Hence a complex network involving microRNAs evolves. Many studies have provided insights into interactions of specific microRNAs and their mRNA targets. However, prediction of the outcomes these specific interactions has been obscured by the complexity of their inter-related interactions. It is thus surprising that change in expression of specific microRNAa enables patient responses to be dichotomized or segregated into multiple groupings of interventional therapies by their change in expression of specific microRNAs. Initial experiments permit the segregation of patients into two distinguishable groups by the pattern of expression of miR-132. In one group, low expression of miR-132 remains low or became somewhat lower following treatment with intravenously administered pooled immunoglobulin (IVIg). The other group presented with a relatively higher concentration of miR-132 but experienced a significant decrease in its level following IVIg therapy. Using this single parameter of discrimination, samples were interrogated from four members of each group for all known human microRNAs before and after IVIg therapy on micro-array: http://www.chem.agilent.com/Library/usermanuals/Public/G4170-90011_miRNA_complete_2.4.pdf

The results are displayed in tables (4-21). They display a collection of microRNAs with different levels of expression comparing values detected prior to and following IVIg therapy. Further, many of these microRNAs as well as additional microRNAs display different absolute levels between the two groups in either single measurements before or after intervention. To exclude the possibility that these findings might have been randomly detected amongst the large number of microRNAs examined, members of the two groups were reassorted so that results of two members of the first group were combined with results of two members of the second group and the remainder of the two groups were likewise combined to form the two sham groups. When the data were examined for similar differential expression of changes between pre- and post-IVIg therapy and for absolute single values, differential expression as noted previously could not be identified.

Additional patient samples were collected and examined by microarray analysis in the same manner as just described where clinical outcomes were known: 1) healthy pregnancy, 2) miscarriage and 3) preeclampsia. Results were combined with similar outcome data from the patients described above.

The presently disclosed subject matter comprises a method for diagnosis of patients with reproductive immunological disorders. It is understood that these disorders additionally may have inflammatory and coagulation aspects. The method comprises providing a biological specimen wherein said specimen comprises cells of the immune system, separating said cells and isolating microRNA from said cells and quantifying the amounts of one or more microRNAs comprised within said cells and comparing the amount of one or more microRNAs to one or more microRNA control levels. The subject is diagnosed as having a reproductive immunologic disorder if the amount of one or more microRNA is differentially expressed.

Another aspect of the invention is the use of microRNAs to distinguish patients whom are likely to respond to a therapeutic intervention from those who are unlikely to respond. In particular, specific microRNAs may identify patients whom are likely to respond to intravenous immunoglobulin therapy (IVIG) from those who are not likely to respond. It is understood that such patient identification extends to patients not suffering reproductive disorders but rather to include all patients in whom IVIG therapy is contemplated. MicroRNA monitoring can be extended to the monitoring of IVIG efficacy in those patient candidates for IVIG therapy for other conditions. Moreover, the invention may be utilized in the same manner to determine the suitablility of other immunotherapeutic agents for example a TNFα blocker such as Humira as well as steroids, intralipid, lymphocyte immunization and IL-1 blockers (Anakinra), drugs. Other systems interact with the immune system wherein drugs moderately them have effects on the immune system, inflammation and coagulation. These include, for example, affecting the rennin-angiotensin system, for example angiotensin converting enzyme inhibitors ("ACE-I"), angiotensin receptor blockers ("ARB"), rennin inhibitors, angiotensin receptor type II agonists (for example "C21") and drugs affecting mevalonic acid synthesis such as HMGCoA reductase inhibitors ("statins"). As used herein each of these therapeutic modalities shall be considered "immunotherapeutic interventions".

Said method constitutes assessment of the quantity of one or more microRNAs wherein quantification of said microRNAs clinically correlates to microRNA present in cells of the immune system within the biologic sample. Thus, wherein a biologic sample is whole blood and microRNA is extracted from whole blood, so long as quantification of individual microRNAs correlates clinically with microRNA present within cells of the immune system, it is regarded as quantification of microRNA wherein said microRNA is isolated from cells of the immune system isolated from whole blood. Quantification of microRNAs from cells of the immune system may require normalization to a standard such as a housekeeping gene. Quantification as meant herein contemplates the use of an internal standard.

In an embodiment of the presently-disclosed subject matter, a method for diagnosis of women with reproductive disorders is disclosed wherein a single microRNA known to be differentially expressed in patients affected by a reproductive disorder is interrogated. The method involves isolating microRNA from immune cells isolated from a biologic specimen and identifying a microRNA known to be differentially expressed in patients with a reproductive disorder comprised within said cells and comparing the subject's microRNA to a microRNA control. The subject is diagnosed as having a reproductive disorder if the microRNA is differentially expressed.

In an embodiment of the presently-disclosed subject matter, a method for diagnosis of women with reproductive disorders is disclosed wherein a plurality of microRNAs known to be differentially expressed in patients affected by a reproductive disorder are interrogated. The method comprises providing a particular microRNA profile identified with a reproductive disorder or risk of reproductive disorders. The method involves isolating microRNA from said cells and identifying a microRNA profile comprised within said cells and comparing the subject's microRNA profile to a microRNA control profile. The subject is diagnosed as having a reproductive disorder if said microRNA profile is seen to be present.

In an embodiment of the presently-disclosed subject matter, a method for diagnosis of women with reproductive disorders is disclosed wherein one or more microRNAs markers are selected from the group consisting of mir-155, mir- 146a, mir-16-1, mir16-2, let7a-1, let7a-2, let7a-3, let7e, let7g, mir-132, mir-9, mir-142-3b, mir-17-92, mir-223, mir-181a are interrogated. The method comprises providing a particular microRNA profile identified with a reproductive disorder or risk of reproductive disorders. The method involves isolating microRNA from said cells and identifying a microRNA profile comprised within said cells and comparing the subject's microRNA profile to a microRNA control profile. The subject is diagnosed as having a reproductive disorder if said microRNA profile is seen to be present.

In another embodiment of the presently-disclosed subject matter, a method for evaluating treatment efficacy and/or the progression of a reproductive disorder is disclosed. The process comprises provision of a plurality of biological specimens over a period of time wherein said specimen comprises cells of the immune system, separating said cells and isolating microRNA from said cells and quantifying the type and amount of multiple microRNAs comprised within said cells and comparing this microRNA profile to one or more microRNA control profiles for the purpose of determining differential expression of the profiles, thereby permitting assessment of the progress of the condition or the efficacy of therapy.

Subjects may be human or other animal. "Reproductive disorders" comprise one or more disorder selected from a group exemplified by but is not limited to premature rupture of membranes, preeclampsia, preterm birth, intrauterine growth restriction, and recurrent pregnancy loss and anti-phospholipid antibody syndrome.

Immunologic dysfunction includes immunologic disorders caused by abnormal immunologic mechanisms, whether humoral, cell-mediated, or both and/or is related to an immune related mechanism such as an inflammatory, complement or coagulation- related condition.

Immunologic dysfunction may increase the risk of bearing children who later develop conditions, for example, asthma, autism, attention deficit hyperactivity disorder (ADHD), Tourette's syndrome, diabetes and schizophrenia wherein said immunologic dysfunction are included within the concept of reproductive and /or immunologic disorders. The invention also contemplates peri-pregnancy periods comprising about one year preceding or following pregnancy. However, it is within the scope of this invention to include the period of time involved in oogenesis. This period may exceed one year prior to pregnancy. As noted in Winger and Reed, the period prior to pregnancy may constitute an immunologic state adverse to pregnancy outcome (Winger EE, Reed JL, Ashoush S, Sapna A, El-Toukhy T, Taranissi M: "Treatment with adalimumab (Humira) and intravenous immunoglobulin (IVIG) improves pregnancy rates in women undergoing IVF. American Journal of Reproductive Immunology, 2009; 61:113-120). Likewise the period following pregnancy may be affected as in, for example, autoimmune diseases such as rheumatoid arthritis, a disorder known to flare following pregnancy. A biologic specimen comprising cells of the immune system from a subject suspected of a reproductive disorder is processed wherein said cells are isolated by a variety of means known to those skilled in the art. In a preferred embodiment, the biologic sample is whole blood. In a more preferred embodiment, cells from the blood are isolated by Ficoll-hypaque density gradient centrifugation in the method taught by Boyum (Boyum A 1983. Isolation of human blood monocytes with Nycodenz, a new non-ionic iodinated gradient medium. Scand J Immunol 17: 429-436). RNA is extracted utilizing a method suitable for extracting short RNA sequences. Preferably said method utilizes a kit optimized for recovery of microRNA sequences such as mirNeasy Mini Kit Qiagen catalogue 217004 following instructions provided. Quantification of microRNA may be determined a variety of techniques known to those skilled in the art. In a preferred embodiment, individual microRNAs are quantified by real-time polymerase chain reaction (PCR). In a more preferred embodiment, a kit provided by SABiosciences provide reagents and methods for individual microRNA's known to be involved in human immunopathologic conditions for said quantification optimized for specific real-time thermocycling equipment such as the Stratagene Mx3005p (catalog MAH-104A) (www.SABioscies.com). Operating instructions for the Stratagene Mx3005p are provided by the manufacturer. Comprised therein are instructions for spectrophotometric quantification of recovered RNA, recommendations for input quantity of RNA and PCR master mix. Quantification may be performed concurrently with quantification of a "housekeeper gene" (a gene that is expressed with relative constancy in the cells being interrogated thereby permitting relative quantification). Housekeeping genes may be selected from, for example, beta actin, glyceraldehyde-3P-dehydrogenase (GAPDH), annexin A2 (ANXA2), glutathione S-transferase (GST), ornithine decarboxylase (ODC), hypoxanthine phosphoribosyltransferase (HPRT1), ubiquitin (UBQ glyceraldehyde-3P-dehydrogenase (GAPDH), annexin A2 (ANXA2), glutathione S-transferase (GST), ornithine decarboxylase (ODC), hypoxanthine phosphoribosyltransferase (HPRT1), ubiquitin (UBQ), 18s RNA. The resulting ratio comprises a relative quantity that is independent of the quantity of RNA input into the assay system. This permits comparison with a control sample quantified in a similar manner as a ratio of the analyte signal and the selected housekeeping gene.

MicroRNAs of interest known to participate in immunopathologic conditions comprise but are not limited to mir146a, 1 mir-46b, mir-155, mir-605, mir-623, mir-583, mir-26a, mir-519d, 1 mir-26, 1 mir-6, 3 mir-69-3, Let-7a and 125b mir-126, mir-155, mir-21, Let-7a, let-7c, let-7d, let-7e, let-7g, mir- 214, mir- 409-3p, mir-451, mir-103, mir-105, mir-106a, mir-125a-5p, mir-125b, mir-126, mir-128, mir-130a, mir-132, mir-134, mir-135a, mir-135b, mir-138, mir-142- 3p, mir-142-5p, mir-143, mir-145, mir-147, mir-148a, mir-149, mir-150, mir-15a , mir-15b, mir-16, mir-181a, mir-183, mir-184, mir-185, mir-187, mir-18a, mir-191, mir-195, mir-196a, mir-198, mir-19a , mir-19b, mir-200a, mir-203, mir-205, mir-206, mir-20a , mir-20b , mir-21, mir-214, mir-223, mir-23b, mir-26a, mir-26a ,mir-26b, mir-27a, mir-27b, mir-298, mir-299-3p, mir-29b, mir-29c, mir-302a, mir-302c, mir-30b, mir-30c, mir-30e, mir-31, mir-325, mir-335, mir-34a, mir-369-3, mir-370, mir-379, mir-383, mir-409-3p, mir-46b , mir-493, mir-519d, mir-574-3p, mir-577, mir-583, mir-605, mir-623, mir-9, mir-98, mir-99b.

In a preferred embodiment, one or more microRNAs known to be differentially expressed from control sample values are used. Also preferably, a plurality of microRNAs is identified as indicating a pattern or signature of differentially expressed microRNAs. Determination of said single or plurality of microRNAs may be determined by quantifying microRNA levels in both patients suffering from reproductive disorders or at risk of reproductive disorders in panels of microRNAs and comparing microRNA determining from biologic samples of patients levels with those derived from control samples.

In a preferred embodiment, patient samples are separated into groups. Groups may be defined by their clinical profile or other means. An alternate means, for example and used herein, one or more microRNAs have distinguishable levels amongst patients or may respond to an immunotherapeutic intervention in different ways. For example, and used herein, groups could be distinguished by their response to IVIg therapy by their different response of mir-132. On the basis of their different mir-132 response to IVIg, they could be placed into two groups ("A" and "B"). Subsequently interrogation of samples for their differential absolute expression of microRNAs or their differential microRNA responses to immunotherapeutic intervention can be performed. These methods can be used to define microRNA response patterns between groups established by the above means.

The invention may be used to suggest the suitability of subjects with reproductive and/or immunologic disorders for specific immunotherapies that might mitigate such disorders and may also be used to assess a patient's risk for developing such disorders. Further, the invention may be used to monitor the progress of the disease treatment or monitor reduction in disease risk by providing a series of assays and comparing the results. Serial studies may be performed during, prior to and following pregnancy as well as during the course therapy and compared to determine the success and adequacy of the therapy provided.

Quantification of microRNA from PBMCs permits the investigator or clinician to compare the result to the appropriate control wherein a difference in expression is identified. For example, wherein an microRNA such as mir-155 in a patient with a reproductive disorder or risk of such disorder such as recurrent abortion, is differentially expressed from a control comprised of individuals who have not experienced recurrent abortion, the patient may be diagnosed as having a reproductive disorder and that the patient is a candidate for immunologic intervention such as with a TNF alpha blocker. Patients so diagnosed may be treated as described for patients defined as having similar disorders by Winger and Reed (Edward E. Winger, Jane L. Reed, Treatment with Tumor Necrosis Factor Inhibitors and Intravenous Immunoglobulin Improves Live Birth Rates in Women with Recurrent Spontaneous Abortion, 60(1), 8 - 16, Published Online: 28 Jun 2008, Edward E. Winger, Jane L. Reed, Sherif Ashoush, Sapna Ahuja, Tarek El-Toukhy, Mohamed Taranissi, Treatment with Adalimumab (Humira) and Intravenous Immunoglobulin Improves Pregnancy Rates in Women Undergoing IVF, American Journal of Reproductive Immunology 61 (2009) 113-120)

In some embodiments, the cells from which the RNA is extracted shall be PBMCs while in others cells shall be immune cells derived from body tissues such as endometrium, decidua, fetal and placental tissues and secondary lymphoid organs such as lymph node. Mononuclear cells may be further selected by a variety of techniques, for example, by flow cytometric sorting following labeling of the cells with markers such as monoclonal antibodies that permit their segregation into immune cell subtypes. For example, T regulatory cells can be selected following their labeling by monoclonal antibodies such as CD4, CD 127 and CD25 (with or without the addition of CD3) according to the Becton Dickinson Co. (utilizing reagents and equipment from the company according to http://www.bdbiosciences.com/research/tcell/regulatorytcells/ workflow/ identifyingtregs.jsp downloaded 4/28/10. RNA may be extracted from cells isolated cells selected by said means may be prepared by extraction according to instructions from the manufacturer (Qiagen catalogue 763134). microRNA such as for mir-155 may be detected and quantified by PCR (polymerase chain reaction) by the method described by Chen et al. (http://www3.appliedbiosystems.com/cms/groups/ mcb_marketing/documents/generaldocuments/cms_040548.pdf downloaded 5/11/10). Primers and reagents may be selected for individual microRNAs from those described in product overview (http://www3.appliedbiosystems.com/cms/groups/mcb_marketing/ documents/generaldocuments/cms_068884.pdf downloaded 5/11/10). This document provides information teaching the detection and quantification of individual microRNAs.

The method comprises providing a biological sample from a subject with a history of reproductive and/or immunologic disorder or risk of such disorder said sample being derived from immune cells, for example, derived from peripheral blood, and then isolating mononuclear cells as taught by Boyum (Boyum A 1983. Isolation of human blood monocytes with Nycodenz, a new non-ionic iodinated gradient medium. Scand J Immunol 17: 429-436) and then determining the amount of non-coding RNA such as preferably microRNA (microRNAs) and comparing to the amount of the corresponding RNA in the sample to similarly treated biological sample from control individuals. In addition, the method can comprise quantification of a plurality of individual microRNAs from the biological sample and quantifying the individual microRNAs and comparing the amount of microRNAs to corresponding microRNA control levels. The subject is then diagnosed as having a reproductive and/or immunologic disorder or risk of developing such a disorder if there is differential expression in the amount of one or more of the RNAs from the sample as compared to corresponding RNA control levels. In some embodiments, the method further comprises selecting a treatment or modifying a treatment based on the amount of the one or more RNAs determined. This determination may be based upon assessment of specific individual or combinations of the individual microRNAs.

In other embodiments of the presently-disclosed subject matter, a method for evaluating treatment efficacy and/or progression of a patient with a reproductive or immunologic disorder and/or risk of developing a reproductive disorder in a subject is provided. In some embodiments, the method comprises providing a series of biological samples over a time period from a subject, isolating the RNA as described above comprising RNAs from the series of biological samples, determining an amount of one or more of the microRNAs in each of the biological samples from the series and determining any measurable change in the amounts of the one or more microRNAs in each of the biological samples from the series permitting a measure of treatment efficacy and/or progression of the reproductive disorder or risk of reproductive disorder in the subject.

In still other embodiments of the presently-disclosed subject matter, a method for characterizing a reproductive disorder in a subject is provided. In some embodiments, the method comprises providing a biological sample from a subject, isolating the RNA comprising microRNAs from the biological sample, determining an amount of one or more of the RNAs and comparing the amount of one or more microRNAs to corresponding microRNA control levels. The reproductive disorder is then characterized based on differential expression of the amount of the one or more microRNAs from the sample as compared to the one or more microRNA control levels. In some embodiments, the reproductive disorder is characterized when compared with well characterized individuals with known reproductive disorders.

In some of these methods, quantification of microRNAs comprises using a real-time polymerase chain reaction. SABiosciences provide reagents and methods for individual microRNA's known to be involved in human immunopathologic conditions for said quantification optimized for specific real-time thermocycling equipment such as the Stratagene Mx3005p (catalog MAH-104A) (www.SABioscies.com). Quantification may be performed concurrently with quantification of a "housekeeper gene" (a gene that is expressed with relative constancy in the cellular material being interrogated permitting standardization). Further, in some embodiments of these methods, the mirRNAs are one or more microRNAs. Amongst microRNAs quantified include but are not limited.

MicroRNAs, their flanking regions within pre-miRNA or in target sites have been demonstrated to affect physiologic and pathologic processes. Single nucleotide polymorphisms (SNP) within both microRNAs and their flanking regions and target mRNAs may alter target specificity resulting in loss or diminished effect between wild-type species and SNP-comprising counterparts. Further such polymorphisms may generate new mRNA targets interactions. (Gong J, Tong Y, Zhang HM, Wang K, Hu T, Shan G, Sun J, Guo AY, Genome-wide identification of SNPs in microRNA genes and the SNP effects on microRNA target binding and biogenesis Human Mutation (2012) 33(1):254-63. doi: 10.1002/humu.21641. Epub 2011 Nov 23) These polymorphisms may be result in altered efficiency of microRNA regulation of target mRNAs. (Jazdzewski K, Murray EL, Franssila K, Jarzab B, Schoenberg DR, de la Chapelle A. Common SNP in pre-mir-146a decreases mature mir-expression and predisposes to papillary thyroid carcinoma. Proc Natl Acad Sci USA 2008;105:7269-74.) Further, Polymorphisms may potentially affect microRNA-mediated regulation of the cell can be present in the 3'-UTR of a microRNA target gene. Additional polymorphisms may also be present in the genes involved in microRNA biogenesis as well as in pri-, pre- and mature-microRNA sequences. The consequences of such polymorphisms in processed microRNAs may have profound effects on the expression of a multiplicity of target genes and have serious consequences, whereas a polymorphism in microRNA target site, in the 3'-UTR of the target mRNA, may be more target and/or pathway specific (Prasun J Mishra and Joseph R Bertino, MicroRNA polymorphisms: the future of pharmacogenomics, molecular epidemiology and individualized medicine, Pharmacogenomics. 2009 March; 10(3): 399-416.doi: 10.2217/14622416.10.3.399).

Detection of such polymorphisms by such techniques as real-time allele discrimination, for example, is also within the scope of this invention. Methods can be found in Mx3000P instruction manual (www.bio.davidson.edu/courses/Bio343/ Mx3000P_Manual.pdf downloaded 10/24/10)). Recognition of allele interactions with wild and SNP-comprising targets has been catalogued. See http://www.bioguo.org/miRNASNP/index.php, (downloaded 10/21- 2012) for example. The invention is particularly well suited for use in personalized medicine. Nucleic acid characterization and quantification are used to assess the probability of success of a particular therapeutic intervention. It is the goal of personalized medicine to identify patients whom are likely, or conversely unlikely to respond to a candidate therapy. Cost, side-effects and improved therapeutic response are accepted reasons for pursuing nucleic acid testing as a means of selecting therapies and for following the course of therapy. Not only might quantification of microRNAs be helpful in identifying patients suffering from reproductive disorders, but such quantifications would be of corresponding assistance in selecting and directing therapeutic choices and monitoring their effects in a virtually unlimited variety of disorders.

The ability to separate patients into groups of two or more by characteristics of their response to an intervention such as for example immunotherapy for example IVIG, permits more specific prediction of therapeutic response to the specific intervention and also may permit prediction of response to other interventions. Another aspect of such separation is better prognostication and vulnerability to other disorders for example autoimmune diseases. If a microRNA were identified whose response pattern amongst the patient cohort was bimodal, then patients could be grouped according to their response into said groups.

The invention comprises collecting immune cells, preferably PBMCs, before and after an intervention, preferably immunotherapeutic, for example IVIG, extracting microRNA-comprising RNA from said cells, quantifying microRNAs within the extracted RNA, determining whether one or more microRNAs quantified display a bimodal response amongst a statistically sufficient number of patient samples. If a bimodal response pattern is demonstrated in one or more microRNAs, then patients may be segregated into groups according to their response.

A "response" as used herein is defined as the difference between a result on a first sample and a second sample wherein there is an intervening intervention or the intervening effect of an intervention previously made. It may be an increase, a decrease or an absence of change. The term "bimodal" or bimodality" as used herein refers to a non-normal distribution of responses wherein two distinct modes characterize the data. The data can be displayed graphically in a histogram and bimodality assessed by inspection. Statistical methods have been developed to discern bimodality. A first step is the recognition of clustering of results into two populations. While recognition of the separation point between the two populations is commonly done by visual inspection of histograms of the data, statistical methods can be applied by one of ordinary skill in the art of statistics. The mean and standard deviation of the two clusters are calculated. In a preferred definition of bimodality data are considered bimodal if the means of the two clusters are equal or further apart than the sum of the standard deviations of the two clusters (Schilling, Watkins and Watkins, "Is Human Height Bimodal?", The American Statistician (2002), 56(3): 223-229).

Populations are regarded as dichotomous with respect to the microRNA responses where a bimodal distribution amongst responses of the test cohort can be demonstrated for one or more microRNA. It is understood that not all patient cohorts are dichotomous with respect to their response to the individual microRNA responses following any given intervention. When a bimodal response is found in one or more microRNAs, then the patients populations are regarded as dichotomous.

In a preferred embodiment, an individual of ordinary skill in the art using a human microRNA array from Agilant Technologies (for example cat. G4471A-029297) and following the directions of the manufacturer) quantifies all known human microRNAs on specimens RNA extracted from PBMCs according to instructions of the microarray manufacturer. Blood collected is drawn into heparinized tubes and maintained at room temperature preferably for approximately 24 hours prior to isolation of PBMCs. RNA sampling and extraction: PBMC or sorted cell populations (< 1x10^7 viable cells) were collected in 1ml TRIzol (Invitrogen) and stored at -80c until use). Total RNA was isolated according to the TRIzol protocol (Invitrogen) or RNeasy Mini Kit (Qiagen). For using the RNeasy Mini Kit, the entire procedure was carried out at room temperature with the QIAcube automated robot (Qiagen). Total RNA yield was assessed using the Thermo Scientific NanoDrop 1000 micro-volume spectrophotometer (absorbance at 260 nm and the ratio of 260/280 and 260/230). RNA integrity was assessed using the Agilent's Bioanalyzer NANO Lab-on-Chip instrument (Agilent). MicroRNA Microarray processing. The microRNA microarray data was normlized by using the Agilent's GeneSpring GX v11.5.1 (see the link) (http://www.chem.agilent.com/en-US/Products-Services/Software-Informatics/GeneSpring-GX/pages/default.aspx) downloaded 10/7/2012. Testing is performed on specimens drawn preferably one to three weeks or less prior to and one to three weeks or less following a therapeutic intervention. In a preferred embodiment, a statistically significant number of patients are selected who have similar demographic and clinical characteristics such as age, sex, and clinical condition, e.g. recurrent pregnancy loss. It is the goal of analysis of these data to segregate patients into groups of two or more. In a more preferred embodiment, it is the goal to dichotomize patients into two groups each possessing a unique microRNA profile.

A variety of methods are suitable for determining unique microRNA profiles defining each group. In a first step, the differences between each microRNA sampled prior to therapy and subsequent to therapy are calculated. The means and standard deviations of each of differences between the various "before and after" sample subgroups are calculated and microRNAs sorted in order of statistical significance. Subgroups with microRNAs with the most statistically significant difference in mean and SD are selected (Graphpad software t test). By inspection one identifies a microRNA where the results are distributed dichotomously. Patient results are then sorted into two groups as determined by the group into which that selected microRNA falls.

As discussed below, a bimodal hsa-mir-132 response is shown amongst a cohort of seventeen patients. The RNA before and after specimens from patients from each the two cohorts defined by the bimodal distribution of hsa-mir-132 were subsequently assessed in a microarray of all "known" or suspected human microRNAs. As such, hsa-mir-132 is identified as meeting the above stated criterion.

Seventeen female patients (average age 35.8±4.8 years) being seen at the Alan E. Beer Center for recurrent miscarriage and infertility and being treated with intravenous immunoglobulin (IVIG) (average 1.5±1.8 prior miscarriages; 1.6 ±1.5 prior IVF failures) were selected. Each patient had signed a consent permitting their blood to be used for research purposes. Each patient selected for the study had a blood draw an average of 13.2±6.0 days prior to IVIG therapy and an average of 11.8±5.6 days following IVIG therapy (an average of 25.1±7.9 days between microRNA blood draws). Blood was drawn as a part of routine blood studies performed on patients. PBMCs were isolated from twenty-four to forty-eight hour old heparinized blood that had been maintained at room temperature by Ficoll-Hypaque density gradient centrifugation. Approximately 10x10⁶ cells were preserved in one ml Trizol (Invitrogen) and maintained at -20° C until used. Table 1 summarizes the characteristics of the patients.

**Table 1**

| Population parameters | Mean±SD |
|---|---|
| Age (yrs) | 35.8±4.8 |
| #prior miscarriages | 1.5±1.8 |
| #prior IVF failures | 1.6±1.5 |
| #days prior to IVIG therapy (Sample 1) | 13.2±6.0 |
| #days after IVIG therapy (Sample2) | 11.8±5.6 |
| #days between Sample 1 and 2 | 25.1±7.9 |

The PBMCs from the seventeen patients were then interrogated for mir-16, mir-132, mir-146a, mir-155, mir-181a, mir-196a, mir-223 using RNU48 as a housekeeping gene for purposes of normalization on reversed transcribed RNA by real-time PCR. Each patient had been treated with intravenous immunoglobulin (IVIG) therapy and blood collected prior to and following therapy and the microRNAs quantified in each sample. Candidate microRNAs were selected from review of the literature.The microRNAs were selected based on the studies in autoimmune /inflammatory disorders (Lupus and rheumatoid arthritis on PBMCs. (mir16, mir-132, mir146a, mir155, mir181a, mir196a and mir223 were selected based information from the following articles: (1)Pauley KM, Satoh M, Chan AL, Bubb MR, Reeves WH, Chan EK. Upregulated mir-146a expression in peripheral blood mononuclear cells from rheumatoid arthritis patients. Arthritis Res Ther. 2008;10(4):R101.; (2)Dai Y, Huang YS, Tang M, Lv TY, Hu CX, Tan YH, Xu ZM, Yin YB. Microarray analysis of microRNA expression in peripheral blood cells of systemic lupus erythematosus patients. Lupus. 2007;16(12):939-46.; (3) Fehniger TA, Wylie T, Germino E, Leong JW, Magrini VJ, Koul S, Keppel CR, Schneider SE, Koboldt DC, Sullivan RP, Heinz ME, Crosby SD, Nagarajan R, Ramsingh G, Link DC, Ley TJ, Mardis ER. Next-generation sequencing identifies the natural killer cell microRNA transcriptome. Genome Res. 2010 Nov;20(11):1590-604.)

Blood samples are collected in EDTA-treated tubes and PBMCs are isolated by standard Ficoll density-gradient cenrifugation according to the procedure known to those of ordinary skill in the art. Alternatively, the procedure utilizing Accupsin Tubes from Sigma-Aldrich following the manufacturer's procedure (procedure no AST-1) may be followed. PBMCs are washed once in sterile phosphate- buffered saline (PBS) before RNA isolation. Total RNA is isolated from freshly obtained PBMCs using the mir- Vana microRNA Isolation kit (Ambion, Austin, TX, USA), in accordance with the manufacturer's protocol. RNA concentrations are determined by absorption spectroscopy due to the peak absorption of DNA and RNA at 260 nm. 10 ng of each RNA sample are used for quantitative real-time RT-PCR (qRT-PCR). microRNA qRT-PCR was performed using the TaqMan MicroRNA Reverse Transcription Kit, TaqMan Universal PCR Master Mix, and TaqMan MicroRNA Assay (Applied Biosystems, Foster City, CA, USA.) Primers from SABiosciences for these specific human microRNAs were used: mir-16 (MPH00062A), mir-132 (MPH01167A), mir-146a (MPH00047A), mir-155 (MPH00059A), mir-223 (MPH01231A), let-7a (MPH00001A). mRNA qRT-PCR may be performed using the TaqMan High-Capacity cDNA Reverse Transcription Kit, TaqMan Fast PCR Master Mix, and TaqMan mRNA assay primers (Applied Biosystems). See http://www3.appliedbiosystems.com/cms/groups/ mcb_support/documents/ generaldocuments/cms_042167.pdf. Reactions may be analyzed using StepOne Real-Time PCR System (Applied Biosystems). The levels of microRNA is normalized to 18S RNA, for example. The cycle threshold (Ct) values, corresponding to the PCR cycle number at which fluorescence emission reaches a threshold above baseline emission, were determined and the relative microRNA or mRNA expression was calculated using the 2-ΔΔCt method (Applied Biosystems User Bulletin No. 2)

Based on the literature, a coordinate change in microRNAs could be expected. Moreover, changes in the range of several fold between samples would also be expected. For example, changes in mir-146a might be expected from the above Pauley reference. Changes in mir-16 could also be anticipated. However, unexpectedly, a single microRNA, mir-132 was suppressed up to approximately 100-fold. IVIG therapy appears to suppress the expression of mir-132 very specifically and very markedly. Further, initial values varied significantly between patients by up to approximately 100 fold. Table 2 shows that amongst 17 patients in whom before and after studies were performed, IVIG treatment resulted in a statistically significant decrease.

**Table 2**

| 17 patient sequential cases | Mean mir-132 CT levels (mean±SD) |
|---|---|
| Before IVIG | 22.8±4.7* |
| After IVIG | 26.7±2.1* |
| Difference | +3.9±4.0 |

| | |
|---|---|
| *p=0.004 | |

As discussed herein, the treatment group may be divided into two groups on the basis of the expression pattern one microRNA, mir-132, prior to IVIG therapy. Group A had low initial CTs (threshold crossing) indicating relatively high initial levels of hsa-mir-132. The remaining patients, Group B, had high initial CTs indicating low levels of hsa-mir-132. Following IVIG treatment, both groups converged in their levels of hsa-mir-132 to high CTs indicating significantly diminished levels of mir-132. The changes were substantially greater in Group A than in Group B. IVIG appears to have been most effective in lowering mir-132 in the group that had the highest levels of mir-132 pretreatment.

Although both groups responded statistically to IVIG treatment, Table 3 dichotomizes patients by their initial hsa-mir-132 CT. It can be seen that the initial CTs cluster into those with relatively low CTs (high concentrations of hsa-mir-132) and those with relatively high CTs (low levels of hsa-mir-132). Following IVIG therapy, the two groups converge such that the CT change in the low CT group is significantly greater than that of the high CT group. Accordingly, an embodiment of the invention is the separation of the patients into two distinct groups (dichotomization) on the basis of microRNA expression.

**Table 3**

| | Low mir-132 group: 6 cases (Sample1 >21.0) | High mir-132 group: 11 cases (Sample 1 <19.0) |
|---|---|---|
| Mean initial mir-132 value (Sample 1) | 17.1±0.8 | 25.9±2.2 |
| Mean post-IVIG mir-132 value (Sample 2) | 25.7±1.2 | 27.4±2.2 |
| Mean microRNA change | +8.5±1.2** | +1.5±2.4** |

| | | |
|---|---|---|
| **p<0.0001 | | |

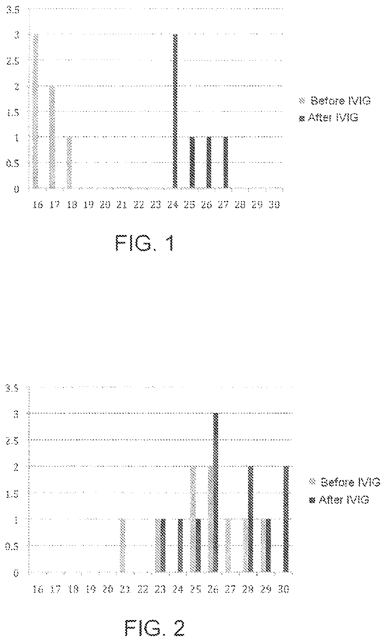

These results are represented in Figs. 1 and 2, which show the CT levels of the patients before and after IVIG treatment, for Groups A and B respectively. As shown in Fig. 1, prior to IVIG treatment, Group A exhibited CT levels of 16-18 and exhibited levels of 24-27 after treatment. On the other hand, Fig. 2 shows that Group B exhibited levels of 21-29 before treatment and levels of 23-30 after treatment.

Those patients with initially very low levels had modest to very small degrees of suppression. Following IVIG therapy, mir-132 values from both the patients groups appeared to converge at relatively the same low microRNA activity (or high CT) level (CT value between 23-28). It appears that the effect of IVIG on mir-132 expression is largely confined to those patients with initially relatively large quantities of mir-132 (those with low CT levels). This population has an initial mir-132 CT range between 16-18. Thus IVIG therapy is most effective in suppression of mir-132 in the group of patients with the highest pre-treatment levels of mir-132.

Using hsa-mir-132 to identify patients belonging to two discrete groups as described above, four patients from each of Group A and Group B were then assessed by an Agilant microarray comprising all of the known human microRNAs (approximately 900 microRNAs).

Data from each of the two groups were assessed separately and each group sorted by the mean differences between the first and second quantification of the specific microRNA for each group separately and listed in order of decreasing differences. From the said lists, the differences that are most increased in a first group are compared to the most decreased in a second group. Conversely the most decreased in a first group are compared to the most increased in a second group. Those individual microRNAs that display the greatest differences between the means of the differences in each group are considered to be provisionally optimum markers. The power of these optimum microRNAs are further assessed by the sum of the standard deviations of the means of the respective groups to confirm their status as optimum markers wherein the ratio of the differences is divided by the sum of the standard deviations. Optimum markers have the highest ratios. For example, all of the microRNAs listed below in Tables 4-6 exhibit ratios with an absolute value greater than or equal to one. More preferably, microRNAs may be selected that exhibit a ratio with an absolute value greater than or equal to two.

The data in Table 4 identifies useful microRNA markers meeting the above criteria. Hereinafter, a value is "decreased" wherein the reported difference between a first value minus a second value is negative and "increased" wherein a first value minus a second value is positive.

**Table 4**

| Selected MicroRNA markers | Group A Mean difference after IVIG | Group A Mean SD | Group B Mean difference after IVIG | Group B Mean SD |
|---|---|---|---|---|
| hsa-mir-136 | 2.52122 | 1.497334 | -5.43651 | 0.82098 |
| hsa-mir-141 | 1.083703 | 0.288785 | -4.77102 | 0.469596 |
| hsa-mir-142-5p | 1.041446 | 0.142776 | -5.94462 | 0.44929 |
| hsa-mir-144 | 0.335061 | 1.466308 | -5.88934 | 1.274726 |
| hsa-mir-153 | 2.082454 | 0.849342 | -7.84218 | 0.691729 |
| hsa-mir-1537 | 1.301357 | 0.995047 | -7.47155 | 1.143166 |
| hsa-mir-193a-3p | 0.952666 | 0.441789 | -4.08226 | 0.877131 |
| hsa-mir-219-5p | 2.083515 | 1.609553 | -6.60915 | 0.991381 |
| hsa-mir-29b | 1.068173 | 0.318097 | -5.26843 | 0.506796 |
| hsa-mir-301a | 1.140525 | 0.150147 | -4.74399 | 0.497264 |
| hsa-mir-32 | 1.757013 | 0.663302 | -6.60811 | 0.775835 |
| hsa-mir-33a | 2.208455 | 0.541141 | -6.66695 | 0.528549 |
| hsa-mir-545 | 1.955594 | 1.558846 | -6.95672 | 0.388828 |
| hsa-mir-582-3p | 0.885343 | 0.493095 | -5.63284 | 1.148245 |
| hsa-mir-590-5p | 1.195371 | 0.219509 | -5.34069 | 0.410219 |
| hsa-mir-1181 | -1.82126 | 1.984385 | 4.492508 | 1.085892 |
| hsa-mir-513a-5p | -0.90253 | 1.698756 | 8.720988 | 1.695736 |

As can be seen from inspection of the listed microRNAs from each of the two groups, two distinct observations can be made. First, the values moved in opposite directions following IVIG therapy in the two groups. For example, hsa-mir-136 increases following IVIG while it decreases in group B. Conversely, hsa-mir-513-5p decreases in group A while it increases in group B. Second, taken as a single group, the standard deviations are quite broad while the standard deviations within each group, A or B, are greatly diminished. These findings confirm the recognition of two distinct groupings of patients. From a single microRNA result for a patient, one can assign the patient to one of the two groups statistically. One of ordinary skill in the art can assign patients to the appropriate group.

Additional sequences have been identified that may be used separately or in combination to define members of group A or B are selected on the basis of demonstrating opposite behavior after IVIG. Table 5 lists common microRNAs selected from the top 100 most increased in Group A and 100 most decreased in Group B while Table 6 lists common microRNAs selected from the top 100 most decreased in Group A and 100 most increased in Group B.

**Table 5**

| MicroRNA | Group A Mean Difference | Group A SD | Group B Mean Difference | Group B SD |
|---|---|---|---|---|
| hsa-mir-1 | 1.270745 | 1.003145 | -3.44195 | 2.957159 |
| hsa-mir-101 | 0.698965 | 0.235172 | -4.10335 | 0.423924 |
| hsa-mir-1183 | 0.332102 | 2.511271 | -7.18912 | 2.240812 |
| hsa-mir-1249 | 0.511253 | 0.559915 | -2.90416 | 0.69329 |
| hsa-mir-136 | 2.52122 | 1.497334 | -5.43651 | 0.82098 |
| hsa-mir-140-5p | 0.419807 | 0.265255 | -2.03009 | 0.349193 |
| hsa-mir-141 | 1.083703 | 0.288785 | -4.77102 | 0.469596 |
| hsa-mir-142-3p | 1.0677 | 0.202879 | -3.59343 | 0.583092 |
| hsa-mir-142-5p | 1.041446 | 0.142776 | -5.94462 | 0.44929 |
| hsa-mir-144 | 0.335061 | 1.466308 | -5.88934 | 1.274726 |
| hsa-mir-153 | 2.082454 | 0.849342 | -7.84218 | 0.691729 |
| hsa-mir-1537 | 1.301357 | 0.995047 | -7.47155 | 1.143166 |
| hsa-mir-15a | 0.533715 | 0.100281 | -2.8992 | 0.322076 |
| hsa-mir-18a | 0.382843 | 0.13646 | -1.92863 | 0.464262 |
| hsa-mir-18b | 0.405855 | 0.187818 | -2.19576 | 0.531698 |
| hsa-mir-193a-3p | 0.952666 | 0.441789 | -4.08226 | 0.877131 |
| hsa-mir-19a | 0.872497 | 0.133295 | -4.3393 | 0.511408 |
| hsa-mir-19b | 0.457604 | 0.158196 | -2.57291 | 0.322324 |
| hsa-mir-21 | 0.441811 | 0.278029 | -2.1423 | 0.382552 |
| hsa-mir-219-5p | 2.083515 | 1.609553 | -6.60915 | 0.991381 |
| hsa-mir-27a | 0.53133 | 0.25451 | -2.12834 | 0.263011 |
| hsa-mir-29b | 1.068173 | 0.318097 | -5.26843 | 0.506796 |
| hsa-mir-29c | 0.443375 | 0.117018 | -2.75245 | 0.365226 |
| hsa-mir-301a | 1.140525 | 0.150147 | -4.74399 | 0.497264 |
| hsa-mir-301b | 0.976275 | 0.357315 | -3.26383 | 0.442348 |
| hsa-mir-30e | 0.59185 | 0.144703 | -3.24738 | 0.251854 |
| hsa-mir-32 | 1.757013 | 0.663302 | -6.60811 | 0.775835 |
| hsa-mir-324-5p | 0.698976 | 0.359357 | -1.87021 | 0.15051 |
| hsa-mir-335 | 0.617394 | 0.711001 | -2.4438 | 0.722963 |
| hsa-mir-337-5p | 1.10453 | 1.617308 | -4.14723 | 2.179625 |
| hsa-mir-338-3p | 0.391677 | 0.0348 | -3.00793 | 0.498721 |
| hsa-mir-33a | 2.208455 | 0.541141 | -6.66695 | 0.528549 |
| hsa-mir-340 | 0.830669 | 0.481914 | -4.16497 | 0.300849 |
| hsa-mir-362-3p | 0.8536 | 0.294971 | -3.28599 | 0.665745 |
| hsa-mir-371-5p | 0.971021 | 1.083568 | -2.46387 | 1.359333 |
| hsa-mir-376b | 0.46162 | 1.952296 | -6.23567 | 1.991346 |
| hsa-mir-374a | 0.363715 | 0.19741 | -2.06665 | 0.272203 |
| hsa-mir-377 | 0.873289 | 1.253133 | -1.92671 | 1.06699 |
| hsa-mir-421 | 0.824237 | 1.119436 | -2.52667 | 0.979143 |
| hsa-mir-424 | 0.676429 | 0.131418 | -3.30699 | 0.509319 |
| hsa-mir-542-3p | 0.835738 | 0.202614 | -5.38285 | 0.608003 |
| hsa-mir-545 | 1.955594 | 1.558846 | -6.95672 | 0.388828 |
| hsa-mir-548c-5p | 0.710325 | 0.382632 | -1.977 | 0.399167 |
| hsa-mir-551b | 1.132591 | 0.828951 | -2.30701 | 0.520072 |
| hsa-mir-582-3p | 0.885343 | 0.493095 | -5.63284 | 1.148245 |
| hsa-mir-590-5p | 1.195371 | 0.219509 | -5.34069 | 0.410219 |

**Table 6**

| MicroRNA | Group A Mean difference | Group A SD | Group B Mean difference | Group B SD |
|---|---|---|---|---|
| hsa-mir-100 | -0.75803 | 1.061571 | 1.154144 | 0.766084 |
| hsa-mir-1181 | -1.82126 | 1.984385 | 4.492508 | 1.085892 |
| hsa-mir-1227 | -0.36135 | 0.981072 | 3.887691 | 0.570974 |
| hsa-mir-1271 | -0.49835 | 0.575051 | 1.251907 | 0.418329 |
| hsa-mir-127-3p | -1.07828 | 1.295253 | 1.312743 | 0.450348 |
| hsa-mir-1275 | -0.47159 | 0.576344 | 0.968348 | 0.519534 |
| hsa-mir-1300_v13.0 | -1.27118 | 1.97 | 2.768853 | 0.297402 |
| hsa-mir-1307 | -0.41137 | 1.091938 | 5.269371 | 0.913262 |
| hsa-mir-139-3p | -0.37258 | 0.494547 | 2.047472 | 0.768133 |
| hsa-mir-181a-2* | -0.39928 | 0.430931 | 2.779431 | 0.677482 |
| hsa-mir-182 | -0.53534 | 0.745866 | 3.998238 | 0.721076 |
| hsa-mir-191 | -0.60784 | 0.657613 | 3.506353 | 0.577494 |
| hsa-mir-224 | -0.5147 | 1.993012 | 2.390506 | 1.670223 |
| hsa-mir-300 | -1.48121 | 1.881716 | 2.084424 | 1.659764 |
| hsa-mir-339-5p | -1.7283 | 1.326469 | 1.405327 | 0.459023 |
| hsa-mir-483-3p | -0.58871 | 2.243166 | 2.07591 | 0.843905 |
| hsa-mir-486-5p | -1.20003 | 1.525774 | 1.160433 | 1.15879 |
| hsa-mir-501-5p | -0.48667 | 1.118063 | 4.393115 | 0.758058 |
| hsa-mir-513a-5p | -0.90253 | 1.698756 | 8.720988 | 1.695736 |
| hsa-mir-564 | -0.38684 | 0.709046 | 1.351019 | 0.832139 |
| hsa-mir-602 | -0.45617 | 0.664475 | 1.969851 | 0.830315 |
| hsa-mir-630 | -1.20769 | 3.331194 | 0.968519 | 2.660711 |
| hsa-mir-647 | -0.84299 | 1.117714 | 4.483333 | 1.436393 |
| hsa-mir-770-5p | -0.57186 | 1.473769 | 1.18536 | 0.33416 |
| hsa-mir-885-5p | -0.47354 | 3.442087 | 3.238278 | 1.381079 |
| hsa-mir-892b | -0.48425 | 1.487033 | 2.127233 | 0.882215 |
| hsa-mir-92b | -0.5896 | 0.830008 | 5.23262 | 0.53149 |

Identification of the dichotomized groups can be determined from a single microRNA assay performed. As can be seen from above, hsa-mir-132 discretely separates the A and B groups. Additional sequences tabulated in table 6 provide additional sequences that may be used to define the two groupings. They may be used singly or in combination. The dichotomous nature of a population can be ascertained from selection of microRNAs that display large standard deviation. These microRNAs are likely to comprise a bimodal distribution of microRNA means. Inspection or application of statistical tests by one of ordinary skill in the art of statistics can verify bimodality.

To test the hypothesis that patients can be divided into dichotomous groups wherein individual microRNAs can be identified that respond differently to a therapeutic intervention, a sham grouping of the same patients wherein dichotomous groupings demonstrated differential microRNA response as seen in tables 4-6, were constructed. In the above examples, patients were designated as group A or B according to the response of hsa-mir-132 following IVIG therapy. Each group comprised four patients. In the sham experiment, two patients were arbitrarily selected from Group A and two from Group B to comprise a sham group C while the remaining two patients from group A and the two remaining patients from group B comprise a sham group D. The differences in the means and standard deviations before and after therapy were calculated and displayed as the 30 most increased following therapy and the 30 most decreased as shown in Table 7.

**Table 7**

| MicroRNA | Mean Δ Group C | SD | MicroRNA | Mean Δ Group D | SD |
|---|---|---|---|---|---|
| hsa-miR-24-1 * | 1.139664 | 1.751306 | hsa-miR-34b* | -0.495378 | 0.661462 |
| hsa-miR-513b | 1.10647 | 1.745331 | hsa-miR-1207-5p | -0.499623 | 0.345578 |
| hsa-miR-513a-5p | 1.081663 | 2.387287 | hsa-miR-1274a | -0.507595 | 0.863872 |
| hsa-miR-513c | 1.006773 | 1.331825 | hsa-miR-1280 | -0.516564 | 0.713269 |
| hsa-miR-345 | 0.873768 | 1.69969 | hsa-miR-28-3p | -0.525916 | 0.890524 |
| hsa-miR-892b | 0.823898 | 0.561379 | hsa-miR-181a* | -0.526303 | 0.449876 |
| hsa-miR-1826_v15.0 | 0.753516 | 0.90899 | hsa-miR-1226* | -0.537768 | 0.995808 |
| hsa-miR-501-5p | 0.721953 | 1.183809 | hsa-miR-582-5p | -0.538055 | 0.930185 |
| hsa-miR-760 | 0.720185 | 1.313588 | hsa-miR-500a | -0.539322 | 0.799103 |
| hsa-miR-92b | 0.719088 | 0.986911 | hsa-miR-133a | -0.553997 | 1.031349 |
| hsa-miR-23a* | 0.711232 | 0.624211 | hsa-miR-720 | -0.570416 | 0.810315 |
| hsa-miR-607 | 0.694857 | 0.669177 | hsa-miR-1260 | -0.575785 | 0.947614 |
| hsa-miR-338-5p | 0.694838 | 0.66914 | hsa-miR-135a* | -0.577329 | 1.032842 |
| hsa-miR-665 | 0.657335 | 0.594854 | hsa-miR-92a-1* | -0.595985 | 0.496119 |
| hsa-miR-576-3p | 0.640433 | 0.562254 | hsa-miR-1202 | -0.62187 | 0.826414 |
| hsa-miR-574-3p | 0.636028 | 1.161566 | hsa-miR-194* | -0.632359 | 1.510496 |
| miRNABrightCorn er30 | 0.619267 | 0.43915 | hsa-miR-629* | -0.635601 | 1.061238 |
| hsa-miR-494 | 0.574397 | 0.384558 | hsa-miR-26b* | -0.66143 | 0.511278 |
| hsa-miR-1288 | 0.572928 | 0.526309 | hsa-miR-192* | -0.672978 | 1.370112 |
| hsa-miR-378 | 0.508068 | 0.394278 | hsa-miR-630 | -0.679997 | 2.042544 |
| hsa-miR-139-3p | 0.463569 | 0.641463 | hsa-miR-183 | -0.687276 | 2.202468 |
| hsa-let-7a* | 0.448805 | 0.297109 | hsa-miR-373* | -0.707562 | 1.093235 |
| hsa-let-7c* | 0.448805 | 0.297109 | hsa-miR-1181 | -0.727918 | 2.188832 |
| hsa-let-7e* | 0.448805 | 0.297109 | hsa-miR-557 | -0.759721 | 0.679964 |
| hsa-let-7f-2* | 0.448805 | 0.297109 | hsa-miR-134 | -0.761638 | 0.814211 |
| hsa-let-7g* | 0.448805 | 0.297109 | hsa-miR-892b | -0.78585 | 0.907683 |
| hsa-miR-100* | 0.448805 | 0.297109 | hsa-miR-18b* | -0.803171 | 1.135087 |
| hsa-miR-103-as | 0.448805 | 0.297109 | hsa-miR-188-5p | -0.885158 | 1.808363 |
| hsa-miR-105 | 0.448805 | 0.297109 | hsa-miR-760 | -0.901819 | 1.305335 |
| hsa-miR-105* | 0.448805 | 0.297109 | hsa-miR-150* | -0.93806 | 0.866707 |

Not only were the differences in means between the two sham groups relatively small, but the standard deviations were sufficiently large such that no microRNA was identified that could be considered bimodal. In other words, the absolute value of the differences in means divided by the sum of the standard deviations was less than one for each microRNA using this random grouping. Therefore a random "dichotomization" does not identify separate patient groups as defined in this disclosure.

To predict assignment of patients to a response group, in a sample acquired before or after an intervention, microRNAs are sorted by the maximum difference between microRNA levels between the two groups defined above. The power of these microRNA markers is further assessed by the ratio of the differences in means of the microRNA values divided by the sum of their standard deviations. Tables 8 and 9 comprise a selective list and a more comprehensive list of such microRNAs, respectively, with the means and standard deviations of expression levels before IVIG treatment.

**Table 8**

| Ratio from calculation | microRNA |
|---|---|
| 5.120617 | hsa-miR-548d-5p |
| 4.969583 | hsa-miR-548a-5p |
| 3.438886 | hsa-miR-1537 |
| 2.615145 | hsa-miR-590-5p |
| 2.558528 | hsa-miR-33a |
| 2.547821 | hsa-let-7e |
| 2.480264 | hsa-miR-32 |
| 2.330219 | hsa-miR-301a |
| 2.27536 | hsa-miR-30e |
| 2.22848 | hsa-miR-19a |
| 2.151041 | hsa-miR-142-5p |
| 2.144081 | hsa-miR-362-3p |
| 2.10237 | hsa-miR-301b |
| 2.052236 | hsa-miR-1183 |
| 2.051132 | hsa-miR-142-3p |
| 2.027244 | hsa-miR-340 |
| 1.977995 | hsa-miR-371-5p |
| -1.98464 | hsa-miR-154 |
| -2.11305 | hsa-miR-423-3p |
| -2.1648 | hsa-miR-1224-5p |
| -2.19 | hsa-miR-191 |
| -2.19444 | hsa-miR-127-3p |
| -2.24513 | hsa-miR-574-5p |
| -2.4263 | hsa-miR-139-3p |
| -2.7148 | hsa-miR-432 |

**Table 9**

| Ratio from calculation | microRNA |
|---|---|
| 5.120617 | hsa-miR-548d-5p |
| 4.969583 | hsa-miR-548a-5p |
| 3.438886 | hsa-miR-1537 |
| 2.615145 | hsa-miR-590-5p |
| 2.558528 | hsa-miR-33a |
| 2.547821 | hsa-let-7e |
| 2.480264 | hsa-miR-32 |
| 2.330219 | hsa-miR-301a |
| 2.27536 | hsa-miR-30e |
| 2.22848 | hsa-miR-19a |
| 2.151041 | hsa-miR-142-5p |
| 2.144081 | hsa-miR-362-3p |
| 2.10237 | hsa-miR-301b |
| 2.052236 | hsa-miR-1183 |
| 2.051132 | hsa-miR-142-3p |
| 2.027244 | hsa-miR-340 |
| 1.977995 | hsa-miR-371-5p |
| 1.861578 | hsa-miR-15a |
| 1.857642 | hsa-miR-548c-5p |
| 1.850123 | hsa-miR-1225-3p |
| 1.837303 | hsa-miR-29b |
| 1.834596 | hsa-miR-21 |
| 1.820351 | hsa-miR-1237 |
| 1.813523 | hsa-miR-101 |
| 1.802173 | hsa-miR-1539 |
| -1.38055 | hsa-miR-602 |
| -1.38147 | hsa-miR-132 |
| -1.44539 | hsa-miR-1471 |
| -1.45911 | hsa-miR-495 |
| -1.59946 | hsa-miR-1181 |
| -1.60944 | hsa-miR-339-5p |
| -1.62878 | hsa-miR-134 |
| -1.6329 | hsa-miR-183 |
| -1.72047 | hsa-miR-557 |
| -1.81989 | hsa-miR-125a-3p |
| -1.83853 | hsa-miR-423-5p |
| -1.94998 | hsa-miR-382 |
| -1.98464 | hsa-miR-154 |
| -2.11305 | hsa-miR-423-3p |
| -2.1648 | hsa-miR-1224-5p |
| -2.19 | hsa-miR-191 |
| -2.19444 | hsa-miR-127-3p |
| -2.24513 | hsa-miR-574-5p |
| -2.4263 | hsa-miR-139-3p |
| -2.7148 | hsa-miR-432 |

Correspondingly, Tables 10 and 11 comprise a selective list and a more comprehensive list of such microRNAs, respectively, representing significant ratios in patients after IVIG treatment.

**Table 10**

| Ratio from calculation | microRNA |
|---|---|
| 2.883848 | hsa-miR-125a-Sp |
| 2.589854 | hsa-miR-92b |
| 2.108284 | hsa-let-7e |
| 2.083486 | hsa-miR-1307 |
| -2.13615 | hsa-miR-376b |
| -2.25541 | hsa-miR-29b |
| -2.28617 | hsa-miR-543 |
| -2.29295 | hsa-miR-301a |
| -2.35023 | hsa-miR-15a |
| -2.37193 | hsa-miR-1249 |
| -2.40239 | hsa-miR-542-3p |
| -2.43732 | hsa-miR-136 |
| -2.44794 | hsa-miR-140-5p |
| -2.5479 | hsa-miR-32 |
| -2.56767 | hsa-miR-33a |
| -2.58454 | hsa-miR-545 |
| -2.75021 | hsa-miR-340 |
| -2.7781 | hsa-miR-590-5p |
| -2.80882 | kshv-miR-K12-10b |
| -2.82348 | hsa-miR-142-5p |
| -3.0019 | hsa-miR-923_v12.0 |
| -3.02147 | hsa-miR-101 |
| -3.20361 | hsa-miR-19a |
| -3.88505 | hsa-miR-141 |
| -4.08184 | hsa-miR-548c-5p |
| -4.53511 | hsa-miR-30e |
| -5.1227 | hsa-miR-153 |

**Table 11**

| Ratio from calculation | microRNA |
|---|---|
| 2.883848 | hsa-miR-125a-5p |
| 2.589854 | hsa-miR-92b |
| 2.130259 | miRNABrightCorner30 |
| 2.108284 | hsa-let-7e |
| 2.083486 | hsa-miR-1307 |
| 2.073077 | hsa-miR-886-3p_v15.0 |
| 1.956005 | hsa-miR-222 |
| 1.860369 | mr_1 |
| 1.798634 | hsa-miR-338-5p |
| 1.793474 | hsa-miR-664 |
| 1.781682 | hsa-miR-1227 |
| 1.717443 | hsa-miR-99b |
| 1.676623 | hsa-miR-363 |
| -1.6569 | hsa-miR-377 |
| -1.68107 | hsa-miR-450a |
| -1.72193 | hsa-miR-376c |
| -1.76059 | hsa-miR-382 |
| -1.78329 | hsa-miR-1537 |
| -1.82416 | hsa-miR-29c |
| -1.8549 | hsa-miR-144 |
| -1.85679 | hsa-miR-19b |
| -1.88001 | hsa-miR-106b |
| -1.88841 | hsa-miR-499-5p |
| -1.90758 | hsa-miR-376a |
| -1.96386 | hsa-miR-362-3p |
| -1.99976 | hsa-miR-154 |
| -2.035 | hsa-miR-337-5p |
| -2.04325 | hsa-miR-424 |
| -2.06191 | hsa-miR-219-5p |
| -2.13615 | hsa-miR-376b |
| -2.25541 | hsa-miR-29b |
| -2.28617 | hsa-miR-543 |
| -2.29295 | hsa-miR-301a |
| -2.35023 | hsa-miR-15a |
| -2.37193 | hsa-miR-1249 |
| -2.40239 | hsa-miR-542-3p |
| -2.43732 | hsa-miR-136 |
| -2.44794 | hsa-miR-140-5p |
| -2.5479 | hsa-miR-32 |
| -2.56767 | hsa-miR-33a |
| -2.58454 | hsa-miR-545 |
| -2.75021 | hsa-miR-340 |
| -2.7781 | hsa-miR-590-5p |
| -2.80882 | kshv-miR-K12-10b |
| -2.82348 | hsa-miR-142-5p |
| -3.0019 | hsa-miR-923 v12.0 |
| -3.02147 | hsa-miR-101 |
| -3.20361 | hsa-miR-19a |
| -3.88505 | hsa-miR-141 |
| -4.08184 | hsa-miR-548c-5p |
| -4.53511 | hsa-miR-30e |
| -5.1227 | hsa-miR-153 |

To assess to ability of microRNAs to predict microRNA response to therapy, microRNAs that function similarly upon treatment between the dichotomized groups, microRNAs that either increase together or decrease together were collected and listed in Table 12.

**Table 12**

| MicroRNA | | Mean Δ Group A | SD | Mean Δ Group B | SD | | Net Δ A and B |
|---|---|---|---|---|---|---|---|
| hsa-mir-1470 | dn | -0.7673864 | 1.235473 | -4.12664194 | 1.5502503 | dn | -3.35926 |
| hsa-mir-1290 | dn | -0.0147208 | 1.1018548 | -3.15440146 3 | 1.1577212 | dn | -3.13968 |
| hsa-mir-1202 | dn | -0.3401371 | 0.8489831 | -3.3743004 | 1.1013233 | dn | -3.03416 |
| hsa-mir-212 | dn | -0.694552 | 0.7209563 | -3.7040352 | 0.89993 | dn | -3.00948 |
| hsa-mir-26b | dn | -0.0189347 | 0.3277201 | -1.486439 | 0.2695336 | dn | -1.4675 |
| hsa-mir-623 | up | 0.5154214 | 0.3045152 | 3.2896302 | 1.1049068 | up | 2.774209 |
| hsa-mir-1826_v15.0 | up | 0.0226423 | 0.5811046 | 2.88962271 | 1.1343319 | up | 2.86698 |
| hsa-mir-574-3p | up | 0.2439299 | 1.5461713 | 3.300805 | 0.7679533 | up | 3.056875 |
| hsa-mir-1471 | up | 0.6108116 | 2.9157016 | 4.0440545 | 1.3350063 | up | 3.433243 |
| hsa-mir-337-3p | up | 0.2684066 | 1.4140522 | 4.8525722 | 0.9392156 | up | 4.584166 |
| hsa-mir-513b | up | 0.6818242 | 1.3846447 | 5.8524707 | 1.7107123 | up | 5.170647 |

Certain patterns of response may indicate resistance or responsiveness of one the two groups. While there may be a significant change in value of one or more microRNAs in one group there may be little response in the other. The group exhibiting lesser change may indicate a lack of response to the therapeutic intervention. This group may be resistant to the intervention or that the intended therapeutic effect is not needed. The response of hsa-mir-132 is exemplary. Group A patients had relatively low CTs by PCR (relatively high concentrations of the microRNA) while group B patients had relatively high CTs. A brisk response was noted following therapy in group A patients substantially converging on the low levels noted in group B patients. The possibility that the therapeutic effect on the levels of the microRNA in group B were already at a level that the therapeutic intervention was capable of effecting. The following tables demonstrate subsets of microRNAs exhibiting relatively significant change in one group and reduced change in the other group. Further, it can be seen that the group exhibiting a significant change may either converge to a level of expression corresponding to the other group or diverge. Specifically, Tables 13 and 14 are selective lists of microRNAs having divergent and convergent behavior, respectively, which exhibit significant change in Group A but relatively little change in Group B.

**Table 13**

| Mean Δ Group A | SD | SystematicName | Direction |
|---|---|---|---|
| 2.52122 | 1.497334 | hsa-miR-136 | Most increased |
| 2.208455 | 0.541141 | hsa-miR-33a | Most increased |
| 2.083515 | 1.609553 | hsa-miR-219-5p | Most increased |
| 2.082454 | 0.849342 | hsa-miR-153 | Most increased |
| 1.955594 | 1.558846 | hsa-miR-545 | Most increased |
| 1.757013 | 0.663302 | hsa-miR-32 | Most increased |
| 1.301357 | 0.995047 | hsa-miR-1537 | Most increased |
| 1.195371 | 0.219509 | hsa-miR-590-5p | Most increased |
| -1.82126 | 1.984385 | hsa-miR-1181 | Most decreased |

**Table 14**

| Mean Δ Group A | SD | SystematicName | Direction |
|---|---|---|---|
| 1.270745 | 1.003145 | hsa-miR-1 | Most increased |
| 0.933754 | 1.321562 | hsa-miR-376a | Most increased |
| -1.20769 | 3.331194 | hsa-miR-630 | Most decreased |
| -1.26517 | 0.328726 | hsa-miR-886-3p_v15.0 | Most decreased |
| -1.27118 | 1.97 | hsa-miR-1300_v13.0 | Most decreased |
| -1.29486 | 3.144593 | hsa-miR-485-3p | Most decreased |
| -1.32161 | 1.728257 | hsa-miR-1224-5p | Most decreased |
| -1.48121 | 1.881716 | hsa-miR-300 | Most decreased |
| -1.50279 | 0.935894 | hsa-miR-132 | Most decreased |
| -1.7283 | 1.326469 | hsa-miR-339-5p | Most decreased |
| -1.74399 | 3.594059 | kshv-miR-K12-9 | Most decreased |

Likewise, Tables 15 and 16 are more comprehensive lists of microRNAs having divergent and convergent behavior, respectively, which exhibit significant change in Group A but relatively little change in Group B.

**Table 15**

| Mean Δ Group A | SD | SystematicName | Direction |
|---|---|---|---|
| 2.52122 | 1.497334 | hsa-miR-136 | Most increased |
| 2.208455 | 0.541141 | hsa-miR-33a | Most increased |
| 2.083515 | 1.609553 | hsa-miR-219-5p | Most increased |
| 2.082454 | 0.849342 | hsa-miR-153 | Most increased |
| 1.955594 | 1.558846 | hsa-miR-545 | Most increased |
| 1.757013 | 0.663302 | hsa-miR-32 | Most increased |
| 1.301357 | 0.995047 | hsa-miR-1537 | Most increased |
| 1.195371 | 0.219509 | hsa-miR-590-5p | Most increased |
| 1.140525 | 0.150147 | hsa-miR-301a | Most increased |
| 1.083703 | 0.288785 | hsa-miR-141 | Most increased |
| 1.068173 | 0.318097 | hsa-miR-29b | Most increased |
| 1.041446 | 0.142776 | hsa-miR-142-5p | Most increased |
| 0.952666 | 0.441789 | hsa-miR-193a-3p | Most increased |
| 0.885343 | 0.493095 | hsa-miR-582-3p | Most increased |
| -0.90253 | 1.698756 | hsa-miR-513a-Sp | Most decreased |
| -1.82126 | 1.984385 | hsa-miR-1181 | Most decreased |

**Table 16**

| Mean Δ Group A | SD | SystematicName | Direction |
|---|---|---|---|
| 1.270745 | 1.003145 | hsa-miR-1 | Most increased |
| 1.13486 | 1.316732 | hsa-miR-376c | Most increased |
| 1.132591 | 0.828951 | hsa-miR-551b | Most increased |
| 1.10453 | 1.617308 | hsa-miR-337-5p | Most increased |
| 1.0677 | 0.202879 | hsa-miR-142-3p | Most increased |
| 1.049269 | 1.235692 | hsv1-miR-H1_v14.0 | Most increased |
| 1.035347 | 2.190203 | hsa-miR-410 | Most increased |
| 0.976275 | 0.357315 | hsa-miR-301b | Most increased |
| 0.971021 | 1.083568 | hsa-miR-371-5p | Most increased |
| 0.933754 | 1.321562 | hsa-miR-376a | Most increased |
| 0.88329 | 0.745268 | hsa-miR-133b | Most increased |
| -0.75803 | 1.061571 | hsa-miR-100 | Most decreased |
| -0.76739 | 1.235473 | hsa-miR-1470 | Most decreased |
| -0.80433 | 0.948616 | hsa-miR-1260 | Most decreased |
| -0.84299 | 1.117714 | hsa-miR-647 | Most decreased |
| -0.8683 | 2.617779 | hsa-miR-595 | Most decreased |
| -0.87714 | 1.87364 | hsa-miR-525-5p | Most decreased |
| -0.88588 | 2.114378 | hsa-miR-188-5p | Most decreased |
| -0.94706 | 0.701991 | hsa-miR-28-3p | Most decreased |
| -0.99436 | 2.03129 | hsa-miR-96 | Most decreased |
| -1.02709 | 1.633777 | hsa-miR-451 | Most decreased |
| -1.07284 | 0.683653 | hsa-miR-134 | Most decreased |
| -1.07828 | 1.295253 | hsa-miR-127-3p | Most decreased |
| -1.08028 | 2.11365 | hsa-miR-663 | Most decreased |
| -1.20003 | 1.525774 | hsa-miR-486-5p | Most decreased |
| -1.20769 | 3.331194 | hsa-miR-630 | Most decreased |
| -1.26517 | 0.328726 | hsa-miR-886-3p_v15.0 | Most decreased |
| -1.27118 | 1.97 | hsa-miR-1300_v13.0 | Most decreased |
| -1.29486 | 3.144593 | hsa-miR-485-3p | Most decreased |
| -1.32161 | 1.728257 | hsa-miR-1224-5p | Most decreased |
| -1.48121 | 1.881716 | hsa-miR-300 | Most decreased |
| -1.50279 | 0.935894 | hsa-miR-132 | Most decreased |
| -1.7283 | 1.326469 | hsa-miR-339-5p | Most decreased |
| -1.74399 | 3.594059 | kshv-miR-K12-9 | Most decreased |

Correspondingly, Tables 17 and 18 are a selective list of microRNAs having divergent and convergent behavior, respectively, which exhibit significant change in Group B and relatively little change in Group A.

**Table 17**

| Mean Δ Group B | SD | SystematicName | Direction |
|---|---|---|---|
| 8.720988 | 1.695736 | hsa-miR-513a-5p | Most increased |
| 4.492508 | 1.085892 | hsa-miR-1181 | Most increased |
| -5.88934 | 1.274726 | hsa-miR-144 | Most decreased |
| -5.94462 | 0.44929 | hsa-miR-142-5p | Most decreased |
| -6.60811 | 0.775835 | hsa-miR-32 | Most decreased |
| -6.60915 | 0.991381 | hsa-miR-219-5p | Most decreased |
| -6.66695 | 0.528549 | hsa-miR-33a | Most decreased |
| -6.95672 | 0.388828 | hsa-miR-545 | Most decreased |
| -7.47155 | 1.143166 | hsa-miR-1537 | Most decreased |
| -7.84218 | 0.691729 | hsa-miR-153 | Most decreased |

**Table 18**

| Mean Δ Group B | SD | SystematicName | Direction |
|---|---|---|---|
| 6.045644 | 1.810638 | hsa-miR-1296 | Most increased |
| 5.852471 | 1.710712 | hsa-miR-513b | Most increased |
| 5.269371 | 0.913262 | hsa-miR-1307 | Most increased |
| 5.23262 | 0.53149 | hsa-miR-92b | Most increased |
| 4.852572 | 0.939216 | hsa-miR-337-3p | Most increased |
| 4.483333 | 1.436393 | hsa-miR-647 | Most increased |
| 4.446777 | 2.354756 | hsa-miR-345 | Most increased |
| 4.393115 | 0.758058 | hsa-miR-501-5p | Most increased |
| -6.23567 | 1.991346 | hsa-miR-376b | Most decreased |
| -7.18912 | 2.240812 | hsa-miR-1183 | Most decreased |

Finally, Tables 19 and 20 are more comprehensive lists of microRNAs having divergent and convergent behavior, respectively, which exhibit significant change in Group B but relatively little change in Group A

**Table 19**

| Mean Δ Group B | SD | SystematicName | Direction |
|---|---|---|---|
| 8.720988 | 1.695736 | hsa-miR-513a-5p | Most increased |
| 4.492508 | 1.085892 | hsa-miR-1181 | Most increased |
| -4.08226 | 0.877131 | hsa-miR-193a-3p | Most decreased |
| -4.74399 | 0.497264 | hsa-miR-301a | Most decreased |
| -4.77102 | 0.469596 | hsa-miR-141 | Most decreased |
| -5.26843 | 0.506796 | hsa-miR-29b | Most decreased |
| -5.34069 | 0.410219 | hsa-miR-590-5p | Most decreased |
| -5.43651 | 0.82098 | hsa-miR-136 | Most decreased |
| -5.63284 | 1.148245 | hsa-miR-582-3p | Most decreased |
| -5.88934 | 1.274726 | hsa-miR-144 | Most decreased |
| -5.94462 | 0.44929 | hsa-miR-142-5p | Most decreased |
| -6.60811 | 0.775835 | hsa-miR-32 | Most decreased |
| -6.60915 | 0.991381 | hsa-miR-219-5p | Most decreased |
| -6.66695 | 0.528549 | hsa-miR-33a | Most decreased |
| -6.95672 | 0.388828 | hsa-miR-545 | Most decreased |
| -7.47155 | 1.143166 | hsa-miR-1537 | Most decreased |
| -7.84218 | 0.691729 | hsa-miR-153 | Most decreased |

**Table 20**

| Mean Δ Group B | SD | SystematicName | Direction |
|---|---|---|---|
| 6.045644 | 1.810638 | hsa-miR-1296 | Most increased |
| 5.852471 | 1.710712 | hsa-miR-513b | Most increased |
| 5.269371 | 0.913262 | hsa-miR-1307 | Most increased |
| 5.23262 | 0.53149 | hsa-miR-92b | Most increased |
| 4.852572 | 0.939216 | hsa-miR-337-3p | Most increased |
| 4.483333 | 1.436393 | hsa-miR-647 | Most increased |
| 4.446777 | 2.354756 | hsa-miR-345 | Most increased |
| 4.393115 | 0.758058 | hsa-miR-501-Sp | Most increased |
| 4.139791 | 1.284721 | hsa-miR-765 | Most increased |
| 4.10815 | 2.241581 | hsa-miR-299-5p | Most increased |
| 4.044055 | 1.335006 | hsa-miR-1471 | Most increased |
| 3.998238 | 0.721076 | hsa-miR-182 | Most increased |
| 3.887691 | 0.570974 | hsa-miR-1227 | Most increased |
| 3.841755 | 0.995814 | hsa-miR-34b | Most increased |
| 3.622937 | 1.26476 | hsa-miR-491-3p | Most increased |
| 3.506353 | 0.577494 | hsa-miR-191 | Most increased |
| 3.399269 | 1.692025 | hsa-miR-513c | Most increased |
| 3.300805 | 0.767953 | hsa-miR-574-3p | Most increased |
| 3.28963 | 1.104907 | hsa-miR-623 | Most increased |
| 3.238278 | 1.381079 | hsa-miR-885-5p | Most increased |
| 3.047651 | 0.813464 | hsa-miR-338-5p | Most increased |
| 2.961104 | 1.572459 | hsa-miR-609 | Most increased |
| 2.889623 | 1.134332 | hsa-miR-1826 v15.0 | Most increased |
| -4.09785 | 0.929085 | kshv-miR-K12-10b | Most decreased |
| -4.10335 | 0.423924 | hsa-miR-101 | Most decreased |
| -4.12664 | 1.55025 | hsa-miR-1470 | Most decreased |
| -4.14723 | 2.179625 | hsa-miR-337-5p | Most decreased |
| -4.16497 | 0.300849 | hsa-miR-340 | Most decreased |
| -4.3393 | 0.511408 | hsa-miR-19a | Most decreased |
| -4.3514 | 0.995718 | hsa-miR-487a | Most decreased |
| -5.38285 | 0.608003 | hsa-miR-542-3p | Most decreased |
| -6.23567 | 1.991346 | hsa-miR-376b | Most decreased |
| -7.18912 | 2.240812 | hsa-miR-1183 | Most decreased |

The tables reproduced above represent a number of suitable techniques for classifying microRNAs that exhibit dichotomous behavior in the groups of patients. In turn, it can be expected that such microRNAs may be used to segregate patient populations and to assign individual patients to appropriate groups for the purpose of diagnosis, treatment or the like. Further, one of skill in the art will recognize that certain microRNAs stand out as being identified in a number of the tables. Accordingly, the techniques of this disclosure can be practiced using one or more microRNAs selected from the following group:
hsa-let-7e, hsa-mir-1181, hsa-miR-1183, hsa-miR-1224-Sp, hsa-miR-127-3p, hsa-mir-1296, hsa-mir-132, hsa-mir-136, hsa-miR-139-3p, hsa-mir-141, hsa-miR-142-3p, hsa-mir-142-5p, hsa-mir-144, hsa-mir-153, hsa-mir-1537, hsa-miR-154, hsa-miR-191, hsa-mir-193a-3p, hsa-miR-19a, hsa-mir-219-5p, hsa-mir-29b, hsa-mir-301a, hsa-miR-301b, hsa-miR-30e, hsa-mir-32, hsa-mir-33a, hsa-miR-340, hsa-miR-362-3p, hsa-miR-371-5p, hsa-377, hsa-miR-423-3p, hsa-miR-432, hsa-mir-513a-5p, hsa-mir-545, hsa-miR-548a-5p, hsa-miR-574-5p, hsa-mir-582-3p, hsa-mir-590-5p, hsa-mir-15a, hsa-mir-548c-5p, hsa-mir-1225-3p, hsa-mir-29b, hsa-mir-21, hsa-mir-1237, hsa-mir-101, hsa-mir-1539, hsa-mir-557, hsa-mir-125a-3p and hsa-mir-423-5p. In another aspect, Table 21 provides a list of 19 selected microRNAs having been selected on the basis of one or more of the indicated criteria that may be used in the techniques of this disclosure.

**Table 21**

| | Top 17 bidirectional (A&B) movers | Unidirectional mover (A or B) | Before IVIG AorB predictor | After IVIG AorB predictor | Known AB divergent/ convergent behavior |
|---|---|---|---|---|---|
| hsa-mir-136 | x | x | | x | x |
| hsa-mir-141 | x | | | x | |
| hsa-mir-142-5p | x | | x | x | |
| hsa-mir-144 | x | | | | |
| hsa-mir-153 | x | x | | | |
| hsa-mir-1537 | x | x | x | | |
| hsa-mir-193a-3p | x | | | | |
| hsa-mir-219-5p | x | | | | |
| hsa-mir-29b | x | | | x | |
| hsa-mir-301a | x | | x | | |
| hsa-mir-32 | x | | x | x | |
| hsa-mir-33a | x | x | x | x | |
| hsa-mir-545 | x | | | | |
| hsa-mir-582-3p | x | | | | |
| hsa-mir-590-5p | x | | x | x | |
| hsa-mir-1181 | x | x | | | |
| hsa-mir-513a-5p | x | x | | | |
| hsa-mir-132 | | | | x | x |
| hsa-mir-1296 | | x | | | |

As stated previously, Tables 5 and 6 were constructed by listing the most divergent microRNAs (greatest differences between pre- and post-therapy "delta") in groups A and B. In Table 5, the A group were those microRNAs selected from the 100 most positive deltas and B microRNAs the negative 100 deltas with converse in Table 6. In Table 22 these microRNAs are listed wherein column III lists microRNAs from group A and column IV lists microRNAs from group B. The microRNAs are individually marked with a single X ("X") where they are derived from the top and bottom most 100 microRNAs from Tables 5 and 6 and with a double X ("XX") where they are derived from a shorter list comprising the top and bottom-most 25 microRNAs.

**TABLE 22**

| | | Group A most increased | Group A most decreased |
|---|---|---|---|
| | | Group B most decreased | Group B most increased |
| | | XX | XX |
| | MicroRNA | from top 25s | from top 25s |
| | | X | X |
| | | from top 100s | from top 100s |
| I | II | III | IV |
| 1. | hsa-miR-136 | xx | |
| 2. | hsa-miR-141 | xx | |
| 3. | hsa-miR-142-5p | xx | |
| 4. | hsa-miR-144 | xx | |
| 5. | hsa-miR-153 | xx | |
| 6. | hsa-miR-1537 | xx | |
| 7. | hsa-miR-193a-3p | xx | |
| 8. | hsa-miR-219-5p | xx | |
| 9. | hsa-miR-29b | xx | |
| 10. | hsa-miR-301a | xx | |
| 11. | hsa-miR-32 | xx | |
| 12. | hsa-miR-33a | xx | |
| 13. | hsa-miR-545 | xx | |
| 14. | hsa-miR-582-3p | xx | |
| 15. | hsa-miR-590-5p | xx | |
| 16. | hsa-miR-1181 | | xx |
| 17. | hsa-miR-1 | x | |
| 18. | hsa-miR-101 | x | |
| 19. | hsa-miR-1183 | x | |
| 20. | hsa-miR-142-5p | x | |
| 21. | hsa-miR-15a | x | |
| 22. | hsa-miR-19a | x | |
| 23. | hsa-miR-301b | x | |
| 24. | hsa-miR-30e | x | |
| 25. | hsa-miR-340 | x | |
| 26. | hsa-miR-362-3p | x | |
| 27. | hsa-miR-371-5p | x | |
| 28. | hsa-miR-424 | x | |
| 29. | hsa-miR-548a-5p | x | |
| 30. | hsa-miR-1249 | x | |
| 31. | hsa-miR-140-5p | x | |
| 32. | hsa-miR-18a | x | |
| 33. | hsa-miR-19b | x | |
| 34. | hsa-miR-21 | x | |
| 35. | hsa-miR-324-5p | x | |
| 36. | hsa-miR-335 | x | |
| 37. | hsa-miR-337-5p | x | |
| 38. | hsa-miR-374a | x | |
| 39. | hsa-miR-376b | x | |
| 40. | hsa-miR-377 | x | |
| 41. | hsa-miR-421 | x | |
| 42. | hsa-miR-542-3p | x | |
| 43. | hsa-miR-548c-5p | x | |
| 44. | hsa-miR-551b | x | |
| 45. | hsa-miR-224 | | x |
| 46. | hsa-miR-1224-5p | | x |
| 47. | hsa-miR-191 | | x |
| 48. | hsa-miR-423-5p | | x |
| 49. | hsa-miR-513a-5p | | x |
| 50. | hsa-miR-630 | | x |
| 51. | hsa-miR-92b | | x |
| 52. | hsa-miR-100 | | x |
| 53. | hsa-miR-1227 | | x |
| 54. | hsa-miR-1271 | | x |
| 55. | hsa-miR-1275 | | x |
| 56. | hsa-miR-1307 | | x |
| 57. | hsa-miR-182 | | x |
| 58. | hsa-miR-18b | | x |
| 59. | hsa-miR-300 | | x |
| 60. | hsa-miR-338-3p | | x |
| 61. | hsa-miR-501-Sp | | x |
| 62. | hsa-miR-513b | | x |
| 63. | hsa-miR-513c | | x |
| 64. | hsa-miR-564 | | x |
| 65. | hsa-miR-647 | | x |
| 66. | hsa-miR-760 | | x |
| 67. | hsa-miR-770-5p | | x |
| 68. | hsa-miR-885-5p | | x |
| 69. | hsa-miR-892b | | x |

Twelve patients with clinical outcomes were examined in the same manner (microarray of known human microRNAs). Six patients had normal outcomes while three each had miscarriage or preeclampsia respectively drown in the first trimester prior to development of pathology. Two microRNA samples were drawn for each patient, one sample drawn a week before IVIG and the second sample drawn a week after IVIG. Table 23 lists microRNAs by clinical outcome. The letter "H" designates a pregnancy without diagnosis of miscarriage or preeclampsia while the letter "P" designates pregnancies with a diagnosis of preeclampsia and the letter "M" designates pregnancies with a diagnosis of miscarriage. Double X ("XX") designates microRNAs from the 25 most divergent between patient groups and Single X ("X") designates microRNAs from the 100 most divergent microRNAs between the two patient groups. Hereinafter a down arrow shall mean that the first value minus a second value is negative and an up arrow shall mean that the first value minus a second value is positive.

**Table 23**

| | (XX) | (XX) | (XX) | Reference from Table 22: miR Group A - B behavior category in response to IVIG |
|---|---|---|---|---|
| | H↓P↑ from top 25s | H↑ M↓from top 25s | H↓ M↑from top 25 s | |
| *Key:* | | | | |
| ↑*Most increased* | | | | |
| ↓*,Most decreased* | (X) | (X) | (X) | |
| *H=Healthy* | H↓P↑ from top | H↑ M↓from top | H↓ M↑ from top | |
| *P=Preeclampsia* | 100s | 100s | 100s | (XX) |
| *M=Miscarriage* | | | | from top 25s |
| | | | | |
| MicroRNAs | | | | (X) from top 100s |
| A | B | C | D | E |
| hsa-miR-144-3p | xx | | x | XX |
| hsa-miR-199a-5p | xx | | x | |
| hsa-miR-199b-5p | xx | | | |
| hsa-miR-210 | xx | | | |
| hsa-miR-221-5p | xx | | | |
| hsa-miR-30e-3p | xx | | | x |
| hsa-miR-33a-5p | xx | | | |
| hsa-miR-340-5p | xx | | | x |
| hsa-miR-424-5p | xx | | | x |
| hsa-miR-575 | xx | | | |
| hsa-miR-582-5p | xx | | | XX |
| hsa-miR-7-Sp | xx | | | |
| hsa-miR-1229 | | xx | | |
| hsa-miR-1267 | | xx | | |
| hsa-miR-671-3p | | xx | | |
| hsa-miR-144-5p | | | xx | XX |
| hsa-miR-425-5p | x | | | |
| hsa-miR-125b-5p | x | | | |
| hsa-miR-136-5p | x | | x | XX |
| hsa-miR-148b-3p | x | | | |
| hsa-miR-152 | x | | | |
| hsa-miR-181c-5p | x | | x | |
| hsa-miR-193a-3p | x | | | XX |
| hsa-miR-223-5p | x | | | |
| hsa-miR-301a-3p | x | | | XX |
| hsa-miR-32-5p | x | | | XX |
| hsa-miR-338-3p | x | | | X |
| hsa-miR-34a-5p | x | | | |
| hsa-miR-362-3p | x | | | |
| hsa-miR-376a-3p | x | | | |
| hsa-miR-377-3p | x | | | X |
| hsa-miR-548am-5p | x | | | X |
| hsa-miR-584-5p | x | | | |
| hsa-miR-95 | x | | | |
| hsa-let-7b-3p | | x | | |
| hsa-let-7f-1-3p | | x | | |
| hsa-miR-1225-3p | | x | | |
| hsa-miR-1234 | | x | | |
| hsa-miR-1237 | | x | | |
| hsa-miR-1238 | | x | | |
| hsa-miR-1244 | | x | | |
| hsa-miR-1274b_v16.0 | | x | | |
| hsa-miR-129-1-3p | | x | | |
| hsa-miR-129-2-3p | | x | | |
| hsa-miR-1825 | | x | | |
| hsa-miR-191-3p | | x | | |
| hsa-miR-29a-5p | | x | | |
| hsa-miR-29b-1-5p | | x | | XX |
| hsa-miR-33b-3p | | x | | |
| hsa-miR-340-5p | | x | | X |
| hsa-miR-362-5p | | x | | |
| hsa-miR-371a-Sp | | x | | X |
| hsa-miR-425-3p | | x | | |
| hsa-miR-557 | | x | | |
| hsa-miR-563 | | x | | |
| hsa-miR-602 | | x | | |
| hsa-miR-634 | | x | | |
| hsa-miR-767-3p | | x | | |
| hsa-miR-876-3p | | x | | |
| hsa-miR-92b-3p | | x | | |
| hsa-miR-933 | | x | | |
| hsa-miR-373-5p | | x | | |
| hsa-miR-125a-5p | | | x | |
| hsa-miR-22-5p | | | x | X |
| hsa-miR-335-5p | | | x | X |
| hsa-miR-423-5p | | | x | |
| hsa-miR-505-5p | | | x | |
| hsa-miR-543 | | | x | |
| hsa-miR-548m | | | x | |
| hsa-miR-99a-5p | | | x | |

In a preferred embodiment, microRNAs lists from Table 23 may be shortened to comprise only those microRNAs that demonstrate the most extreme divergent results between clinical groupings (designated "XX"). Only those microRNAs that are found in these more select groups are used. In addition, in a preferred embodiment, said select microRNAs are used together. While an individual microRNA demonstrating divergent behavior may provide trending and screening information about clinical responses of patient groups, select groups of divergent microRNAs provide more valuable and personalized information about individual patient cases. That may be useful, for example, in initial diagnosis, prognostication and monitoring of therapeutic interventions. Results of such testing may, of course, be used in concert with additional laboratory and clinical data. Lists may be shorted further to comprise 5, 10, 15 microRNAs for example. MicroRNAs from a single sample, preferably drawn prior to therapy and more preferably in contemplation of therapy, may be quantified by any technique known to those of ordinary skill in the art. In a preferred embodiment, a microarray comprising all known human microRNAs is used and the quantitative results arranged from highest to lowest. If a microRNA in the list displayed in Table 23 falls within the highest or lowest 100 of the list, then it receives a positive or negative score of "1". It is given a positive sign (+) wherein a high or low value corresponds to a high or low value in healthy pregnancies. It is given a negative sign (-) wherein a high or low value corresponds to a high or low value that in unhealthy pregnancies ("M", miscarriage, "P", preeclampsia). Table 24 is a shortened list from Table 23.

Other systems for quantification can be used that are well known in the art such as real-time PCR. Here standards are developed that provide threshold values for assigning positive and negative scores.

**Table 24**

| | (XX) | (XX) | (XX) | Reference from Table 22: |
|---|---|---|---|---|
| | H↓P↑ from top 25s | H↑ M↓from top 25s | H↓ M↑from top 25s | miR Group A -B response to IVIG category |
| | | (X) | | |
| Key: | (X) | H↑ M↓from top 100s | (X) | (XX) |
| ↑Most increased | H↓P↑ from top 100s | | H↓ M↑from top 100s | from top 25s |
| ↓Most decreased | | | | |
| H=Healthy | | | | (X) from top |
| P=Preeclampsia | | | | 100s |
| M=Miscarriage | | | | |
| A | **B** | **C** | **D** | **E** |
| hsa-miR-144 | xx | | xx | XX |
| hsa-miR-582-5p | xx | | | XX |
| hsa-miR-30e-3p | xx | | | x |
| hsa-miR-340-5p | xx | | | x |
| hsa-miR-424-5p | xx | | | x |
| hsa-miR-199a-5p | xx | | x | |
| hsa-miR-199b-5p | xx | | | |
| hsa-miR-210 | xx | | | |
| hsa-miR-221-5p | xx | | | |
| hsa-miR-33a-5p | xx | | | |
| hsa-miR-575 | xx | | | |
| hsa-miR-7-5p | xx | | | |
| hsa-miR-1229 | | xx | | |
| hsa-miR-1267 | | xx | | |
| hsa-miR-671-3p | | xx | | |

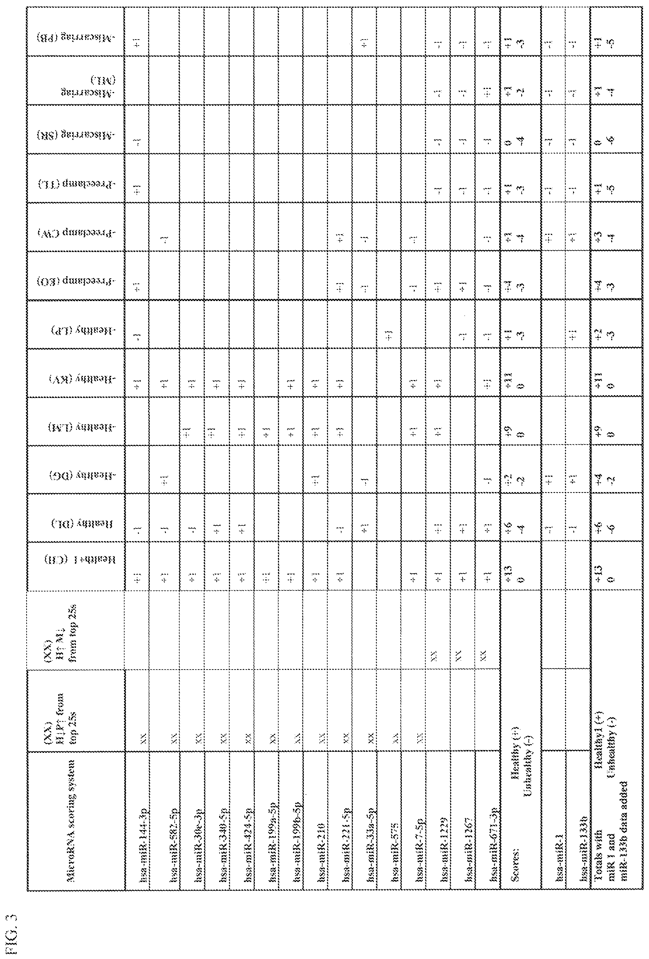

In more preferred embodiments, additional methods may be added to make a microRNA scoring system. In addition to the "divergent microRNA" selection system illustrated in Table 23, microRNAs may also be selected based on the relative strength of single signal measurements. In one embodiment, microRNAs from a group of patients with a similar condition are listed in order by decreasing signal strength. The 900+ MicroRNAs are listed from highest to lowest signal strengths. The top 25 highest and the bottom 25 lowest microRNAs are selected as potential markers for the condition of interest. In an additional embodiment, these top microRNA selections may be combined with other selection methods to form more powerful combined scoring systems. For example, MicroRNA- 1 and MicroRNA- 133b demonstrate the highest mean signal strengths of 900+ miRs seen in these miscarriage cases. In the scoring system illustrated in Fig. 3, MicroRNA- 1 and MicroRNA- 133b results have been added to the original 15 "divergent MicroRNA" totals to create a more predictive final score.

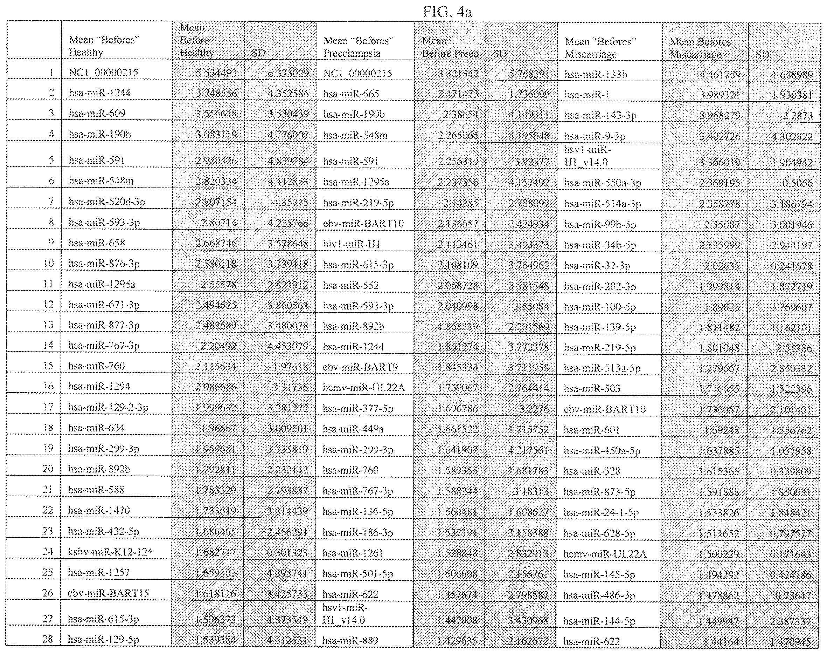

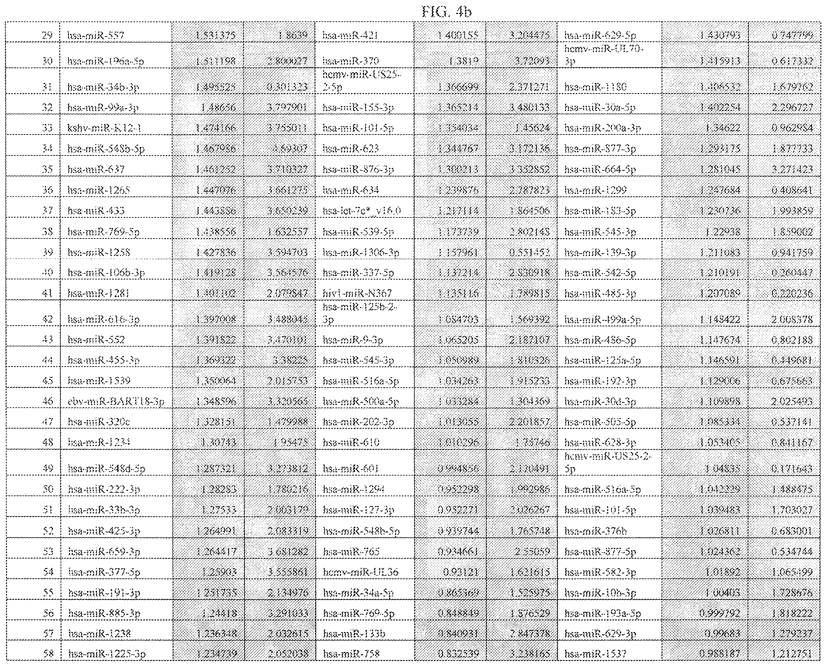

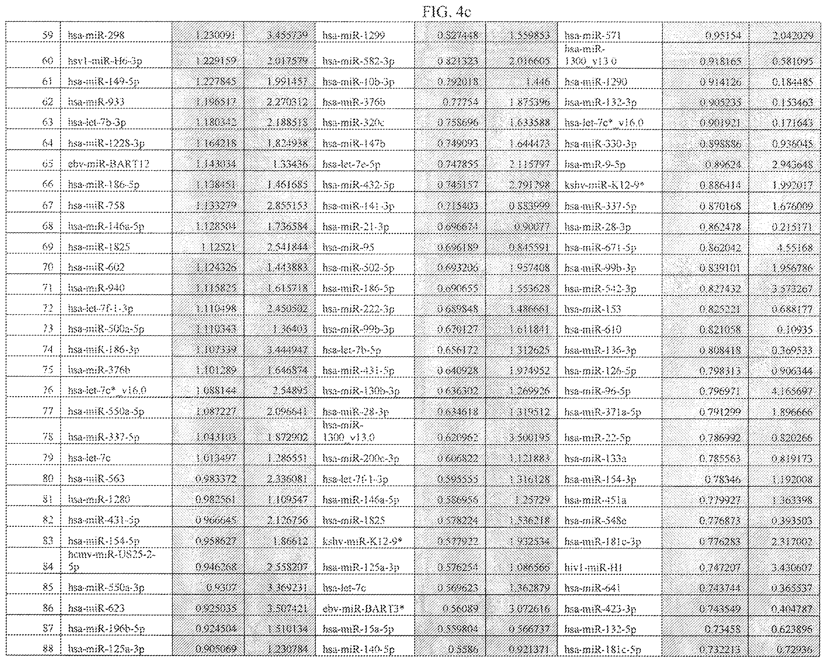

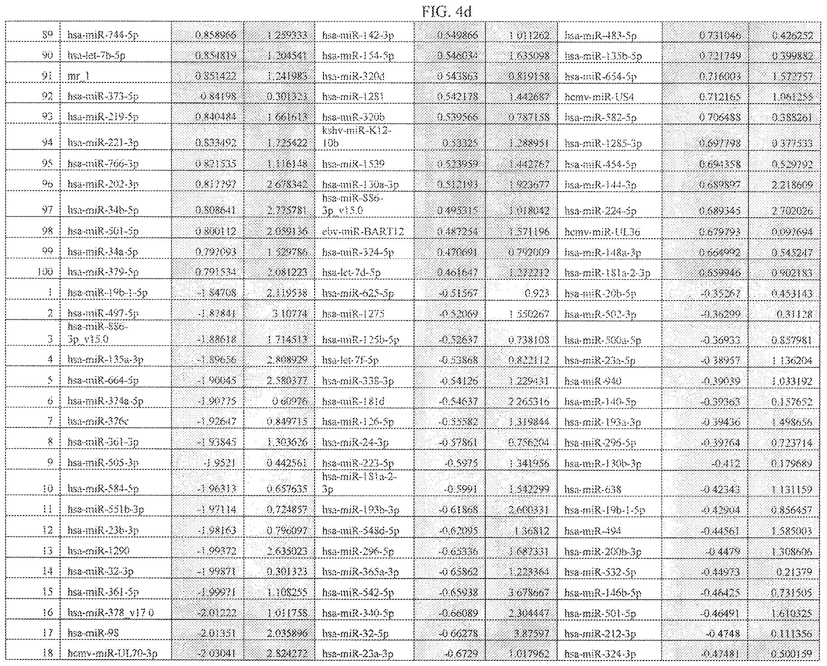

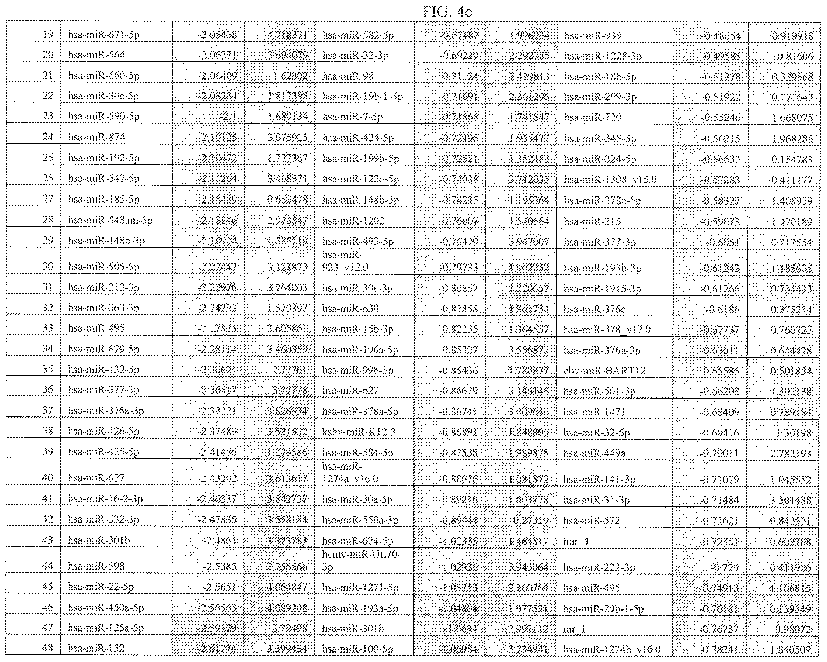

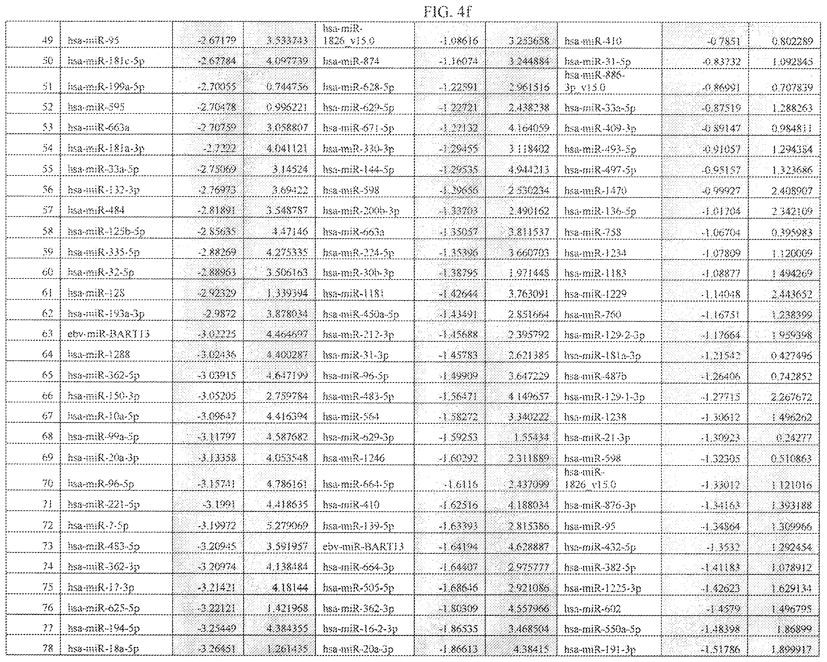

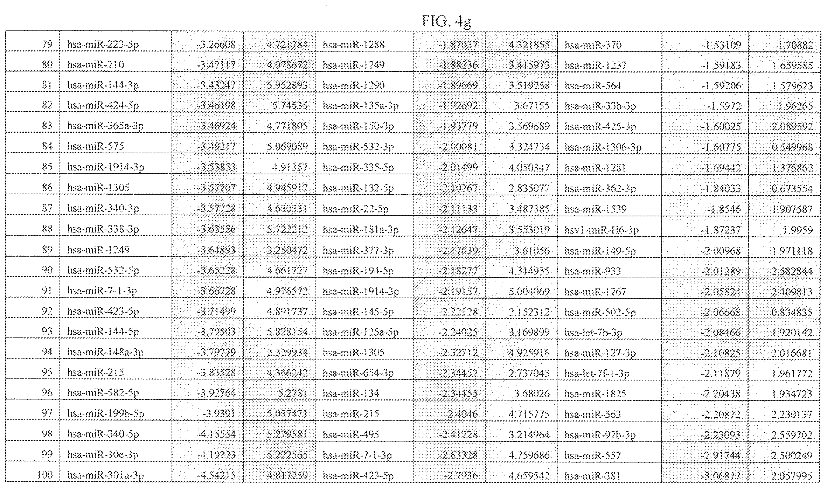

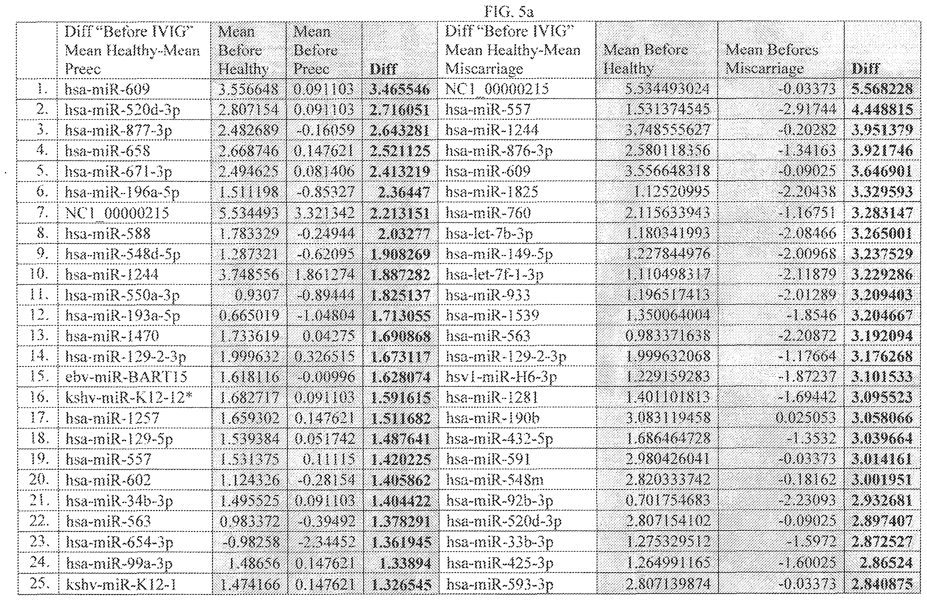

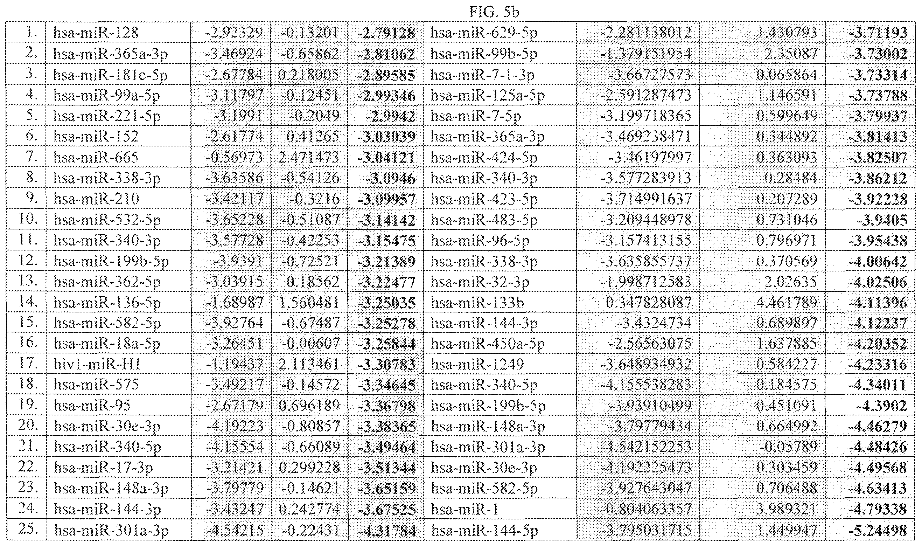

Detection and quantification of these microRNAs of this invention in immune cells, preferably in peripheral blood, is useful for a number of purposes. These include, for example, prediction of outcome, prognosis, determination of candidacy for therapeutic intervention, selection of therapeutic intervention, dosing, timing of intervention, determination of therapeutic efficacy, screening of patients for risk and for further investigation. In a preferred embodiment, microRNA testing is provided for assessment in pre-pregnant or pregnant women. As such, Fig. 3 indicates efficacy of individual microRNAs for the above listed uses. A single microRNA, for example, mir-1229 or mir-671-3p may be used singly for screening. Increased diagnostic power may be provided by detection and quantification of more than a single microRNA. For example, a panel comprising mir-7-5p, mir-1229, mir-1267, and mir-671-3p provides strong evidence for a pathologic process whereas a panel comprising mir-340-5p, mir-424-5p, mir-199b-Sp and mir-210 would provide strong evidence of a benign course of pregnancy. Other combinations of two or more micro-RNAs, including other micro-RNAs from the list in Fig. 3 or the other lists and tables of this disclosure may be useful and may include the entire table or list. It is obvious that combinations of any of the microRNAs listed ranging from 1 to 17 are understood to provide additional diagnostic power. Combined results of expression of the top and bottom 100 micro-RNAs before IVIG treatment to outcomes are listed in Figs. 4a-g, which may also be used to select one or more suitable micro-RNAs for implementing the techniques of this disclosure. Figs. 5a-b show sorted differences between mean levels of pregnancy outcomes (Top Highest 25 and Bottom Lowest 25 level differences) of the micro-RNAs from Figs. 4a-g.

Several groups of sequences have been discovered by use of the invention wherein the sequences are identified with groups dichotomous with respect to their response to an immunotherapy. Paired sequences, those that represent levels or differences in levels of one or more immune-cell comprised microRNA may be used clinically.

Accordingly, one embodiment includes the method of collecting samples of immune cells, preferably PBMCs, from a statistically sufficient number of patients contemplating an immunotherapy before therapy and following therapy. It is understood that while PBMCs may be used, subsets of PBMCs may be selected by means known in the art, for example flow cytometric sorting. Patients are sorted into dichotomous groups by the differences. Expected response ranges or normal ranges for patients in each group are established in accordance with practices well-known in the clinical laboratory field. Patients may be classified as members of one or the other group wherein their individual results fall within non-overlapping ranges by one or more microRNAs demonstrated to have the power to distinguish a patient population into the groups as described above.

In another embodiment, a panel of microRNA sequences of non-sequential microRNAs quantified on immune-cell samples preferably PBMCs, taken prior to a contemplated immunotherapy may be used clinically to catagorize patients into dichotomous groups. Samples of immune cells, preferably PBMCs, from a statistically sufficient number of patients contemplating an immunotherapy are collected before therapy. Patients are sorted into dichotomous groups by the differences. Expected response ranges or normal ranges for patients in each group are established in accordance with practices well-known in the clinical laboratory field. Patients may be classified as members of one or the other group wherein their individual results fall within non-overlapping ranges by one or more microRNA. Said classification permits determining suitability of the contemplated immunotherapy, for example resistance to the contemplated immunotherapy.

The invention not only teaches a method for the dichotomization or multimerization of groups of clinically similar patients into groups distinguishable by their microRNA profiles, it also discloses important microRNA species that are different within the groups that may be of great clinical significance. For example, in a genome-wide dissection of microRNA functions predicted by a computer algorithm, a number of microRNAs were identified whose response patterns segregate with dichotomization were ascribed in vivo functions (John S. Tsang, Margaret S. Ebert, and Alexander van Oudenaarden, Genome-wide dissection of microRNA functions and co-targeting networks using gene-set signatures, Mol Cell. 2010 April 9; 38(1): 140-153. doi: 10.1016/j.molcel.2010.03.007). Amongst microRNAs that showed significant differences in response between the two groups, hsa-mir-582-3p and 140-5p was identified as differentially expressed in non-functioning pituitary adenomas (Pituitary (2011) 14:112-124). These microRNAs were predicted to target Smad3, a member of the TGFβ signaling cascade. It does not escape attention that TGFβ is of primary importance in maintenance of pregnancy. Onouchi and Hata identify IVIG resistance in approximately fifteen percent of patients treated for Kawasaki Disease requiring increased dosing or alternative therapy. They associated genetic variations (SNPs) in the genes coding for ITPKC and caspase-3 with IVIG unresponsiveness (Yoshihiro Onouchi and Akira Hata, Responsible Genetic Factors for Vasculitis in Kawasaki Disease, Advances in the Etiology, Pathogenesis and Pathology of Vasculitis, 71-92). Identification of molecular markers that assess drug resistance or responsiveness is very important clinically. Further it does not escape us identification of patients into dichotomous groupings might allow defintion of individuals with higher risk of diseases including but not limited to autoimmune disease is an important aspect of this invention.

In a preferred embodiment the method comprises providing a biological sample comprising immune cells from a subject with a history of reproductive disorder or risk of reproductive disorder said sample being derived from immune cells, for example, derived from peripheral blood, and then isolating mononuclear cells as taught by Boyum (Boyum A 1983. Isolation of human blood monocytes with Nycodenz, a new non-ionic iodinated gradient medium. Scand J Immunol 17: 429-436). A kit optimized for recovery of microRNA sequences such as mirNeasy Mini Kit Qiagen catalogue 217004 may be used following instructions provided. Quantification of microRNA may be determined by a variety of techniques known to those skilled in the art. In a preferred embodiment, individual microRNAs are quantified by real-time polymerase chain reaction (PCR). In a more preferred embodiment, instructions provided for real-time, quantitative PCR are followed as provided by SABiosciences, Frederick, MD for use with primers listed below and reagents optimized for specific real-time thermocycling equipment such as the Stratagene Mx3005p (www.SABioscies.com). Operating instructions for the Stratagene Mx3005p are provided by the manufacturer. Comprised therein are instructions for spectrophotometric quantification of recovered RNA, recommendations for input quantity of RNA and PCR master mix. Quantification may be performed concurrently with quantification of a "housekeeper gene" (a gene that is expressed with relative constancy in the cells being interrogated thereby permitting relative quantification, preferably 18s RNA) and then determining the amount of microRNAs and comparing to the amount of the corresponding microRNA in the sample to similarly treated biological sample from control individuals. The subject is then diagnosed as being a candidate for immunotherapy such as IVIG, lymphocyte immunotherapy or anti-TNF alpha therapy after the methods of Winger et. al. if there is differential expression and/or pattern of expression in the amount of one or more of the microRNAs from the sample as compared to corresponding microRNA control levels and/or control patterns. Differential expression is defined statistically from three or more samples from control individuals wherein the patient value and/or pattern is two standard deviations above the control mean value and/or pattern. Preferably control values/patterns and patient values/patterns are determined on specimens drawn at the same time with respect to pregnancy, for example, during the period of preconception and at the same time during a menstrual cycle or following implantation. In addition, the method can comprise quantification of a plurality of samples wherein the control sample is the first sample value for one or more of the above listed microRNAs. Progress of the condition can be assessed by comparing values and/ or patterns subsequent to the control value and/or pattern. Efficacy of therapeutic intervention can be determined by comparing values and/or patterns subsequent to initiation of a therapeutic intervention to a control value and/or pattern on a biologic sample taken prior to initiation of therapy.

Other embodiments are directed to the diagnosis and treatment of immunological reproductive problems. According to the methods described above, PMBC MicroRNA levels and patterns may be used to diagnose immunological reproductive problems. Examples of such reproductive problems include implantation failure, infertility, miscarriage, preterm labor, PROM (premature rupture of membranes), IUGR(intrauterine growth retardation), antiphospholipid antibody syndrome, stillbirth, endometriosis and others. Further, PMBC MicroRNA levels and patterns may also be used to monitor treatment for these immunological reproductive problems.

As will be appreciated, PBMC MicroRNA levels and patterns may also be used to diagnose and monitor treatment for reproductive problems that may not be immunological but may be correlated with immunological reproductive problems. Representative conditions include increased tissue factor levels (microRNA 19b and 20a specifically) in Anti-phospholipid Antibody Syndrome, increased coagulation risk factors, PCOS (polycystic ovary syndrome) and premature ovarian failure (POF.)

In another aspect, PBMC MicroRNA levels and patterns may be used to diagnose and monitor treatment to prevent long term risks to the baby resulting from immunologically compromised pregnancy. These may include risk of the baby developing asthma, autism, ADHD, diabetes, schizophrenia, Tourette' syndrome, bipolar disorder or other conditions. Further, PBMC MicroRNA levels and patterns may be used to diagnose and monitor treatment for problems that may not be reproductive but may be correlated with reproductive immunological-problems, including autoimmune thyroiditis, migraines, lupus (SLE), rheumatoid arthritis flares, estrogen deficiency, osteoporosis, insulin resistance and others.

The methods and compositions of the invention may also be applied to the diagnosis or treatment of non-reproductive immunological diseases. Specifically, PBMC MicroRNA levels and patterns may be used to diagnose patients with non-reproductive immunological diseases that may that may benefit from immunotherapy (such as IVIG), including Kawasaki disease, ITP, Guillain-Barre Syndrome, autism, MS, lupus (SLE) or other conditions not yet identified that respond to immunotherapy treatment.

By identifying representative patient groups and microRNA responses, optimal immunologic and other therapies may be selected. Suitable representative therapies include use of IVIG, intralipid, G-CSF (Neupogen), corticosteroids (Prednisone, prednisolone, dexamethasone, etc), anti-TNFα alpha therapies (Humira , Enbrel, Simponi), fish oil (omega-3 oils), vitamin D, lymphocyte immunization therapy (LIT), levothyroxin, metformin, heparin and others.

Futher embodiments include using PBMC MicroRNA levels and patterns to determine optimal doses and treatment choices and/or combinations to control the specific immunologic conditions identified. Doses and combinations may include a single 25g of IVIG dose combined with Clexane 20 mg qd followed by microRNA retesting 2 weeks later, monthly Humira injections followed by no retesting, monthly Intralipid only, 2 mg dexamethasone daily for 3 months then drug tapering, or others.

As will be appreciated, PBMC MicroRNA levels and patterns may be used to identify patients that will not benefit or may experience negative side effects from therapy despite a positive disease diagnosis.

Yet another aspect of this disclosure is directed to identification of characteristic behavioral levels and patterns of a PBMC MicroRNA relative to other MicroRNAs. These levels and patterns may be associated with different disease and treatment phenotypes. These may be defined by ranges at which individual microRNAs operate, including basal levels, fully active levels, mean levels, observed standard deviations, from patterns observed in individual levels, from sequential microRNA samples based on patterns of deltas (differences) between sequential samples, and patterns and pattern changes observed between multiple microRNAs from individual or sequential samples.

PBMC MicroRNA levels and patterns may also be defined in part by polymorphisms. As discussed above, polymorphisms may occur in the 22-24 base of the microRNA itself, in the region around the microRNA in the pre-mircroRNA that affect folding of the hairpin and its transcription, in the target mRNA, or in the primary RNA strand complementary to the guide strand entering the RISC complex, for example.

Some microRNa levels and patterns may be best measured by effectively testing microRNA changes within the mononuclear cell population via the PBMCs. Effectively testing microRNA mononuclear cell population is demonstrated when removal of PBMCs eliminates the mononuclear microRNA measurement.

As discussed above, microRNA patterns and levels may be determined in a number of suitable manners. In a preferred embodiment, bimodal (dichotomous) or multimodal behavior of one or more microRNAa may be verified in before and/or after treatment groups. Further, useful clinical characteristics associated with these separated patient groups (such as degree of clinical improvement with IVIG), may be identified. Similarly, bimodal or multimodal behavior microRNAs may be used as markers to select optimal groups for further microRNA analysis or other diagnostic procedures. Also preferably, suitable methods include selection of microRNAs in common between most increased and most decreased top fractions of separated groups refines the identification of the groups.

Described herein are presently preferred embodiments. However, one skilled in the art that pertains to the present invention will understand that the principles of this disclosure can be extended easily with appropriate modifications to other applications.

## Claims

1. A method for identifying at least two characteristic groups in a patient population on the basis of microRNA expression, wherein one characteristic group is associated with a reproductive disorder or a risk of developing such a disorder, comprising the steps of:
a) quantifying at least one microRNA from a biological sample derived from immune cells; and
b) segregating the patient population into the groups on the basis of expression of the at least one microRNA.

2. The method of claim 1, further comprising the additional step of isolating mononuclear cells from the biological sample.

3. The method of claim 2, wherein the mononuclear cells comprise peripheral blood mononuclear cells.

4. A method according to any one of claims 1-3 , further comprising the additional step of extracting microRNA-comprising RNA from the biological sample.

5. A method according to any one of claims 1-4, wherein the step of segregating the patient population comprises assigning patients expressing a relatively high level of the at least one microRNA to a first group and assigning patients expressing a relatively low level of the at least one microRNA to a second group.

6. A method according to any one of claims 1-5, further comprising the steps of:
**a)** quantifying at least one microRNA from a biological sample derived from immune cells from an additional patient; and
**b)** identifying the additional patient as belonging to one of the segregated groups on the basis of expression of the at least one microRNA.

7. A method according to any one of claims 1-6, further comprising the step of diagnosing a patient as having said reproductive disorder or risk of developing such a disorder based on membership in a segregated group.

8. A method according to any one of claims 1-7, wherein the reproductive disorder is preeclampsia

9. A method according to any one of claims 1-8, wherein the at least one microRNA is selected from the group consisting of hsa-let-7e, hsa-miR-1181, hsa-miR-1183, hsa-miR-1224-5p,hsa-miR-127-3p, hsa-miR-1296, hsa-miR-132, hsa-miR-136, hsa-miR-139-3p, hsa-miR-141, hsa-miR-142-3p, hsa-miR-142-5p, hsa-miR-144, hsa-miR-153, hsa-miR-1537, hsa-miR-154,hsa-miR-191, hsa-miR-193a-3p, hsa-miR-19a, hsa-miR-219-5p, hsa-miR-29b, hsa-miR-301a, hsa-miR-301b, hsa-miR-30e, hsa-miR-32, hsa-miR-33a, hsa-miR-340, hsa-miR-362- 3p, hsa-miR-371-5p, hsa-miR-377, hsa-miR-423-3p, hsa-miR-432, hsa-miR-513a-5p, hsa-miR-545, hsa-miR-548a-5p, hsa-miR-574-5p, hsa-miR-582-3p, hsa-miR-590-5p, hsa-miR-15a, hsa-miR-548c-5p, hsa-miR-1225-3p, hsa-miR-21, hsa-miR-1237, hsa-miR-101, hsa-miR-1539, hsa-miR-557, hsa-miR-125a-3p, and hsa-miR-423-5p.

10. The method of claim 9, wherein the at least one microRNA is selected from the group consisting of hsa-miR-136, hsa-miR-141, hsa-miR-142-5p, hsa-miR-144, hsa-miR-153, hsa-miR-1537, hsa-miR-193a-3p, hsa-miR-219-5p, hsa-miR-29b, hsa-miR-301a, hsa-miR-32, hsa-miR-33a, hsa-miR-545, hsa-miR-582-3p, hsa-miR-590-5p, hsa-miR-1181, hsa-miR-513a-5p, hsa-miR-132, and hsa-miR-1296.

11. A method according to any one of claims 1-8, wherein the at least one microRNA is selected from the group consisting of hsa-miR-144, hsa-miR-582-5p, hsa-miR-30e-3p, hsa-miR- 340-5p, hsa-miR-424-5p, hsa-miR-199a-5p, hsa-miR-199b-5p, hsa-miR-210, hsa-miR-221-5p, hsa-miR-33a-5p, hsa-miR-575, hsa-miR-7-5p, hsa-miR-1229, hsa-miR-1267, hsa-miR-671-3p, hsa-miR-1244, hsa-miR-1, and hsa-miR-133b.

12. The method of claim 11, wherein the at least one microRNA is selected from the group consisting of hsa-miR-1229 and hsa-miR-671-3p.

13. A method according to any one of claims 1-8, wherein the at least one microRNA comprises at least four microRNAs selected from the group consisting of hsa-miR-7-5p, hsa-miR-1229, hsa-miR-1267, hsa-miR-671-3p, hsa-miR-340-5p, hsa-miR-1, hsa-miR-133b, and hsa-miR-33a-5p.

14. A method according to any one of claims 1-13, wherein the one characteristic group is associated with a risk of developing a reproductive disorder.

## Patentansprüche

1. Verfahren zum Identifizieren von mindestens zwei charakteristischen Gruppen in einer Patientengruppe auf der Basis eines microRNA-Ausdrucks, wobei eine charakteristische Gruppe einer reproduktiven Störung oder einem Risiko zur Entwicklung einer solchen Störung zugeordnet ist, umfassend folgende Schritte:
**a)** Quantifizieren von mindestens einer microRNA aus einer biologischen Probe, die aus Immunzellen stammt; und
**b)** Aufspalten der Patientengruppe in die Gruppen auf der Basis des Ausdrucks der mindestens einen microRNA.

2. Verfahren nach Anspruch 1, des Weiteren umfassend den zusätzlichen Schritt zum Isolieren mononuklearer Zellen aus der biologischen Probe.

3. Verfahren nach Anspruch 2, wobei die mononuklearen Zellen periphere mononukleare Blutzellen umfassen.

4. Verfahren nach einem der Ansprüche 1-3, des Weiteren umfassend den zusätzlichen Schritt zum Extrahieren von microRNA-umfassender RNA aus der biologischen Probe.

5. Verfahren nach einem der Ansprüche 1-4, wobei der Schritt zum Abspalten der Patientengruppe das Zuweisen von Patienten, die ein relativ hohes Niveau der mindestens einen microRNA aufweisen, zu einer ersten Gruppe, und das Zuweisen von Patienten, die ein relativ niedriges Niveau der mindestens einen microRNA aufweisen, zu einer zweiten Gruppe umfasst.

6. Verfahren nach einem der Ansprüche 1-5, des Weiteren umfassend folgende Schritte:
**a)** Quantifizieren von mindestens einer microRNA aus einer biologischen Probe, die aus Immunzellen von einem zusätzlichen Patienten stammt; und
**b)** Identifizieren des zusätzlichen Patienten als zugehörig zu einer der aufgespaltenen Gruppen auf der Basis des Ausdrucks der mindestens einen microRNA.

7. Verfahren nach einem der Ansprüche 1-6, des Weiteren umfassend den Schritt zum Diagnostizieren eines Patienten als Träger der reproduktiven Störung oder des Risikos zur Entwicklung einer solchen Störung basierend auf der Zugehörigkeit zu einer aufgespaltenen Gruppe.

8. Verfahren nach einem der Ansprüche 1-7, wobei die reproduktive Störung Präeklampsie ist.

9. Verfahren nach einem der Ansprüche 1-8, wobei die mindestens eine microRNA aus der Gruppe gewählt wird, die aus hsa-let-7e, hsa-miR-1181, hsa-miR-1183, hsa-miR-1224-5p, hsa-miR-127-3p, hsa-miR-1296, hsa-miR-132, hsa-miR-136, hsa-miR-139-3p, hsa-miR-141, hsa-miR-142-3p, hsa-miR-142-5p, hsa-miR-144, hsa-miR-153, hsa-miR-1537, hsa-miR-154, hsa-miR-191, hsa-miR-193a-3p, hsa-miR-19a, hsa-miR-219-5p, hsa-miR-29b, hsa-miR-301a, hsa-miR-301b, hsa-miR-30e, hsa-miR-32, hsa-miR-33a, hsa-miR-340, hsa-miR-362- 3p, hsa-miR-371-5p, hsa-miR-377, hsa-miR-423-3p, hsa-miR-432, hsa-miR-513a-5p, hsa-miR-545, hsa-miR-548a-5p, hsa-miR-574-5p, hsa-miR-582-3p, hsa-miR-590-5p, hsa-miR-15a, hsa-miR-548c-5p, hsa-miR-1225-3p, hsa-miR-21, hsa-miR-1237, hsa-miR-101, hsa-miR-1539, hsa-miR-557, hsa-miR-125a-3p und hsa-miR-423-5p besteht.

10. Verfahren nach Anspruch 9, wobei die mindestens eine microRNA aus der Gruppe gewählt wird, die aus hsa-miR-136, hsa-miR-141, hsa-miR-142-5p, hsa-miR-144, hsa-miR-153, hsa-miR-1537, hsa-miR-193a-3p, hsa-miR-219-5p, hsa-miR-29b, hsa-miR-301a, hsa-miR-32, hsa-miR-33a, hsa-miR-545, hsa-miR-582-3p, hsa- miR-590-5p, hsa-miR-1181, hsa-miR-513a-5p, hsa-miR-132 und hsa-miR-1296 besteht.

11. Verfahren nach einem der Ansprüche 1-8, wobei die mindestens eine microRNA aus der Gruppe gewählt wird, die aus hsa-miR-144, hsa-miR-582-5p, hsa-miR-30e-3p, hsa-miR-340-5p, hsa-miR-424-5p, hsa-miR-199a-5p, hsa-miR-199b-5p, hsa-miR-210, hsa-miR-221-5p, hsa-miR-33a-5p, hsa-miR-575, hsa-miR-7-5p, hsa-miR-1229, hsa-miR-1267, hsa-miR-671-3p, hsa-miR-1244, hsa-miR-1 und hsa-miR-133b besteht.

12. Verfahren nach Anspruch 11, wobei die mindestens eine microRNA aus der Gruppe gewählt wird, die aus hsa-miR-1229 und hsa-miR-671-3p besteht.

13. Verfahren nach einem der Ansprüche 1-8, wobei die mindestens eine microRNA mindestens vier microRNAs umfasst, die aus der Gruppe gewählt werden, die aus hsa-miR-7-5p, hsa-miR-1229, hsa-miR-1267, hsa-miR-671-3p, hsa-miR-340-5p, hsa-miR-1, hsa-miR-133b und hsa-miR-33a-5p besteht.

14. Verfahren nach einem der Ansprüche 1-13, wobei die eine charakteristische Gruppe einem Risiko zur Entwicklung einer reproduktiven Störung zugeordnet ist.

## Revendications

1. Procédé destiné à identifier au moins deux groupes caractéristiques dans une population de patients, en fonction de l'expression du microARN, dans lequel un groupe caractéristique est associé à un trouble de la reproduction ou un risque de développer ledit trouble, comprenant les étapes consistant à :
**a)** quantitier au moins un microARN dans un échantillon biologique provenant de cellules immunitaires ; et
**b)** séparer la population de patients de groupes, en fonction de l'expression du au moins un microARN.

2. Procédé selon la revendication 1, comprenant en outre l'étape supplémentaire consistant à isoler des cellules mononuclées de l'échantillon biologique

3. Procédé selon la revendication 2, dans lequel les cellules mononuclées comprennent des cellules mononuclées du sang périphérique.

4. Procédé selon l'une quelconque des revendications 1 à 3, comprenant en outre l'étape supplémentaire consistant à extraire l'ARN comprenant le microARN de l'échantillon biologique.

5. Procédé selon l'une quelconque des revendications 1 à 4, dans lequel l'étape consistant à séparer la population de patients comprend l'assignation de patients qui expriment un degré relativement élevé du au moins un microARN à un premier groupe et l'assignation des patients qui expriment un degré relativement faible du au moins un microARN à un second groupe.

6. Procédé selon l'une quelconque des revendications 1 à 5, comprenant en outre les étapes consistant à :
**a)** quantifier au moins un microARN dans un échantillon biologique provenant de cellules immunitaires d'un autre patient ; et
**b)** identifier l'autre patient comme appartenant à l'un des groupes séparés, en fonction de l'expression du au moins un microARN.

7. Procédé selon l'une quelconque des revendications 1 à 6, comprenant en outre l'étape consistant à diagnostiquer un patient comme ayant ledit trouble de la reproduction ou un risque de développer ledit trouble, d'après son appartenance à un groupe séparé.

8. Procédé selon l'une quelconque des revendications 1 à 7, dans lequel le trouble de la reproduction est la pré-éclampsie.

9. Procédé selon l'une quelconque des revendications 1 à 8, dans lequel le au moins un microARN est sélectionné dans le groupe composé de hsa-let-7e, hsa-miR-1181, hsa-miR-1183, hsa-miR-1224-5p, hsa-miR-127-3p, hsa-miR-1296, hsa-miR-132, hsa-miR-136, hsa-miR-139-3p, hsa-miR-141, hsa-miR-142-3p, hsa-miR-142-5p, hsa-miR-144, hsa-miR-153, hsa-miR-1537, hsa-miR-154, hsa-miR-191, hsa-miR-193a-3p, hsa-miR-19a, hsa-miR-219-5p, hsa-miR-29b, hsa-miR-301a, hsa-miR-301b, hsa-miR-30e, hsa-miR-32, hsa-miR-33a, hsa-miR-340, hsa-miR-362- 3p, hsa-miR-371-5p, hsa-miR-377, hsa-miR-423-3p, hsa-miR-432, hsa-miR-513a-5p, hsa-miR-545, hsa-miR-548a-5p, hsa-miR-574-5p, hsa-miR-582-3p, hsa-miR-590-5p, hsa-miR-15a, hsa-miR-548c-5p, hsa-miR-1225-3p, hsa-miR-21, hsa-miR-1237, hsa-miR-101, hsa-miR-1539, hsa-miR-557, hsa-miR-125a-3p, et hsa-miR-423-5p.

10. Procédé selon la revendication 9, dans lequel le au moins un microARN est sélectionné dans est sélectionné dans le groupe composé de hsa-miR-136, hsa-miR-141, hsa-miR-142-5p, hsa-miR-144, hsa-miR-153, hsa-miR-1537, hsa-miR-193a-3p, hsa-miR-219-5p, hsa-miR-29b, hsa-miR-301a, hsa-miR-32, hsa-miR33a, hsa-miR-545, hsa-miR-582-3p, hsa-miR-590-5p, hsa-miR-1181, hsa-miR-513a-5p, hsa-miR-132, et hsa-miR-1296.

11. Procédé selon l'une quelconque des revendications 1 à 8, dans lequel le au moins un microARN est sélectionné dans le groupe composé de hsa-miR-144, hsa-miR-582-5p, hsa-miR-30e-3p, hsa-miR-340-5p, hsa-miR-424-5p, hsa-miR-199a-5p, hsa-miR-199b-5p, hsa-miR-210, hsa-miR-221-5p, hsa-miR-33a-5p, hsa-miR-575, hsa-miR-7-5p, hsa-miR-1229, hsa-miR-1267, hsa-miR-671-3p, hsa-miR-1244, hsa-miR-1, et hsa-miR-133b.

12. Procédé selon la revendication 11, dans lequel le au moins un microARN, est sélectionné dans le groupe composé de hsa-miR-1229 et hsa-miR-671-3p.

13. Procédé selon l'une quelconque des revendications 1 à 8, dans lequel le au moins un microARN comprend au moins quatre microARN sélectionné dans le groupe composé de hsa-miR-7-5p, hsa-miR-1229, hsa-miR-1267, hsa-miR-671-3p, hsa-miR-340-5p, hsa-miR-1, hsa-miR-133b, et hsa-miR-33a-5p.

14. Procédé selon l'une quelconque des revendications 1 à 13, dans lequel le groupe caractéristique est associé à un risque de développer un trouble de la reproduction.
